(19) Europäisches Patentamt
European Patent Office
Office européen des brevets

(11) EP 4 217 464 B1

(12) **EUROPEAN PATENT SPECIFICATION**

(45) Date of publication and mention of the grant of the patent:
**22.05.2024 Bulletin 2024/21**

(21) Application number: **21801685.5**

(22) Date of filing: **22.09.2021**

(51) International Patent Classification (IPC):
***C12N 5/00*** *(2006.01)*

(52) Cooperative Patent Classification (CPC):
**C12M 47/04; C12M 25/16; C12N 5/0006; C12N 5/0012;** C12N 2537/10

(86) International application number:
**PCT/US2021/051542**

(87) International publication number:
**WO 2022/066760 (31.03.2022 Gazette 2022/13)**

(54) **SELECTIVE ENZYMATIC GELATION**
SELEKTIVE ENZYMATISCHE GELIERUNG
GÉLIFICATION ENZYMATIQUE SÉLECTIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.09.2020 US 202063082325 P**
**26.05.2021 US 202163193571 P**

(43) Date of publication of application:
**02.08.2023 Bulletin 2023/31**

(60) Divisional application:
**24169739.0**

(73) Proprietor: **10X Genomics, Inc.**
**Pleasanton, CA 94588 (US)**

(72) Inventors:
- **GOHIL, Shalini**
**Pleasanton, California 94588 (US)**
- **DELANEY, Joshua**
**Pleasanton, California 94588 (US)**
- **LUO, Yi**
**Pleasanton, California 94588 (US)**
- **LOWE, Adam**
**Pleasanton, California 94588 (US)**
- **BELL, Jason C.**
**Pleasanton, California 94588 (US)**
- **STUBBINGTON, Michael John Terry**
**Bristol BS1 6BX (GB)**
- **MCDONNELL, Wyatt James**
**Pleasanton, California 94588 (US)**

(74) Representative: **J A Kemp LLP**
**80 Turnmill Street**
**London EC1M 5QU (GB)**

(56) References cited:
**WO-A1-2020/176449 US-A1- 2015 376 609**

- **SAKAI SHINJI ET AL: "Cell-selective encapsulation in hydrogel sheaths via biospecific identification and biochemical cross-linking", BIOMATERIALS, vol. 53, 21 March 2015 (2015-03-21), pages 494-501, XP055880745, AMSTERDAM, NL ISSN: 0142-9612, DOI: 10.1016/j.biomaterials.2015.02.119 cited in the application**
- **KHANMOHAMMADI MEHDI ET AL: "Production of hyaluronic-acid-based cell-enclosing microparticles and microcapsules via enzymatic reaction using a microfluidic system", JOURNAL OF APPLIED POLYMER SCIENCE, vol. 133, no. 16, 31 December 2015 (2015-12-31), pages 43107-1-43107-8, XP55882100, US ISSN: 0021-8995, DOI: 10.1002/app.43107**
- **SHINJI SAKAI ET AL: "On-Cell Surface Cross-Linking of Polymer Molecules by Horseradish Peroxidase Anchored to Cell Membrane for Individual Cell Encapsulation in Hydrogel Sheath", ACS MACRO LETTERS, vol. 3, no. 10, 17 September 2014 (2014-09-17), pages 972-975, XP055498145, ISSN: 2161-1653, DOI: 10.1021/mz5004322**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

## CROSS-REFERENCE TO RELATED APPLICATIONS

## FIELD

The present disclosure relates to methods for selective enzymatic gelation of biological particles, such as cells, *e.g.,* immune cells, or nuclei contained in partitions.

## BACKGROUND

A microscopic biological sample, such as an individual cell, may be separated and isolated in an individual partition, such as a liquid droplet, which allow the individual sample to be subjected to a variety of processes. For example, a droplet containing a single cell may be fluidically isolated from other droplets containing different types of cells, enabling accurate control of respective environments in the droplets. A single cell in a partition may be cultured, subjected to chemical or biochemical stimuli, or physical processes such as heating, cooling, or chemical reactions. Changes in the cell, and/or species produced in the partition may be qualitatively or quantitatively processed using processes such as PCR and/or sequencing. The information obtained from carrying out such processes on individual cells can allow early detection of disease states such as cancer.

WO2019/071039 discloses systems and methods for making a hydrogel comprising a cell, cell nucleus, or one or more components derived from a cell or cell nucleus by generating a partition containing a cell and a plurality of polymers with crosslink precursors that undergo a crosslinking reaction via click chemistry to form a hydrogel in the partition containing the cell.

WO2020/176449 discloses systems and methods for high throughput selection, relating in one aspect to cells contained within gel droplets.

Hydrogel generation using HRP-catalyzed crosslinking of phenolic polymers has been used in a variety of drug delivery and tissue engineering applications. See *e.g.,* S. Sakai, Y. Liu, M. Sengoku, M. Taya. "Cell-selective encapsulation in hydrogel sheaths via biospecific identification and biochemical cross-linking." Biomaterials 53 (2015) 494-501; S.V. Gohil, S.B. Brittain, H. Kan, H. Drissi, D.W. Rowe, L.S. Nair. "Evaluation of enzymatically crosslinked injectable glycol chitosan hydrogel." J. Mater. Chem. B 3 (2015) 5511-5522.

There remains a need for methods that provide selective gelation of cell-containing partitions to provide specific cells encapsulated by a hydrogel. The present disclosure fulfills these and other needs.

## SUMMARY

The present disclosure generally relates to, *inter alia,* methods useful for preparing a hydrogel-coated biological particle (e.g., a cell or nucleus).

The present disclosure provides a method of partitioning cells comprising:

(a) generating a partition containing (i) a cell comprising a plurality of crosslink-catalyzing moieties attached to its membrane through a linker comprising a membrane anchor moiety, and (ii) a linear polymer comprising a crosslink-precursor moiety;
(b) contacting the partition with a crosslink-forming initiator; whereby a hydrogel-coating of the cell is formed; and
(c) cleaving the linker; whereby the crosslink-catalyzing moieties are released resulting in an increased thickness of hydrogel-coating of the cell.

Non-limiting exemplary embodiments of the method as described herein can include one or more of the following features.

In at least one embodiment of the method, the thickness of the hydrogel-coating is at least 5 $\mu$m, at least 10 $\mu$m, at least 20 $\mu$m, at least 30 $\mu$m, at least 40 $\mu$m, at least 50 $\mu$m, at least 75 $\mu$m, at least 100 $\mu$m, at least 120 $\mu$m, at least 150 $\mu$m, at least 200 $\mu$m, or more; optionally, wherein the hydrogel-coating has an average thickness of from about 5 $\mu$m to about 200 $\mu$m, from about 25 $\mu$m to about 175 $\mu$m, from about 30 $\mu$m to about 150 $\mu$m, or from about 50 $\mu$m to about 150 $\mu$m. In at least one embodiment, the partition is a discrete droplet.

In at least one embodiment of the method of present disclosure, the membrane anchor moiety is selected from a Biocompatible Anchor for cell Membrane (BAM) moiety; an antibody to a cell membrane protein; a cholesterol-oligonucleotide moiety; a 3'-cholesterol-TEG moiety; a cholesterol-decorated polymer; a target antigen. In at least one embodiment, the membrane anchor moiety is a BAM moiety comprising an oleyl moiety; optionally, wherein the BAM moiety comprises an oleyl-O-$(CH_2CH_2O)_n$-CO-$CH_2CH_2$-COO moiety, wherein the number of polyethylene glycol groups, n, is

such that the moiety has a molecular weight of at least 2000, at least 4000, or at least 8000. In at least one embodiment, the membrane anchor moiety is an antibody to a cell surface protein; optionally, wherein the cell surface protein is a cluster of differentiation ("CD") protein.

In at least one embodiment of the method of present disclosure, the crosslink-catalyzing moiety is an enzyme selected from a peroxidase (*e.g.,* HRP); transglutaminase; tyrosinase; and laccase. In at least one embodiment, the crosslink-catalyzing moiety is a non-enzymatic compound selected from hematin and umbelliferone. In at least one embodiment, the crosslink-forming initiator comprises an enzyme co-substrate, such as a compound comprising peroxide moiety; optionally, wherein the co-substrate is $H_2O_2$.

In at least one embodiment of the method of present disclosure, the crosslink-catalyzing moiety is a peroxidase (*e.g.* HRP), the crosslink-precursor moieties are phenol groups, and the crosslink-forming initiator is an enzyme co-substrate, such as a peroxide compound (*e.g.,* $H_2O_2$).

In at least one embodiment of the method of present disclosure, the crosslink-forming initiator is contained in a micelle. In at least one embodiment, contacting the partition with a crosslink-forming initiator comprises micelle-mediated transport of the co-substrate into the partition.

In at least one embodiment of the method of present disclosure, the linker comprises a cleavable moiety; optionally, wherein the cleavable moiety is selected from a disulfide spacer moiety; a carbamate spacer moiety; a photocleavable spacer; and UDG-cleavable spacer. In at least one embodiment, cleaving the linker comprises contacting the partition with a reagent selected from DTT, and DETA.

In at least one embodiment of the method of the present disclosure, the linear polymer is selected from an olefin copolymer, a polyolefin, an acrylic, a polyacrylamide, a poly(oxazoline), a vinyl polymer, a polyester, a polycarbonate, a polyamide, a polyimide, a formaldehyde resin, a polyurethane, an ether polymer, a cellulosic, a thermoplastic elastomer, and a thermoplastic polyurethane. In at least one embodiment, the linear polymer further comprises a modifiable side-chain; optionally, wherein the modifiable side-chain comprises an amine moiety. In at least one embodiment, the method further comprises contacting under suitable reaction conditions the hydrogel-coated cell with a detectable label moiety comprising a group capable of forming a covalent linkage to the modifiable side-chain of the hydrogel.

In at least one embodiment of the method of the present disclosure, the method further comprises contacting under suitable reaction conditions the hydrogel-coated cell with a surface of a solid substrate, wherein the surface comprises a group capable of forming a covalent linkage to the modifiable side-chain of the hydrogel under the reaction condition, whereby the hydrogel-coated cell is covalently attached to the solid substrate.

In at least one embodiment of the method of the present disclosure, the cell is an immune cell. In at least one embodiment, the immune cell expresses an antigen-binding molecule (ABM) or antigen-binding fragment thereof. In at least one embodiment, the ABM is selected from the group consisting of an antibody or functional fragment thereof, an immune receptor, and an immunoglobulin. In at least one embodiment, the ABM is an immunoglobulin (Ig). In at least one embodiment, the Ig is selected from the group consisting of IgA, IgD, IgE, IgG, and IgM. In at least one embodiment, the Ig is IgG.

In at least one embodiment, the membrane anchor moiety is a target antigen.

In at least one embodiment, the method further comprises, prior to the (a) generating the partition, contacting the immune cell with a target antigen *(the plurality of crosslink-catalyzing moieties attached to its membrane through a linker comprising a membrane anchor moiety),* wherein the contacting provides an immune cell bound to the target antigen *(attached to a crosslink-catalyzing moiety).*

In at least one embodiment of the method of the present disclosure, the immune cell is a B cell. In at least one embodiment, the target antigen is selected from the group consisting of soluble proteins, short polypeptides, virus-like particles, and membrane-bound proteins.

In at least one embodiment of the method of the present disclosure, the immune cell is a T cell. In at least one embodiment, the target antigen is selected from the group consisting of pMHC monomers and multimers.

In at least one embodiment of the method of the present disclosure, the method further comprises subsequent to the (b) partitioning, isolating and/or enriching the immune cell bound to the target antigen.

In at least one embodiment of the present disclosure, the target antigen is coupled to a first reporter oligonucleotide.

In at least one embodiment of the present disclosure, the ABM or antigen-binding fragment thereof is coupled to a second reporter oligonucleotide. In at least one embodiment, the first and/or the second reporter nucleotide is conjugated to labelling agents. In at least one embodiment, the labelling agents are magnetic or fluorescent.

In at least one embodiment of the method of the present disclosure, the partition further comprises a plurality of nucleic acid barcode molecules comprising a partition-specific barcode sequence. In at least one embodiment, the method further comprises generating, in the partition, barcoded nucleic acid molecules, wherein the barcoded nucleic acid molecules comprise: (i) a first barcoded nucleic acid molecule comprising a sequence of the first or second reporter oligonucleotide or a reverse complement thereof and the partition-specific barcode sequence or reverse complement thereof. In an additional embodiment, the barcoded nucleic acid molecules further comprise: (ii) a second barcoded

nucleic acid molecule comprising a nucleic acid sequence encoding at least a portion of the ABM, or antigen-binding fragment thereof, expressed by the immune cell or reverse complement thereof and the partition-specific barcode sequence or reverse complement thereof. In at least one embodiment, the first and/or second barcoded nucleic molecule further comprises a UMI sequence.

In at least one embodiment of the method of the present disclosure, the method further comprises determining sequences of the first and the second barcoded nucleic acid molecule. In at least one embodiment, the method further comprises identifying and/or characterizing the ABM or antigen-binding fragment thereof based on the determined sequence of the second barcoded nucleic acid molecule.

In some embodiments, the ABM or antigen-binding fragment is identified based on the determined sequence of the second barcoded nucleic acid molecule. In some embodiments, the determined sequence comprises a nucleotide sequence. In some embodiments, the determined sequence comprises an amino acid sequence. In some embodiments, the method disclosed herein further comprises assessing affinity of the ABM or antigen-binding fragment thereof, based on the generated first barcoded nucleic acid molecule. In some embodiments, the disclosed herein further comprises contacting the immune cell with (i) a negative control antigen having or being suspected of having little or no binding affinity for the immune cell; and/or (ii) a positive control agent having or being suspected of having a binding affinity for the immune cell.

## BRIEF DESCRIPTION OF THE DRAWINGS

**FIG. 1** depicts an exemplary scheme for preparing a BAM moiety attached to a horseradish peroxidase ("HRP") enzyme moiety through a cleavable linker.

**FIG. 2** depicts an exemplary step of using the BAM-linker-enzyme moieties prepared as depicted in **FIG. 1** to prepare a cell modified (or "decorated") with enzyme moieties

**FIG. 3** depicts an exemplary use of a 5'-biotin-modified oligonucleotide with a 3'-cholesterol cell anchor moiety to decorate a cell.

**FIG. 4** depicts an exemplary scheme for further modification of a cell previously decorated with 5'-biotin-oligonucleotides with HRP-streptavidin moieties.

**FIG. 5** depicts an exemplary process for generating an enzyme decorated cell via antibody binding to cell surface protein or antigen.

**FIG. 6** depicts an exemplary treatment of biotin-antibody decorated cell with streptavidin-enzyme conjugates resulting in an enzyme decorated cell.

**FIG. 7** depicts an exemplary scheme for the peroxidase catalyzed crosslinking of phenol-modified linear polymers in the presence of the co-substrate $H_2O_2$ to form a hydrogel matrix.

**FIG. 8** depicts an exemplary 3-step scheme for preparing phenol-modified linear polymers capable of undergoing enzyme-catalyzed crosslinking to form a hydrogel matrix.

**FIG. 9** depicts an exemplary reaction scheme for modifying a polyacrylamide with a phenol groups attached through a linker comprising a cleavable disulfide moiety.

**FIG. 10** depicts an exemplary discrete droplet partition containing a cell decorated with HRP enzyme moieties through linkers with cleavable disulfide moieties and linear polymers modified with phenol groups.

**FIG. 11** depicts the initial formation of the phenol crosslinked hydrogel matrix around the HRP decorated cell in the present co-substrate, $H_2O_2$ (not shown).

**FIG. 12** depicts the extension of the phenol crosslinked hydrogel matrix to a greater thickness around the cell following cleavage of disulfide linkers which allow the HRP moieties to diffuse into the previously un-crosslinked portions of the linear polymer solution.

**FIG. 13** shows an example of a microfluidic channel structure for generating partitions containing individual biological particles, such as enzyme-decorated cells and linear polymers.

**FIG. 14** shows an example of a microfluidic channel structure for delivering barcodes on beads into partitions.

**FIG. 15** shows an example of a microfluidic channel structure for co-partitioning enzyme-decorated cells, linear polymers, barcodes, and other reagents.

**FIG. 16** shows an example of a microfluidic channel structure for the controlled partitioning of into discrete droplets.

**FIG. 17** shows an example of a microfluidic channel structure for increased discrete droplet generation throughput.

**FIG. 18** shows another example of a microfluidic channel structure for increased discrete droplet generation throughput.

**FIG. 19** depicts the structure of the BAM linker and reaction scheme used to prepare the BAM-HRP moiety used to decorate cells as described in Example 1.

**FIG. 20** depicts an exemplary workflow for the selection of cells using cell-specific HRP decoration and gelation.

**FIG. 21** depicts an exemplary workflow in accordance with some non-limiting embodiments of the disclosure that can be employed for the identification and or characterization of novel antigen-binding molecules (e.g., BCR, TCR, and fragments thereof) by selective gelation of antigen-binding cells.

**FIG. 22** schematically illustrates an example microwell array.

**FIG. 23** shows an exemplary barcode carrying bead

**FIG. 24** illustrates another example of a barcode carrying bead

**FIG. 25** schematically illustrates an example workflow for processing nucleic acid molecules.

**FIG. 26** schematically illustrates examples of labelling agents.

**FIG. 27** depicts an example of a barcode carrying bead.

**FIGS. 28A, 28B, and 28C** schematically depict an example workflow for processing nucleic acid molecules.

**FIG. 29** depicts a block diagram illustrating an example of a computing system, in accordance with some example embodiments.

## DETAILED DESCRIPTION

The present disclosure generally relates to, *inter alia,* to hydrogel-coated biological particle (e.g., cell, cell bead, or nucleus) compositions and methods for their preparation. In one embodiment, the hydrogel coating is via a two-step enzymatically catalyzed gelation process. The disclosed methods are suitable for selectively coating a biological particle, such as a cell, *e.g.,* an immune cell, or a nucleus with a hydrogel at a thickness that is substantially thicker (*e.g.* from at least about 5 μm to about 200 μm) than the hydrogel sheaths of cells produced by known methods. The hydrogel-coated biological particles (e.g., cells or nuclei) of the present disclosure are substantially more robust and useful in a range of single-cell, single-cell bead, or single-nucleus assays. Briefly, the method of the present disclosure involves (a) generating a partition containing (i) a biological particle (e.g., a cell, cell bead, or nucleus) comprising a plurality of crosslink-catalyzing moieties attached to its membrane through a linker comprising a membrane anchor moiety, and (ii) a linear polymer comprising a crosslink-precursor moiety; (b) contacting the partition containing the biological particle (e.g., cell, cell bead or nucleus) with a crosslink-forming initiator, whereby the membrane-tethered enzyme moieties catalyze crosslinking of the linear polymers to form an initial hydrogel-coating around the biological particle (e.g., cell, cell bead, or nucleus); and (c) cleaving the linker tethering the enzyme moieties to the biological particle (e.g., cell or nuclear membrane) thereby allowing the enzyme moieties to diffuse away from the biological particle (e.g., cell or nucleus) and catalyze a growth in the thickness of the hydrogel-coating around the biological particle (e.g., cell or nucleus). The methods can thereby provide hydrogel-coated biological particle (e.g., cell, cell bead, or nucleus) compositions with hydrogel-coatings have an average thickness of from about 5 μm to about 200 μm. As described elsewhere herein, further modifications to the methods allow for the preparation of selectively hydrogel-coated biological particles (e.g., cells, cell beads, or nuclei) from population of biological particles (e.g., cells, cell beads, or nuclei) that can then be further manipulated and used in various partition-based assays.

While various embodiments of the disclosure are described herein, those skilled in the art will recognize that such embodiments are provided by way of example only. Numerous variations, changes, and substitutions to the disclosed embodiments may occur to those skilled in the art without departing from the scope of the invention as defined in the claims. It should be understood that various alternatives to the embodiments described herein may be employed.

### Definitions

For the descriptions herein and the appended claims, the singular forms "a", and "an" include plural referents unless the context clearly indicates otherwise. For example, the term "a cell" includes one or more cells, including mixtures thereof. "A and/or B" is used herein to include all of the following alternatives: "A", "B", "A or B", and "A and B". Thus, for example, reference to "a protein" includes more than one protein, and reference to "a compound" refers to more than one compound. The use of "comprise," "comprises," "comprising" "include," "includes," and "including" are interchangeable and not intended to be limiting. It is to be further understood that where descriptions of various embodiments use the term "comprising," those skilled in the art would understand that in some specific instances, an embodiment can be alternatively described using language "consisting essentially of" or "consisting of."

The term "biological particle," as used herein, generally refers to a discrete biological system derived from a biological sample. The biological particle may be a macromolecule. The biological particle may be a small molecule. The biological particle may be a virus, e.g., a phage. The biological particle may be a cell or derivative of a cell. The biological particle may be an organelle. Examples of an organelle from a cell include, without limitation, a nucleus, endoplasmic reticulum, a ribosome, a Golgi apparatus, an endoplasmic reticulum, a chloroplast, an endocytic vesicle, an exocytic vesicle, a vacuole, and a lysosome. The biological particle may be a rare cell from a population of cells. The biological particle may be any type of cell, including without limitation prokaryotic cells, eukaryotic cells, bacterial, fungal, plant, mammalian, or other animal cell type, mycoplasmas, normal tissue cells, tumor cells, or any other cell type, whether

derived from single cell or multicellular organisms. The biological particle may be a constituent of a cell. The biological particle may be or may include DNA, RNA, organelles, proteins, or any combination thereof. The biological particle may be or may include a matrix (*e.g.,* a gel or polymer matrix) including a cell or one or more constituents from a cell (*e.g.,* cell bead), such as DNA, RNA, organelles, proteins, or any combination thereof, from the cell. The biological particle may be obtained from a tissue of a subject. The biological particle may be a hardened cell. Such hardened cell may or may not include a cell wall or cell membrane. The biological particle may include one or more constituents of a cell, but may not include other constituents of the cell. An example of such constituents is a nucleus or an organelle. A cell may be a live cell. The live cell may be capable of being cultured, for example, being cultured when enclosed in a gel or polymer matrix, or cultured when including a gel or polymer matrix.

The term "sample," as used herein, generally refers to a biological sample of a subject. The sample may be a tissue sample, such as a biopsy, core biopsy, needle aspirate, or fine needle aspirate. The sample may be a fluid sample, such as a blood sample, urine sample, or saliva sample. The sample may be a skin sample. The sample may be a cheek swap. The sample may be a plasma or serum sample.

Where a range of values is provided, unless the context clearly dictates otherwise, it is understood that each intervening integer of the value, and each tenth of each intervening integer of the value, unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of these limits, ranges excluding (i) either or (ii) both of those included limits are also included in the invention. For example, "1 to 50," includes "2 to 25," "5 to 20," "25 to 50," "1 to 10," etc.

Unless defined otherwise, all technical and scientific terms used herein have the same meaning as commonly understood by one of ordinary skill in the art to which the present invention pertains. It is to be understood that the terminology used herein is for describing particular embodiments only and is not intended to be limiting. For purposes of interpreting this disclosure, the following description of terms will apply and, where appropriate, a term used in the singular form will also include the plural form and vice versa.

The present disclosure generally relates to, *inter alia,* methods useful for preparing a hydrogel-coated biological particle (e.g., cell, cell bead, or nucleus) compositions useful in a range of partition-based single-cell, single-cell bead, or single-nucleus methods and assays. In particular, some embodiments of the disclosure relate to a method of preparation of a hydrogel-coated cell (or cell bead or nucleus) composition that comprises:

(a) generating a partition containing (i) a cell comprising a plurality of crosslink-catalyzing moieties attached to its membrane through a linker comprising a membrane anchor moiety, and (ii) a linear polymer comprising a crosslink-precursor moiety;
(b) contacting the partition with a crosslink-forming initiator; whereby a hydrogel-coating of the cell is formed; and
(c) cleaving the linker; whereby the crosslink-catalyzing moieties are released and the thickness of hydrogel-coating of the cell increases.

## Biological particles

Biological particles in the methods disclosed herein include, without limitation, cells, cell beads, or nuclei. Cells, cell beads, or nuclei can be obtained or derived from a tissue sample, a subject or a cell line. The cells may be genetically engineered (e.g., transduced or transformed or transfected) with, for example, a vector construct that can be, for example, a viral vector or a vector for homologous recombination that includes nucleic acid sequences homologous to a portion of the genome of the host cell, or can be an expression vector for the expression of the polypeptides of interest. Host cells can be either untransformed cells or cells that have already been transfected with at least one nucleic acid molecule.

In some embodiments, the recombinant cell or engineered cell is a prokaryotic cell or a eukaryotic cell. In some embodiments, the cell is *ex vivo.* In some embodiments, the cell is *in vitro.* In some embodiments, the engineered cell is a eukaryotic cell. In some embodiments, the engineered cell is an animal cell or a yeast cell. In some embodiments, the animal cell is a mammalian cell. In some embodiments, the animal cell is a human cell. In some embodiments, the cell is a non-human primate cell. In some embodiments, the mammalian cell is an immune cell, a neuron, an epithelial cell, and endothelial cell, or a stem cell. In some embodiments, the cell is a stem cell. In some embodiments, the cell is a hematopoietic stem cell.

In some embodiments, the recombinant cell or engineered cell is an immune cell, *e.g.,* a lymphocyte (*e.g.,* a T cell or NK cell), or a dendritic cell. In some embodiments, the immune cell is a B cell, a monocyte, a natural killer (NK) cell, a natural killer T (NKT) cell, a basophil, an eosinophil, a neutrophil, a dendritic cell, a macrophage, a regulatory T cell, a helper T cell (TH), a cytotoxic T cell ($T_{CTL}$), or other T cell. In some embodiments, the immune cell is a T lymphocyte. In some embodiments, the cell is a precursor T cell or a T regulatory (Treg) cell. In some embodiments, the cell is a CD34+, CD8+, or a CD4+ cell. In some embodiments, the cell is a CD8+ T cytotoxic lymphocyte cell selected from the

group consisting of naive CD8+ T cells, central memory CD8+ T cells, effector memory CD8+ T cells, and bulk CD8+ T cells. In some embodiments of the cell, the cell is a CD4+ T helper lymphocyte cell selected from the group consisting of naive CD4+ T cells, central memory CD4+ T cells, effector memory CD4+ T cells, and bulk CD4+ T cells. In some embodiments, the cell can be obtained by leukapheresis performed on a sample obtained from an individual. In some embodiments, the subject is a human patient.

## Labeling of Biological Particles

The present disclosures provide methods for labeling of biological particles (e.g., cells, cell beads, or nuclei) as part of the biological particle selection workflows described herein. For example, a single or integrated process workflow may permit the labeling of cellular analytes of biological particles (e.g., cells or nuclei) for the purpose of identifying, enriching or selecting such labeled biological particles. The labeling agents will generally be capable of or configured to interact with or couple to a cell or nuclear membrane or a feature at the cell or nuclear membrane (*e.g.,* a surface protein) and will comprise a crosslink-catalyzing moiety (*e.g.,* a crosslink-catalyzing enzyme, such as HRP, as described elsewhere herein) that allows downstream biological particle (e.g., cell or nucleus) selection. The term "labeling agent", as used herein generally refers to an agent capable of interacting with some part of a biological particles, such as a cell or a nucleus) including, without limitation, the cell or nuclear membrane, a molecule on and/or within the cell or nuclear membrane, an intracellular molecule of the cell, etc. The interaction between the agent and some part of the cell, cell bead, or nucleus may be a covalent interaction or a non-covalent interaction, a reversible interaction, or an irreversible interaction. The agent may be specific to some part of the cell, cell bead, or nucleus including, without limitation, a biological molecule of the cell, cell bead, or nucleus (*e.g.,* a polypeptide, a nucleic acid, a lipid, etc.). In some embodiments, the labeling agent may have specificity to a biological target, such as an antibody or an antibody fragment.

In the methods described herein, one or more labelling agents capable of binding to or otherwise coupling to one or more biological particle (e.g., cell, cell bead, or nucleus) features may be used to label such features. In some instances, biological particle features include cell surface features and nuclear features. Cell surface features may include, but are not limited to, a receptor, an antigen, a cell surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. Nuclear features may include, without limitation, the nuclear membrane or a nuclear membrane protein or any other nuclear protein.

In at least one embodiment, the cell feature is cell surface protein such as a cluster of differentiation (CD) protein. A wide range of CD proteins and their cognate antibodies are known in the art. CD cell-surface proteins and their anti-CD antibodies are commonly used in identifying, labelling, sorting, and selecting specific cell types, *e.g.,* using flow cytometry. It is contemplated that any of the CD cell surface proteins known in the art and their associated anti-CD antibodies can be used for cell labelling or decorating with crosslink-catalyzing moieties according to the methods of the present disclosure. For example, any of the following CD cell surface protein markers for specific cell types shown in Table 1 below can be used for labelling cells with accordance with the methods of the present disclosure.

**TABLE 1**

| Specific Cell Type | CD Surface Protein Markers |
|---|---|
| Stem Cells | CD34+, CD31-, CD117 |
| Leukocytes | CD45+ |
| Granulocyte | CD45+, CD11b, CD15+, CD24+, CD114+, CD182+ |
| Monocyte | CD4, CD45+, CD14+, CD114+, CD11a, CD11b, CD91+,CD16+ |
| T lymphocyte | CD45+, CD3+ |
| T helper cell | CD45+, CD3+, CD4+ |
| T regulatory cell | CD4, CD25, FOXP3 |
| Cytotoxic T cell | CD45+, CD3+, CD8+ |
| B lymphocyte | CD45+, CD19+, CD20+, CD24+, CD38, CD22 |
| Thrombocyte | CD45+, CD61+ |
| Natural killer cell | CD16+, CD56+, CD3-, CD31, CD30, CD38 |

In some instances, cell features can include intracellular analytes, such as proteins, protein modifications (*e.g.,* phosphorylation status or other post-translational modifications), nuclear proteins, nuclear membrane proteins, or any combination thereof. A labelling agent can include, but is not limited to, a protein, a peptide, an antibody (or an epitope binding fragment thereof), an antigen, an antigen fragment, a lipophilic moiety (such as cholesterol), a cell surface receptor binding molecule, a receptor ligand, a small molecule, a bi-specific antibody, a B-cell receptor engager, a probody, an aptamer, a monobody, an affimer, a Darpin, a protein scaffold, and a target antigen, or any combination thereof.

In some embodiments, the labelling agents can include (*e.g.,* are attached to) a reporter oligonucleotide that is indicative of the cell surface feature to which the binding group binds. For example, the reporter oligonucleotide can include a barcode sequence that permits identification of the labelling agent. For example, a labelling agent that is specific to one type of cell feature (*e.g.,* a first cell surface feature) can have a first reporter oligonucleotide coupled thereto, while a labelling agent that is specific to a different cell feature (*e.g.,* a second cell surface feature) can have a different reporter oligonucleotide coupled thereto. For a description of exemplary labelling agents, reporter oligonucleotides, and methods of use, see, *e.g.,* U.S. Pat. 10,550,429; U.S. Pat. Pub. 20190177800; and U.S. Pat. Pub. 20190367969.

As discussed above, in a particular example, a library of potential cell feature labelling agents can be provided, where the respective cell feature labelling agents are associated with nucleic acid reporter molecules, such that a different reporter oligonucleotide sequence is associated with each labelling agent capable of binding to a specific cell feature. In some embodiments, the cell feature labelling agents comprise a target antigen and a fragment of the target antigen, as disclosed herein. In some embodiments, the cell feature labelling agents comprise a plurality of non-overlapping fragments of a target antigen. In other aspects, different members of the library can be characterized by the presence of a different oligonucleotide sequence label. For example, an antibody capable of binding to a target protein can have associated with it a first reporter oligonucleotide sequence, while an antibody, (which may be the same antibody), capable of binding to a fragment or fragments of the target antigen can have a different, (or additional if the same antibody), reporter oligonucleotide sequence(s) associated with it. The presence of the particular oligonucleotide sequence(s) can be indicative of the presence of a particular antibody or cell feature which can be recognized or bound by the particular antibody.

Labelling agents capable of binding to or otherwise coupling to one or more cells can be used to characterize a cell as belonging to a particular set of cells. For example, labelling agents can be used to label a sample of cells, *e.g.,* to provide a sample index. For other example, labelling agents can be used to label a group of cells belonging to a particular experimental condition. In this way, a group of cells can be labeled as different from another group of cells. In an example, a first group of cells can originate from a first sample and a second group of cells can originate from a second sample. Labelling agents can allow the first group and second group to have a different labeling agent (or reporter oligonucleotide associated with the labeling agent). This can, for example, facilitate multiplexing, where cells of the first group and cells of the second group can be labeled separately and then pooled together for downstream analysis. The downstream detection of a label can indicate analytes as belonging to a particular group of cells.

For example, a reporter oligonucleotide can be linked to an antibody or an epitope binding fragment thereof, and labeling a cell can include subjecting the antibody-linked barcode molecule or the epitope binding fragment-linked barcode molecule to conditions suitable for binding the antibody to a molecule present on a surface of the cell. The binding affinity between the antibody or the epitope binding fragment thereof and the molecule present on the surface can be within a desired range to ensure that the antibody or the epitope binding fragment thereof remains bound to the molecule. For example, the binding affinity can be within a desired range to ensure that the antibody or the epitope binding fragment thereof remains bound to the molecule during various sample processing steps, such as partitioning and/or nucleic acid amplification or extension. A dissociation constant (Kd) between the antibody or an epitope binding fragment thereof and the molecule to which it binds can be less than about 100 μM, 90 μM, 80 μM, 70 μM, 60 μM, 50 μM, 40 μM, 30 μM, 20 μM, 10 μM, 9 μM, 8 μM, 7 μM, 6 μM, 5 μM, 4 μM, 3 μM, 2 μM, 1 μM, 900 nM, 800 nM, 700 nM, 600 nM, 500 nM, 400 nM, 300 nM, 200 nM, 100 nM, 90 nM, 80 nM, 70 nM, 60 nM, 50 nM, 40 nM, 30 nM, 20 nM, 10 nM, 9 nM, 8 nM, 7 nM, 6 nM, 5 nM, 4 nM, 3 nM, 2 nM, 1 nM, 900 pM, 800 pM, 700 pM, 600 pM, 500 pM, 400 pM, 300 pM, 200 pM, 100 pM, 90 pM, 80 pM, 70 pM, 60 pM, 50 pM, 40 pM, 30 pM, 20 pM, 10 pM, 9 pM, 8 pM, 7 pM, 6 pM, 5 pM, 4 pM, 3 pM, 2 pM, or 1 pM. For example, the dissociation constant can be less than about 10 μM. In some embodiments, the antibody or epitope binding fragment thereof has a desired off rate (koff), such that the antibody or antigen binding fragment thereof remains bound to the target antigen or antigen fragment during various sample processing steps.

In another example, a reporter oligonucleotide can be coupled to a cell-penetrating peptide (CPP), and labeling cells can include delivering the CPP coupled reporter oligonucleotide into a biological particle. Labeling biological particles can include delivering the CPP conjugated oligonucleotide into a cell and/or cell bead by the cell-penetrating peptide. A CPP that can be used in the methods provided herein can include at least one non-functional cysteine residue, which can be either free or derivatized to form a disulfide link with an oligonucleotide that has been modified for such linkage. Non-limiting examples of CPPs that can be used in embodiments herein include penetratin, transportan, plsl, TAT(48-60),

pVEC, MTS, and MAP. Cell-penetrating peptides useful in the methods provided herein can have the capability of inducing cell penetration for at least about 30%, 40%, 50%, 60%, 70%, 80%, 90%, 95%, 96%, 97%, 98%, 99%, or 100% of cells of a cell population. The CPP can be an arginine-rich peptide transporter. The CPP can be Penetratin or the Tat peptide. In another example, a reporter oligonucleotide can be coupled to a fluorophore or dye, and labeling cells can include subjecting the fluorophore-linked barcode molecule to conditions suitable for binding the fluorophore to the surface of the cell. In some instances, fluorophores can interact strongly with lipid bilayers and labeling cells can include subjecting the fluorophore-linked barcode molecule to conditions such that the fluorophore binds to or is inserted into a membrane of the cell. In some cases, the fluorophore is a water-soluble, organic fluorophore. In some instances, the fluorophore is Alexa 532 maleimide, tetramethylrhodamine-5-maleimide (TMR maleimide), BODIPY-TMR maleimide, Sulfo-Cy3 maleimide, Alexa 546 carboxylic acid/succinimidyl ester, Atto 550 maleimide, Cy3 carboxylic acid/succinimidyl ester, Cy3B carboxylic acid/succinimidyl ester, Atto 565 biotin, Sulforhodamine B, Alexa 594 maleimide, Texas Red maleimide, Alexa 633 maleimide, Abberior STAR 635P azide, Atto 647N maleimide, Atto 647 SE, or Sulfo-Cy5 maleimide. See, *e.g.,* Hughes L D, et al. PLoS One. 2014 Feb. 4; 9(2):e87649 for a description of organic fluorophores.

A reporter oligonucleotide can be coupled to a lipophilic molecule, and labeling cells can include delivering the nucleic acid barcode molecule to a membrane of a cell or a nuclear membrane by the lipophilic molecule. Lipophilic molecules can associate with and/or insert into lipid membranes such as cell membranes and nuclear membranes. In some cases, the insertion can be reversible. In some cases, the association between the lipophilic molecule and the cell or nuclear membrane can be such that the membrane retains the lipophilic molecule (*e.g.,* and associated components, such as nucleic acid barcode molecules, thereof) during subsequent processing (*e.g.,* partitioning, cell permeabilization, amplification, pooling, etc.). The reporter nucleotide can enter into the intracellular space and/or a cell nucleus. In some embodiments, a reporter oligonucleotide coupled to a lipophilic molecule will remain associated with and/or inserted into lipid membrane (as described herein) via the lipophilic molecule until lysis of the cell occurs, *e.g.,* inside a partition. Exemplary embodiments of lipophilic molecules coupled to reporter oligonucleotides are described in PCT/US2018/064600.

A reporter oligonucleotide can be part of a nucleic acid molecule including any number of functional sequences, as described elsewhere herein, such as a target capture sequence, a random primer sequence, and the like, and coupled to another nucleic acid molecule that is, or is derived from, the analyte.

Prior to partitioning, the cells can be incubated with the library of labelling agents, that can be labelling agents to a broad panel of different cell features, *e.g.,* receptors, proteins, etc., and which include their associated reporter oligonucleotides. Unbound labelling agents can be washed from the cells, and the cells can then be co-partitioned (*e.g.,* into droplets or wells) along with partition-specific barcode oligonucleotides (*e.g.,* attached to a support, such as a bead or gel bead) as described elsewhere herein. As a result, the partitions can include the cell or cells, as well as the bound labelling agents and their known, associated reporter oligonucleotides.

In other instances, *e.g.,* to facilitate sample multiplexing, a labelling agent that is specific to a particular cell feature can have a first plurality of the labelling agent (*e.g.,* an antibody or lipophilic moiety) coupled to a first reporter oligonucleotide and a second plurality of the labelling agent coupled to a second reporter oligonucleotide. For example, the first plurality of the labeling agent and second plurality of the labeling agent can interact with different cells, cell populations or samples, allowing a particular report oligonucleotide to indicate a particular cell population (or cell or sample) and cell feature. In this way, different samples or groups can be independently processed and subsequently combined together for pooled analysis (*e.g.,* partition-based barcoding as described elsewhere herein). See, *e.g.,* U.S. Pat. Pub. 20190323088.

In some embodiments, to facilitate sample multiplexing, individual samples can be stained with lipid tags, such as cholesterol-modified oligonucleotides (CMOs, see, *e.g.,* FIG. 7), anti-calcium channel antibodies, or anti-ACTB antibodies. Non-limiting examples of anti-calcium channel antibodies include anti-KCNN4 antibodies, anti-BK channel beta 3 antibodies, anti-a1B calcium channel antibodies, and anti-CACNA1A antibodies. Examples of anti-ACTB antibodies suitable for the methods of the disclosure include, but are not limited to, mAbGEa, ACTN05, AC-15, 15G5A11/E2, BA3R, and HHF35.

As described elsewhere herein, libraries of labelling agents can be associated with a particular cell feature as well as be used to identify analytes as originating from a particular cell population, or sample. Cell populations can be incubated with a plurality of libraries such that a cell or cells include multiple labelling agents. For example, a cell can include coupled thereto a lipophilic labeling agent and an antibody. The lipophilic labeling agent can indicate that the cell is a member of a particular cell sample, whereas the antibody can indicate that the cell includes a particular analyte. In this manner, the reporter oligonucleotides and labelling agents can allow multi-analyte, multiplexed analyses to be performed.

In some instances, these reporter oligonucleotides can include nucleic acid barcode sequences that permit identification of the labelling agent which the reporter oligonucleotide is coupled to. The use of oligonucleotides as the reporter can provide advantages of being able to generate significant diversity in terms of sequence, while also being readily attachable to most biomolecules, *e.g.,* antibodies, *etc.,* as well as being readily detected, e.g., using sequencing

or array technologies.

Attachment (coupling) of the reporter oligonucleotides to the labelling agents can be achieved through any of a variety of direct or indirect, covalent or non-covalent associations or attachments. For example, reporter oligonucleotides can be covalently attached to a portion of a labelling agent (such a protein, *e.g.,* an antigen or antigen fragment, an antibody or antibody fragment) using chemical conjugation techniques (*e.g.,* Lightning-Link® antibody labelling kits available from Innova Biosciences), as well as other non-covalent attachment mechanisms, *e.g.,* using biotinylated antibodies (or biotinylated antigens, or biotinylated antigen fragments) and oligonucleotides (or beads that include one or more biotinylated linker, coupled to oligonucleotides) with an avidin or streptavidin linker. Antibody and oligonucleotide biotinylation techniques are available. See, *e.g.,* Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715. Likewise, protein and peptide biotinylation techniques have been developed and are readily available. See, *e.g.,* U.S. Pat. No. 6,265,552. Furthermore, click reaction chemistry such as a Methyltetrazine-PEG5-NHS Ester reaction, a TCO-PEG4-NHS Ester reaction, or the like, can be used to couple reporter oligonucleotides to labelling agents. Commercially available kits, such as those from Thunderlink and Abcam, and techniques common in the art can be used to couple reporter oligonucleotides to labelling agents as appropriate. In another example, a labelling agent is indirectly (*e.g.,* via hybridization) coupled to a reporter oligonucleotide including a barcode sequence that identifies the label agent. For instance, the labelling agent can be directly coupled (*e.g.,* covalently bound) to a hybridization oligonucleotide that includes a sequence that hybridizes with a sequence of the reporter oligonucleotide. Hybridization of the hybridization oligonucleotide to the reporter oligonucleotide couples the labelling agent to the reporter oligonucleotide. In some embodiments, the reporter oligonucleotides are releasable from the labelling agent, such as upon application of a stimulus. For example, the reporter oligonucleotide can be attached to the labeling agent through a labile bond (*e.g.,* chemically labile, photolabile, thermally labile, *etc.)* as generally described for releasing molecules from supports elsewhere herein. In some instances, the reporter oligonucleotides described herein can include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer biding sequence (such as an R1, R2, or partial R1 or R2 sequence).

In some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (*e.g.,* the label can be conjugated to a molecule that can bind to the labelling agent or reporter oligonucleotide). In some cases, a label is conjugated to an oligonucleotide that is complementary to a sequence of the reporter oligonucleotide, and the oligonucleotide may be allowed to hybridize to the reporter oligonucleotide.

**FIG. 20** depicts an example of a cell labeling workflow for cell selection by gelation. A mixed cell population is labeled (or "decorated") with a labeling agent conjugated to a catalyzing moiety - an antibody conjugated to a crosslink-catalyzing moiety, horseradish peroxidase (HRP). The antibody binds to those cells that express a first cell feature of interest (*e.g.,* a first cell surface protein) to provide a mixture of labeled cells and unlabeled cells. The mixture of cells is partitioned along with other reagents for selective gelation, such as a linear polymer comprising a crosslink-precursor moiety. A catalyzing agent or co-substrate for selective gelation may also be provided in the partition. In addition, where the crosslink-catalyzing moiety is coupled to the antibody via a cleavable linker, a cleaving agent may be introduced into the partition to cleave the crosslink-catalyzing moiety (see **FIG. 12).** As depicted in **FIG. 20,** cells expressing the first cell feature will undergo selective gelation within the partition as the horseradish peroxidase (HRP) catalyzes the formation of crosslinks between the linear polymers (see **FIG. 11).** As further depicted in **FIG. 20,** the selectively gelled cells and non-gelled cells are removed from the partitions and the gelled cells separated for further downstream processing. Methods of separating gelled cells from non-gelled cells include gradient-based methods, (*e.g.,* Percoll gradient), centrifugation, and the use of magnetic nanoparticles, which can be incorporated into gel substrates (see (Ilg, Patrick, Soft Matter, 09 April 2013, Issue 13, p. 3439-3682). In one embodiment, magnetic nanoparticles are incorporated into the gel layer that is formed on a cell, e.g., through the reaction involving a linear polymer comprising a crosslink-precursor moiety and a catalyzing agent or co-substrate as described herein. In another embodiment, the magnetic nanoparticles are incorporated into the gel layer in a partition, e.g., a droplet or a well. For a description of methods, compositions, and systems for the gelation or encapsulation of cells and subsequent processing of such cells, see, *e.g.,* U.S. Pat. 10,428,326 and U.S. Pat. Pub. 20190100632.

**FIG. 21** depicts another exemplary workflow in accordance with some non-limiting embodiments of the disclosure that can be employed for the identification and or characterization of novel antigen-binding molecules (*e.g.,* BCR, TCR, and fragments thereof) by selective gelation of antigen-binding cells. A mixed immune cell population is labeled (or "decorated") with a labeling agent conjugated to a catalyzing moiety - a target antigen conjugated to a crosslink-catalyzing moiety, horseradish peroxidase (HRP). The target antigen binds to those cells that express a first cell feature of interest (*e.g.,* an antibody) to provide a mixture of labeled immune cells and unlabeled immune cells. The mixture of immune cells is partitioned along with other reagents for selective gelation, such as a linear polymer comprising a

crosslink-precursor moiety. A catalyzing agent or co-substrate for selective gelation may also be provided in the partition. In addition, where the crosslink-catalyzing moiety is coupled to the antigen via a cleavable linker, a cleaving agent may be introduced into the partition to cleave the crosslink-catalyzing moiety (see **FIG. 12).** As depicted in **FIG. 21,** cells expressing the first cell feature will undergo selective gelation within the partition as the horseradish peroxidase (HRP) catalyzes the formation of crosslinks between the linear polymers (see **FIG. 11).** As further depicted in **FIG. 21,** the selectively gelled cells and non-gelled cells are removed from the partitions and the gelled cells separated for further downstream processing. Methods of separating gelled cells from non-gelled cells include gradient-based methods, (*e.g.,* Percoll gradient), centrifugation, and the use of magnetic nanoparticles. For a description of methods, compositions, and systems for the gelation or encapsulation of cells and subsequent processing of such cells, see, *e.g.,* U.S. Pat. 10,428,326 and U.S. Pat. Pub. 20190100632.

In one aspect, the present disclosure provides methods of cell selection by selective gelation of cells of interest. In one embodiment, the method comprises providing a plurality of cells, which comprise a labelled cell (or a plurality of labelled cells) and an unlabelled cell (or a plurality of unlabelled cells). The labelled cell comprises a cell labelling agent, which comprises a catalyzing moiety. The unlabelled cell does not comprise or is free of the cell labelling agent. In another embodiment, the labelled cell may comprise the first cell labelling agent, e.g., a first agent, such as a first antibody or antigen, capable of coupling to a first cell surface protein (including a first cell surface receptor), and a second cell labelling agent, e.g., a second agent, such as a second antibody or antigen, capable of coupling to a second cell surface protein (including a second cell surface receptor).

In other embodiments, the method comprises partitioning a plurality of cells, which comprise the labelled cell and the unlabelled cell, to provide a plurality of partitions, wherein said plurality of partitions comprises a first partition comprising said labelled cell and a plurality of linear polymers. The plurality of partitions may further comprise a second partition comprising the unlabeled cell and a plurality of linear polymers. In addition, the plurality of partitions may comprise additional partitions comprising an additional partition comprising an additional labelled cell and a plurality of linear polymers and/or an additional unlabelled cell and a plurality of linear polymers.

In further embodiments, the method further comprises subjecting a partition (e.g., the first partition, second partition, additional partition(s), etc.) to conditions to allow formation of a polymer coating on a labelled cell within the partition. The formation may be catalyzed in the partition by the catalyzing moiety using the plurality of linear polymers. In another embodiment, the method further comprises removing said plurality of cells from said plurality of partitions to provide a mixture of cells comprising said polymer coated labelled cell from said first partition and said un-labelled cell from said second partition. In one embodiment, the removing step comprises pooling cells from the partitions. In the case of droplets in an emulsion, the removing step comprises breaking the droplets and pooling the contents. In the case of a well or array of wells, the removing step comprises extracting the cell(s) from each well. In another embodiment, the method further comprises separating said polymer coated labelled cell(s) from said un-labelled cell(s) to allow further processing of said polymer coated labelled cell(s). The cell labeling methods are further depicted in **FIGS. 20-21.**

## Generation of cells decorated with crosslink-catalyzing moieties

Step (a) of the general method of the present disclosure uses a cell that has been modified (or "decorated") by attachment of numerous crosslink-catalyzing moieties to the cell’s membrane. The attachment to the membrane is through a "membrane anchor moiety" which refers to a chemical or biochemical moiety that is capable of a specific, strong binding interaction with a cell membrane. A range of membrane anchor moieties are known in the art that can be used in the methods of the present disclosure. Among the known membrane anchor moieties useful in the methods of the present disclosure are: a "Biocompatible Anchor for cell Membrane" (or "BAM") moiety; an antibody to a cell membrane protein; a cholesterol-oligonucleotide moiety; a 3’-cholesterol-TEG moiety; and a cholesterol-decorated polymer. Other membrane anchor moieties are described herein, also referred to as labeling agents for cells.

In at least one exemplary embodiment, the membrane anchor moiety used in the methods of the present disclosure is a compound having an "oleyl-PEG-X" structure, which is also referred to as a "BAM" moiety for "Biocompatible Anchor for cell Membrane." A generic BAM structure is shown in Scheme 1 below.

## Scheme 1

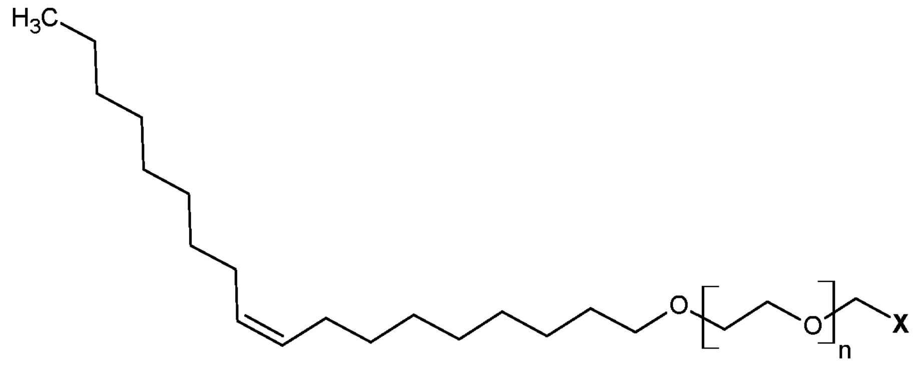

The oleyl moiety portion of the BAM compound is able to enter the plasma membrane of a cell and form strong hydrophobic interaction that anchors the compound to the cell. The number of PEG moieties of the BAM compound (n) can vary, and thereby alter the distance between the cell membrane and the anchored biomolecule as desired. The "X" moiety is a reactive group, such as N-hydroxy-succinimide (NHS), that can form an attachment to a biomolecule, such as an enzyme.

In at least one embodiment, the BAM moiety comprising an oleyl moiety is an oleyl-O-$(CH_2CH_2O)_n$-CO-$CH_2CH_2$-COO moiety, wherein the number of polyethylene glycol (PEG) groups, n, is such that the moiety has a molecular weight of at least 2000, at least 4000, or at least 8000. In at least one embodiment, the number of PEG moieties, n, is 40 and the reactive group is NHS as shown in Scheme 2 below.

## Scheme 2

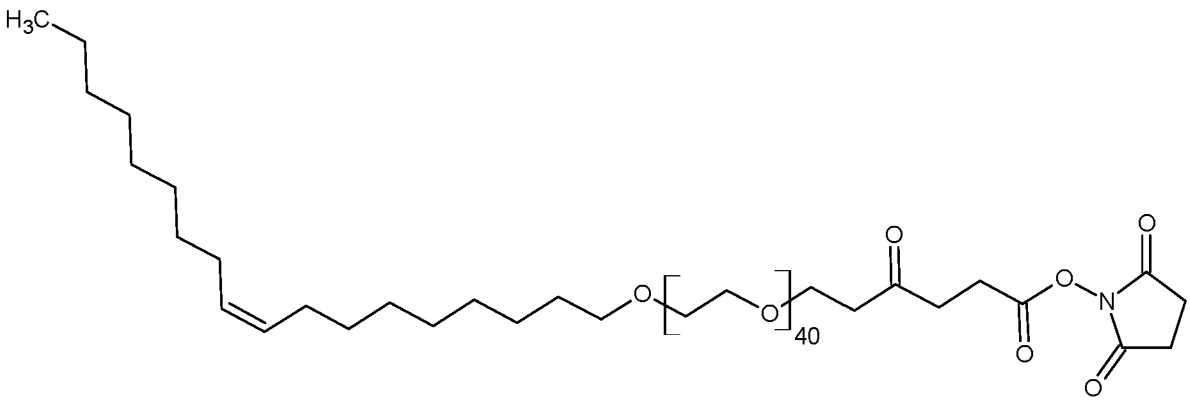

**FIG. 1** depicts an exemplary scheme for preparing a BAM moiety attached to a horseradish peroxidase ("HRP") enzyme moiety through a cleavable linker. In a first step, the HRP enzyme, which previously has been modified at an available amine side chain with an N-hydroxy-succinimide group (NHS), is reacted with cystamine dihydrochloride to provide an enzyme modified with a cleavable disulfide linker. In a second step, the free amine of this cleavable disulfide linker attached to the HRP enzyme moiety is reacted with a BAM compound to yield the BAM-moiety attached to the HRP enzyme moiety through a cleavable linker. **FIG. 2** depicts an exemplary third step of using the BAM-linker-enzyme moieties prepared as depicted in **FIG. 1** to prepare a cell modified (or decorated) with enzyme moieties.

Generally, a cleavable linker moiety useful in the methods of the present disclosure can include any labile bond that can be introduced into a linker and selectively cleaved a chemical or physical stimulus. Non-limiting examples of labile bonds that may be used as cleavable linker moieties include a disulfide linkage (*e.g.,* cleavable with a reducing agent), an ester linkage (*e.g.,* cleavable with an acid, a base, or hydroxylamine), a carbamate linkage (*e.g.,* cleavable with diethylenetriamine "DETA"), a vicinal diol linkage (*e.g.,* cleavable via sodium periodate), a Diels-Alder linkage (*e.g.,* cleavable via heat), a sulfone linkage (*e.g.,* cleavable via a base), a silyl ether linkage (*e.g.,* cleavable via an acid), a glycosidic linkage (*e.g.,* cleavable via an amylase), a peptide linkage (*e.g.,* cleavable via a protease), or a phosphodiester linkage (*e.g.,* cleavable via a nuclease). A range of linkers comprising cleavable moieties that can be used in the methods

f the present disclosure are known. See *e.g.,* US Pat. Publ. Nos. 2019/0100632A1, and 2019/0233878A1. In at least one embodiment, cleavable linker attaching the membrane anchor moiety to the crosslink catalyzing moiety is selected from a disulfide spacer moiety; a carbamate spacer moiety; a photocleavable spacer; and UDG-cleavable spacer.

One of ordinary skill will recognize that the steps of **FIGS. 1** and **2** can be altered and still provide the same final enzyme modified cell. For example, the BAM moieties can be used to decorate the cell and then the enzyme moiety with the cleavable linker can be reacted with the free reactive group of the BAM moieties already attached to the cell membrane. It is also contemplated that the cleavable linker can be first attached to the BAM moiety, which is then used to decorate the cell, before reacting with the free amine of an enzyme to provide the decorated cell.

In at least one exemplary embodiment, the membrane anchor moiety used in the methods of the present disclosure can be an oligonucleotide conjugated to a lipophilic moiety, *e.g.,* a cholesterol-oligonucleotide moiety. For example, an oligonucleotide can be modified with a 3'-cholesterol-TEG moiety using standard automated oligonucleotide synthesis and commercially available phosphoramidite reagents (*e.g.,* available from Integrated DNA Technologies, USA). Like the oleyl moiety of BAM, the 3'-cholesterol moiety is able to enter the plasma membrane of a cell and form strong hydrophobic interaction that anchors the oligonucleotide to the cell surface. The oligonucleotide can also be prepared with a 5'-biotin moiety using a commercially available 5'-biotin phosphoramidite reagent (*e.g.,* available from Integrated DNA Technologies, USA) and standard automated oligonucleotide synthesis. **FIG. 3** depicts a schematic of an oligonucleotide modified with a 5'-biotin moiety and a 3'-cholesterol moiety being used to decorate a cell. The resulting cell has biotin moieties available on its surface for further modification by streptavidin linked moieties, such as a streptavidin modified enzyme. **FIG. 4** provides a schematic depiction of the further modification of a cell previously modified with 5'-biotin-oligonucleotides with HRP-streptavidin moieties. The conjugation of streptavidin to proteins, such as HRP, is well known in the art. The streptavidin-modified HRP forms an extremely strong non-covalent binding interaction with the biotin moieties linked to the surface of the cell via the oligonucleotide resulting in an HRP decorated cell. Further, the oligonucleotide can be prepared with a sequence that includes a uracil (U) base, thereby allowing for facile enzymatic cleavage of the oligonucleotide by the enzyme Uracil-DNA glcosylase (UDG). Alternatively, other oligonucleotide sequences are known that when incorporated allow for facile enzymatic cleavage.

In at least one embodiment, it is contemplate that a cell can be modified with enzymes via specific binding of an enzyme-antibody conjugate to cell surface antigens. **FIGS. 5** and **6** schematically depict an exemplary process for generating an enzyme decorated cell via antibody binding to cell surface antigens. As shown in **FIG. 5,** biotin conjugate of the desired antibody is incubated with the cell under conditions allowing specific binding to the cell surface antigen targeted by the antibody. As shown in **FIG. 6,** the biotin-antibody decorated cell is subsequently treated with a streptavidin-enzyme conjugate which binds strongly to the biotin moieties on the cell surface resulting in an enzyme decorated cell.

One advantage of using an enzyme-antibody conjugate to decorate a cell is that it allows for selective targeting of cell types based on cell surface protein antigen expression. In at least one embodiment, an antibody is used that binds to a cell surface protein, such as a cluster of differentiation (CD) protein, that is expressed (or over-expressed) only on the surface of a specific cell type. As described elsewhere herein, a wide range of antibodies are known in the art that bind to specific cell surface proteins or antigens (*e.g.,* anti-CD antibodies). Such antibodies are commercially available or can be generated using routine methods for antibody preparation. By using an antibody that targets the cell type specific surface receptor, only the targeted cell type is decorated with biotin-antibody conjugates, and then the crosslink-catalyzing moiety, such as the enzyme, HRP. Thus, it is possible to selectively decorate only a single cell type in a pool of cells, and then selectively form a hydrogen-coating around that cell type, using the gelation methods described herein. An exemplary workflow for selectively gelating specific cell types is depicted in **FIG. 20** as described elsewhere herein.

A range of cross-link catalyzing moieties that can be used in the methods of the present disclosure are known in the art. Among the known crosslink-catalyzing moieties useful in the methods are a number of enzymes including, but are not limited to, a peroxidase (*e.g.,* horseradish peroxidase or "HRP"), a laccase, a tyrosinase, or a transglutaminase. Peroxidases, such as HRP, and related enzymes of class E.C. 1.11.1.7, catalyze the oxidation of wide range of organic substrates using the co-substrate hydrogen peroxide ($H_2O_2$). The HRP reaction in the presence of $H_2O_2$ and a substrate with phenol moieties can result in the formation of a phenolic-polymer matrix with C-C bonds crosslinking adjacent phenol moieties. **FIG. 7** depicts an exemplary phenolic crosslinking reaction that occurs between linear polymers (*e.g.,* polyacrylamide) modified with phenol groups in the presence of HRP and the co-substrate.

Similar to HRP, laccase (E.C. 1.10.3.2) catalyzes the oxidation of phenolic substrates formation of crosslinks between phenol-decorated polymers resulting in a polymer matrix. See *e.g.,* Jus et al., "Enzymatic cross-linking of gelatin with laccase and tyrosinase," Biocatalysis and Biotransformation, Vol. 30, 2012, pp. 86-95. Tyrosinase (E.C. 1.14.18.1) catalyzes the formation of crosslinks between phenol-decorated and amine-decorated polymers resulting in a polymer matrix. See *e.g.,* Kim et al., "Tissue adhesive, rapid forming, and sprayable ECM hydrogel via recombinant tyrosinase crosslinking," Biomaterials, 2 May 2018, 178:401-412. Transglutaminase (E.C. 2.3.2.13) catalyzes the formation of crosslinks between glutamine-decorated polymers resulting in a polymer matrix.

Accordingly, in at least one embodiment of the methods of the present disclosure, the crosslink-catalyzing

moiety comprises an enzyme selected from a peroxidase (E.C. 1.11.1.7), a transglutaminase (E.C. 2.3.2.13), a tyrosinase (E.C. 1.14.18.1), and a laccase (E.C. 1.10.3.2). As noted above, the particular substrate and co-substrate(s) used by each enzyme to form crosslinks is known in the art.

In the case of peroxidases, crosslinking of phenolic substrates requires the addition of a compound comprising a peroxide moiety, such as $H_2O_2$, that acts as a co-substrate that initiates the crosslink forming catalysis. Accordingly, in at least one embodiment of the methods of present disclosure, the crosslink-catalyzing moiety is a peroxidase (*e.g.,* HRP), the crosslink-precursor moieties are phenol groups, and the crosslink-forming initiator is $H_2O_2$. The presence of the co-substrate $H_2O_2$ in an aqueous solution along with a peroxidase and phenol-modified linear polymers initiates the enzyme-catalyzed crosslinking reaction that forms the hydrogel.

It is contemplated that the $H_2O_2$ usually will be delivered to a partition simultaneous with the substrate and enzyme, or after the partition has been formed containing a mixture of the peroxidase and phenolic substrate. The timing of delivery of cells and reagents during formation of partitions can be controlled using microfluidic systems, as is known in the art and described elsewhere herein. In at least one embodiment of the method of present disclosure, the crosslink-forming peroxide co-substrate can be delivered to an aqueous partition after it is formed, for example by diffusion into the partition from an immiscible phase outside the partition. It is contemplated in one embodiment that a peroxide co-substrate (*e.g.,* $H_2O_2$) can be contained in a micelle and can be delivered to an aqueous partition via micelle-mediated transport into the partition (see *e.g.,* WO20/167862).

Oxidoreductases, such as laccase and tyrosinases, can also be used as crosslink-catalyzing moieties in the methods of the present disclosure. These oxidoreductases, however, do not require a peroxide, such as $H_2O_2$ as a co-substrate, but instead utilize molecular oxygen ($O_2$) as the co-substrate to drive the catalytic reaction crosslinking phenol moieties. Laccase interacts with $O_2$ to form a bound peroxide complex capable of catalyzing the formation of di-phenol crosslinks. The oxidoreductase, tyrosinase utilizes a two-step catalytic process using $O_2$ to convert phenol to catechol and then further oxidizing the catechol to a reactive quinone moiety, which readily forms a covalent crosslink.

It is also contemplated that the crosslink-catalyzing moiety is a compound that is not an enzyme. For example, hematin is a known non-enzymatic heme compound that is capable of catalyzing the crosslinking of phenol containing polymers to form hydrogels. See *e.g.,* Sakai et al., "Hematin is an Alternative Catalyst to Horseradish Peroxidase for In Situ Hydrogelation of Polymers with Phenolic Hydroxyl Groups In Vivo," Biomacromolecules 11(8):2179-83 (Aug. 2010). Like the enzyme, HRP, hematin requires the presence of a peroxide co-reagent, such as $H_2O_2$, to initiate and maintain the crosslink-catalyzing reaction. Umbelliferone is another non-enzymatic coumarin-type compound that is capable of initiating crosslinking reactions to form hydrogels. See *e.g.,* Hickey et al., "Cross-Coupling of Amide and Amide Derivatives to Umbelliferone Nonaflates: Synthesis of Coumarin Derivatives and Fluorescent Materials," J. Org. Chem. 2020, 85, 12, 7986-7999. Accordingly, in at least one embodiment, the crosslink-catalyzing moiety is a non-enzymatic moiety, optionally selected from hematin and umbelliferone.

## Hydrogel matrix formation

Generally, the methods of the present disclosure relate to the formation of a hydrogel-coating around a cell contained in a partition. The hydrogel-coating is formed by providing in the solution around the cell, one or more linear polymers, wherein the linear polymers are modified with a chemical moiety capable of undergoing a reaction that forms a covalent crosslink (i.e., a crosslink precursor moiety) with another linear polymer in the mixture, *e.g.,* as shown in **FIG. 7.** Formation of these crosslinks between the linear polymers in a partition solution containing a cell results in the formation of a hydrogel with a cell embedded or entrapped in the matrix. Linear polymers useful in the methods of the present disclosure include, an olefin copolymer, a polyolefin, an acrylic, a polyacrylamide, a poly(oxazoline), a vinyl polymer, a polyester, a polycarbonate, a polyamide, a polyimide, a formaldehyde resin, a polyurethane, an ether polymer, a cellulosic, a thermoplastic elastomer, and a thermoplastic polyurethane. Materials and methods for forming hydrogel matrices in partitions by crosslinking linear polymers are known in the art. See *e.g.,* US Pat. Publ. Nos. 2019/0100632A1, and 2019/0233878A1.

**FIG. 8** depicts an exemplary scheme for preparing phenol-modified linear polymers capable of undergoing enzyme-catalyzed crosslinking to form a hydrogel matrix. In a first step, an aqueous monomer solution is prepared of monomers capable of forming linear polymers with modifiable side chains. In at least one embodiment the monomers are acrylamide and 3-aminopropyl methyl-acrylamide. The aminopropyl group provides a side chain that can be modified for attachment of other groups. Also included is sodium formate as a chain transfer agent to facilitate the formation of linear polymers. In at least one embodiment, a 5'-acrydite oligonucleotide is also included to provide linear polymers modified with oligonucleotides.

Linear polymers can be generated in solution via a range of polymerization methods. For example, polymerization can be initiated by initiators, or free-radical generating compounds, such as, for example, benzoyl peroxide, 2,2-azo-isobutyronitrile (AIBN), and ammonium peroxo-disulfate, or by using UV-, gamma-, or electron beam-radiation. In at least one embodiment, shown in step 2 of **FIG. 8,** linear polymers are generated from acrylamide monomers using a

VA-044 thermal initiated polymerization method.

As shown in step 3 of **FIG. 8,** in at least one embodiment, the linear polymers once formed are then modified (or functionalized) with crosslink precursor moieties which are capable of acting as a substrate for a crosslink-catalyzing moiety. In the exemplary embodiment of **FIG. 8,** the crosslink precursor moieties coupled to the linear polymers are phenol groups. The modification of the linear polymers with crosslink precursor moieties, such as phenol groups, can be carried out using any of range of known bioconjugation chemistries used for attaching biomolecules such as enzymes or antibodies to other biomolecules, polymers, and/or solid supports. Typically, the conjugation is not direct to the linear polymer side chain but includes a linker moiety between the enzyme and the amine group. A range of linkers (also referred to as spacers), including linkers comprising cleavable linker moieties, are known in the art of bioconjugation and can be used in the methods of the present disclosure. Among the known linkers useful in the methods of the present disclosure are: non cleavable alkyl linkers, 5'-thiol Modifier C6 S-S linkers, photocleavable spacers, UDG-cleavable spacers, and oligonucleotides.

In at least one embodiment of the methods of the present disclosure, the linear polymers (*e.g.,* polyacrylamide) are modified with crosslink precursor moieties (*e.g.,* phenol groups) that are attached via a cleavable linker. An exemplary reaction scheme for modifying a polyacrylamide with a phenol groups attached through a linker comprising a cleavable disulfide moiety is shown in **FIG. 9.** The disulfide linkages permit the linkers to be cleaved upon exposure to a stimulus, such as a reducing agent (*e.g.,* DTT), thereby allowing for the hydrogel matrix to be selectively degraded or dissolved. The ability to selectively degrade a hydrogel-coating that entraps a cell can provide for the hydrogel-coated cells in a variety of methods of selective cell-culturing, cell-storage, and/or cell assays. Techniques and methods for the preparation and use of degradable hydrogels in partitions is known in the art. See e.g., US Pat. Publ. Nos. 2019/0100632A1, and 2019/0233878A1.

## Two step gelation process

As is described elsewhere herein, the method of the present disclosure includes a step of cleaving the cleavable linker that attaches the crosslink-catalyzing moieties to the biological particle (e.g., a cell, cell bead or nucleus), whereby the crosslink-catalyzing moieties are released and the thickness of hydrogel-coating of the biological particle (e.g., cell, cell bead or nucleus) increases. This step of cleaving the linkers and releasing the crosslink-catalyze enzyme moieties allows for a second stage in the formation of a hydrogel-coating around a biological particle (e.g., a cell, cell bead, or nucleus). The two-stages of this process are depicted schematically in **FIGS. 10-12.** In **FIG. 10,** a cell decorated with HRP enzyme moieties attached to the membrane through a linker with cleavable disulfide moieties is depicted in a partition of an aqueous solution of linear polymers that have been modified with phenol groups. In **FIG. 11,** the presence of the HRP co-substrate, $H_2O_2$ (not shown), results in the catalytic formation of crosslinks between phenol moieties on nearby linear polymers. The crosslinks between linear polymers result in an initial hydrogel layer forming around the cell. This thickness of this initial hydrogel layer, however, is limited due to the HRP crosslink-catalyzing moieties ability to interact with phenol substrate remaining in the solution of the partition outside of this initial layer. In **FIG. 12,** the presence of disulfide cleaving agent (*e.g.,* DTT) or stimulus (*e.g.,* heat) results in cleavage of the linkers thereby allowing the HRP moieties to diffuse through the pores of the initial hydrogel layer and in the un-crosslinked portions of the linear polymer solution. This second stage of activity by the crosslink-catalyzing enzyme extends the formation of the hydrogel coating further around the cell, resulting in a cell entrapped in a substantially thicker hydrogel coating.

This hydrogel-coating around the cell provides for a more robust cell sample that can be more easily manipulated and used in applications and assays, include selective cell culturing, selective cell storage, and selective cell assays. In at least one embodiment of the present disclosure, the hydrogel-coating provided by the two-stage process of the present disclosure is at least about 5 μm in thickness, at least about 10 μm, at least about 20 μm, at least about 30 μm, at least about 40 μm, at least about 50 μm, at least about 75 μm, at least about 100 μm, at least about 120 μm, at least about 150 μm, at least about 200 μm, or an even greater thickness. Thus, typically, the hydrogel-coating has an average thickness of from about 5 μm to about 200 μm, from about 25 μm to about 175 μm, from about 30 μm to about 150 μm, or from about 50 μm to about 150 μm.

In at least one embodiment, the thickness of the hydrogel-coating formed in the two-stage process can be controlled by the volume of the aqueous partition that contains the cell. The partition is a space or volume that is suitable to contain one or more species or conduct one or more reactions, and may be a physical compartment, such as a droplet. The partition is capable of isolating its space or volume from the space or volume of another partition. In some embodiments, partition can be a discrete droplet of a first phase (*e.g.,* aqueous phase) in a second phase (*e.g.,* an oil phase) that is immiscible with the first phase. In at least one embodiment, the partition used in the methods of the present is a discrete droplet in an immiscible phase. As described elsewhere herein, methods, reagents, and microfluidic systems for generating aqueous partitions (*e.g.,* discrete droplets in immiscible solutions) containing biological particles are well known, and can be used to control the volume of partitions containing cells.

## Generating a partition containing a biological particle

Methods, techniques, and protocols useful for generating cells contained in partitions, such as discrete droplets, are described in the art. The discrete droplet partitions generated act a nanoliter-scale container that can maintain separation of the droplet contents from the contents of other droplets in an immiscible emulsion.

In an aspect, the methods described herein provide for the compartmentalization, depositing, or partitioning of one or more particles (*e.g.,* biological particles such as cells, cell beads, or nuclei, macromolecular constituents of biological particles, beads, reagents, *etc.)* into discrete compartments or partitions (referred to interchangeably herein as partitions), where each partition maintains separation of its own contents from the contents of other partitions.

In some embodiments disclosed herein, the partitioned biological particle is a labelled cell of B cell lineage, *e.g.* a memory B cell, which expresses an antigen-binding molecule (*e.g.,* an immune receptor, an antibody or a functional fragment thereof) on its surface. In other examples, the partitioned biological particle can be a labelled cell engineered to express antigen-binding molecules (*e.g.,* an immune receptors, antibodies or functional fragments thereof).

The term "partition," as used herein, generally, refers to a space or volume that can be suitable to contain one or more biological particle (e.g., cells, cell beads, or nuclei), one or more species of features or compounds, or conduct one or more reactions. A partition can be a physical container, compartment, or vessel, such as a droplet, a flow cell, a reaction chamber, a reaction compartment, a tube, a well, or a microwell. In some embodiments, the compartments or partitions include partitions that are flowable within fluid streams. These partitions can include, for example, micro-vesicles that have an outer barrier surrounding an inner fluid center or core, or, in some cases, the partitions can include a porous matrix that is capable of entraining and/or retaining materials within its matrix. In some aspects, partitions comprise droplets of aqueous fluid within a non-aqueous continuous phase (*e.g.,* oil phase). A variety of different vessels are described in, for example, U.S. Patent Application Publication No. 2014/0155295. Materials, methods and systems for creating stable discrete droplets encapsulating a cell or other biological sample in non-aqueous or oil emulsions are described in, *e.g.,* US Pat. Publ. Nos. 2010/0105112A1 and 2019/0100632A1.

In some embodiments, a partition herein includes a space or volume that can be suitable to contain one or more species or conduct one or more reactions. A partition can be a physical compartment, such as a droplet or well. The partition can be an isolated space or volume from another space or volume. The droplet can be a first phase (*e.g.,* aqueous phase) in a second phase (*e.g.,* oil) immiscible with the first phase. The droplet can be a first phase in a second phase that does not phase separate from the first phase, such as, for example, a capsule or liposome in an aqueous phase. A partition can include one or more other (inner) partitions. In some cases, a partition can be a virtual compartment that can be defined and identified by an index (*e.g.,* indexed libraries) across multiple and/or remote physical compartments. For example, a physical compartment can include a plurality of virtual compartments.

In some embodiments, the methods described herein provide for the compartmentalization, depositing or partitioning of individual biological particles (e.g., cells, cell beads, or nuclei) from a sample material containing cells, into discrete partitions, where each partition maintains separation of its own contents from the contents of other partitions. Identifiers including unique identifiers (*e.g.,* UMI) and common or universal tags, *e.g.,* barcodes, can be previously, subsequently or concurrently delivered to the partitions that hold the compartmentalized or partitioned biological particles (e.g. cells, cell beads, or nuclei), in order to allow for the later attribution of the characteristics of the individual biological particles to one or more particular compartments. Further, identifiers including unique identifiers and common or universal tags, *e.g.,* barcodes, can be coupled to labelling agents and previously, subsequently or concurrently delivered to the partitions that hold the compartmentalized or partitioned cells, in order to allow for the later attribution of the characteristics of the individual biological particles (e.g., cells, cell beads, or nuclei) to one or more particular compartments. Identifiers including unique identifiers and common or universal tags, *e.g.,* barcodes, can be delivered, for example on an oligonucleotide, to a partition via any suitable mechanism, for example by coupling the barcoded oligonucleotides to a bead. In some embodiments, the barcoded oligonucleotides are reversibly (*e.g.,* releasably) coupled to a bead. The bead suitable for the methods of the disclosure can have different surface chemistries and/or physical volumes. In some embodiments, the bead includes a polymer gel. In some embodiments, the polymer gel is a polyacrylamide. Additional non-limiting examples of suitable beads include microparticles, nanoparticles, beads, and microbeads. The partition can be a droplet in an emulsion. Materials, methods and systems for creating stable discrete partitions can include one or more particles. A partition can include one or more types of particles. For example, a partition of the present disclosure can include one or more biological particles, *e.g.,* labelled engineered cells, B cells, or memory B cells, organelles (e.g., nuclei), and/or macromolecular constituents thereof. A partition can include one or more gel beads. A partition can include one or more cell beads. A partition can include a single gel bead, a single cell bead, or both a single cell bead and single gel bead. A partition can include one or more reagents. Alternatively, a partition can be unoccupied. For example, a partition cannot comprise a bead. Unique identifiers, such as barcodes, can be injected into the droplets previous to, subsequent to, or concurrently with droplet generation, such as via a bead, as described elsewhere herein. Microfluidic channel networks (*e.g.,* on a chip) can be utilized to generate partitions as described herein. Alternative mechanisms can also be employed in the partitioning of individual biological particles, including porous membranes

through which aqueous mixtures of cells are extruded into non-aqueous fluids.

The partitions can be flowable within fluid streams. The partitions can include, for example, micro-vesicles that have an outer barrier surrounding an inner fluid center or core. In some cases, the partitions can include a porous matrix that is capable of entraining and/or retaining materials (*e.g.,* expressed antibodies or antigen-binding fragments thereof) within its matrix (*e.g.,* via a capture agent configured to couple to both the matrix and the expressed antibody or antigen-binding fragment thereof). The partitions can be droplets of a first phase within a second phase, wherein the first and second phases are immiscible. For example, the partitions can be droplets of aqueous fluid within a non-aqueous continuous phase (*e.g.,* oil phase). In another example, the partitions can be droplets of a non-aqueous fluid within an aqueous phase. In some examples, the partitions can be provided in a water-in-oil emulsion or oil-in-water emulsion. A variety of different vessels are described in, for example, U.S. Patent Application Publication No. 2014/0155295. Emulsion systems for creating stable droplets in non-aqueous or oil continuous phases are described in, for example, U.S. Patent Application Publication No. 2010/0105112.

Briefly, generating a discrete droplet containing a biological particle (e.g., a cell, cell bead, or nucleus) is accomplished by introducing a flowing stream of an aqueous fluid containing the biological particle (e.g., cell, cell bead, or nuclei) into a flowing stream of a non-aqueous fluid with which it is immiscible, such that droplets are generated at the junction of the two streams (see *e.g.,* **FIGS. 13-15).** Fluid properties (*e.g.,* fluid flow rates, fluid viscosities, *etc.*), particle properties (*e.g.,* volume fraction, particle size, particle concentration, *etc.*), microfluidic architectures (*e.g.,* channel geometry, *etc.*), and other parameters can be adjusted to control the occupancy of the resulting partitions (*e.g.,* number of biological particles per partition, number of beads per partition, *etc.*). By providing the aqueous stream at a certain concentration and/or flow rate of the cell, the occupancy of the resulting droplets can be controlled. For example, the relative flow rates of the immiscible fluids can be selected such that, on average, the discrete droplet each contains less than one biological particle, e.g., cell, cell bead, or nucleus (or other particle of a biological sample). Such a flow rate ensures that the droplets that are occupied are primarily occupied by a single biological particle, such as a cell, cell bead, or nucleus. Discrete droplets in an emulsion encapsulating a biological particle, e.g., a cell, cell bead, or nucleus, is also accomplished using a microfluidic architecture comprising a channel segment having a channel junction with a reservoir (see **FIGS. 16-18).** In some cases, the droplets among a plurality of discrete droplets formed in the manner contain at most one particle (*e.g.,* bead, DNA, cell, such as a labelled engineered cells, B cells, or memory B cells, cell bead, nucleus, or cellular material). In some embodiments, the various parameters (*e.g.,* fluid properties, particle properties, microfluidic architectures, *etc.*) can be selected or adjusted such that a majority of partitions are occupied, for example, allowing for only a small percentage of unoccupied partitions. The flows and microfluidic channel architectures also can be controlled to ensure a given number of singly occupied droplets, less than a certain level of unoccupied droplets, and/or less than a certain level of multiply occupied droplets.

In some embodiments, the method further includes individually partitioning one or more single biological particles (e.g., cells, cell beads, or nuclei) from a plurality of biological particles (e.g., cells, cell beads, or nuclei) in a partition of a second plurality of partitions.

In some embodiments, at least one of the first and second plurality of partitions includes a microwell, a flow cell, a reaction chamber, a reaction compartment, or a droplet. In some embodiments, at least one of the first and second plurality of partitions includes individual droplets in emulsion. In some embodiments, the partitions of the first plurality and/or the second plurality of partition have the same reaction volume.

In the case of droplets in emulsion, allocating individual biological particles (e.g., cells, cell beads, or nuclei) to discrete partitions can generally be accomplished by introducing a flowing stream of biological particles, such as cells, cell beads, or nuclei, in an aqueous fluid into a flowing stream of a non-aqueous fluid, such that droplets are generated at the junction of the two streams. By providing the aqueous biological particle-containing (e.g., cell-, cell bead- or nucleus-containing) stream at a certain concentration of biological particles, the occupancy of the resulting partitions (*e.g.,* number of biological particles, such as cells, cell beads, or nuclei, per partition) can be controlled. For example, where single cell partitions are desired, the relative flow rates of the fluids can be selected such that, on average, the partitions contain less than one cell per partition, in order to ensure that those partitions that are occupied, are primarily singly occupied. In some embodiments, the relative flow rates of the fluids can be selected such that a majority of partitions are occupied, *e.g.,* allowing for only a small percentage of unoccupied partitions. In some embodiments, the flows and channel architectures are controlled as to ensure a desired number of singly occupied partitions, less than a certain level of unoccupied partitions and less than a certain level of multiply occupied partitions.

In some embodiments, the methods described herein can be performed such that a majority of occupied partitions include no more than one cell per occupied partition. In some embodiments, the partitioning process is performed such that fewer than 25%, fewer than 20%, fewer than 15%, fewer than 10%, fewer than 5%, fewer than 2%, or fewer than 1% the occupied partitions contain more than one cell. In some embodiments, fewer than 20% of the occupied partitions include more than one cell. In some embodiments, fewer than 10% of the occupied partitions include more than one cell per partition. In some embodiments, fewer than 5% of the occupied partitions include more than one cell per partition. In some embodiments, it is desirable to avoid the creation of excessive numbers of empty partitions. For

example, from a cost perspective and/or efficiency perspective, it may be desirable to minimize the number of empty partitions. While this can be accomplished by providing sufficient numbers of cells into the partitioning zone, the Poissonian distribution can optionally be used to increase the number of partitions that include multiple biological particles (e.g. cells, cell beads, or nuclei). As such, in some embodiments described herein, the flow of one or more of the biological particles (e.g., cells, cell beads, or nuclei), or other fluids directed into the partitioning zone are performed such that no more than 50% of the generated partitions, no more than 25% of the generated partitions, or no more than 10% of the generated partitions are unoccupied. Further, in some aspects, these flows are controlled so as to present non-Poissonian distribution of single occupied partitions while providing lower levels of unoccupied partitions. Restated, in some aspects, the above noted ranges of unoccupied partitions can be achieved while still providing any of the single occupancy rates described above. For example, in some embodiments, the use of the methods described herein creates resulting partitions that have multiple occupancy rates of less than 25%, less than 20%, less than 15%), less than 10%, and in some embodiments, less than 5%, while having unoccupied partitions of less than 50%), less than 40%, less than 30%, less than 20%, less than 10%, and in some embodiments, less than 5%.

Although described in terms of providing substantially singly occupied partitions, above, in some embodiments, the methods as described herein include providing multiply occupied partitions, *e.g.,* containing two, three, four or more biological particles (e.g., cells, cell beads, or nuclei) and/or beads comprising nucleic acid barcode molecules within a single partition.

In some embodiments, the reporter oligonucleotides contained within a partition are distinguishable from the reporter oligonucleotides contained within other partitions of the plurality of partitions.

In some embodiments, it may be desirable to incorporate multiple different barcode sequences within a given partition, either attached to a single or multiple beads within the partition. For example, in some cases, a mixed, but known barcode sequences set can provide greater assurance of identification in the subsequent processing, *e.g.,* by providing a stronger address or attribution of the barcodes to a given partition, as a duplicate or independent confirmation of the output from a given partition.

*Microfluidic channel structures*

Microfluidic channel networks (*e.g.,* on a chip) can be utilized to generate partitions as described herein. Alternative mechanisms can also be employed in the partitioning of individual biological particles, including porous membranes through which aqueous mixtures of biological particles (e.g., cells, cell beads, or nuclei) are extruded into non-aqueous fluids.

**FIG. 13** shows an exemplary microfluidic channel structure **100** useful for generating partitions (*e.g.,* discrete droplets) encapsulating an enzyme-decorated cell and linear polymers modified with crosslink precursor moieties of the present disclosure. The channel structure **100** can include channel segments **102, 104, 106** and **108** communicating at a channel junction **110.** In operation, a first aqueous fluid **112** that that includes suspended biological particles, such as cells, cell beads, nuclei or particle of a biological sample (*e.g.,* labelled engineered cells, B cells, or memory B cells) **114** are transported along channel segment **102** into junction **110,** while a second fluid **116** (or "partitioning fluid") that is immiscible with the aqueous fluid **112** is delivered to the junction **110** from each of channel segments **104** and **106** to create discrete droplets **118, 120** of the first aqueous fluid **112** flowing into channel segment **108,** and flowing away from junction **110.** The channel segment **108** may be fluidically coupled to an outlet reservoir where the discrete droplets can be stored and/or harvested. A discrete droplet generated may include an individual enzyme-decorated cell **114** (such as droplet **118),** or a discrete droplet can be generated that contains more than one biological particle (e.g., a cell, a cell bead, or a nucleus) **114** (not shown in **FIG. 13).** A discrete droplet may contain no biological particle (e.g., cell, cell bead, or nucleus) **114** (such as droplet **120).** Each partition is capable of maintaining separation of its own contents (*e.g.,* an individual enzyme-decorated cell **114)** from the contents of other droplets. Typically, the second fluid **116** comprises an oil, such as a fluorinated oil, that includes a fluoro-surfactant that helps to stabilize the resulting droplets, for example, inhibiting subsequent coalescence of the resulting droplets **118, 120.** Examples of useful partitioning fluids and fluorosurfactants are described in *e.g.,* US Pat. Publ. No. 2010/0105112A1.

The microfluidic channels for generating partitions as exemplified in **FIG. 13** may be coupled to any of a variety of different fluid sources or receiving components, including reservoirs, tubing, manifolds, or fluidic components of other systems. Additionally, the microfluidic channel structure **100** may have other geometries, including geometries having more than one channel junction. For example, the microfluidic channel structure can have 2, 3, 4, or 5 channel segments each carrying an enzyme-decorated cell, linear polymers, and optionally, other assay reagents and/or beads, that meet at a channel junction. Generally, the fluids used in generating the discrete droplets are directed to flow along one or more channels or reservoirs via one or more fluid flow units. A fluid flow unit can comprise compressors (*e.g.,* providing positive pressure), pumps (*e.g.,* providing negative pressure), actuators, and the like to control flow of the fluid. Fluid may also or otherwise be controlled via applied pressure differentials, centrifugal force, electro-kinetic pumping, vacuum, capillary or gravity flow, or the like.

The generated droplets can include two subsets of droplets: (1) occupied droplets **118,** containing one or more biological particles **114,** *e.g.,* labelled engineered cells, B cells, memory B cells, cell bead, or nuclei, and (2) unoccupied droplets **120,** not containing any biological particles **114.** Occupied droplets **118** can include singly occupied droplets (having one biological particle, such as one B cell or memory B cell, a cell bead or a nucleus) and multiply occupied droplets (having more than one biological particle, such as multiple B cells or memory B cells, cell beads, or nuclei). As described elsewhere herein, in some cases, the majority of occupied partitions can include no more than one biological particle, *e.g.,* labelled engineered cells, B cells, memory B cells, cell beads, or nuclei per occupied partition and some of the generated partitions can be unoccupied (of any biological particle, or labelled engineered cells, B cells, memory B cells, cell beads, or nuclei). In some cases, though, some of the occupied partitions can include more than one biological particle, *e.g.,* labelled engineered cells, B cells, memory B cells, cell beads, or nuclei. In some cases, the partitioning process can be controlled such that fewer than about 25% of the occupied partitions contain more than one biological particle, and in many cases, fewer than about 20% of the occupied partitions have more than one biological particle, while in some cases, fewer than about 10% or even fewer than about 5% of the occupied partitions include more than one biological particle per partition.

In some cases, it can be desirable to minimize the creation of excessive numbers of empty partitions, such as to reduce costs and/or increase efficiency. While this minimization can be achieved by providing a sufficient number of biological particles (*e.g.,* biological particles, such as labelled engineered cells, B cells, memory B cells, cell beads, or nuclei **114)** at the partitioning junction **110,** such as to ensure that at least one biological particle is encapsulated in a partition, the Poissonian distribution can expectedly increase the number of partitions that include multiple biological particles. As such, where singly occupied partitions are to be obtained, at most about 95%, 90%, 85%, 80%, 75%, 70%, 65%, 60%, 55%, 50%, 45%, 40%, 35%, 30%, 25%, 20%, 15%, 10%, 5% or less of the generated partitions can be unoccupied.

In some cases, the flow of one or more of the biological particles, such as B cells or memory B cells, (*e.g.,* in channel segment 102), or other fluids directed into the partitioning junction (*e.g.,* in channel segments 104, 106) can be controlled such that, in many cases, no more than about 50% of the generated partitions, no more than about 25% of the generated partitions, or no more than about 10% of the generated partitions are unoccupied. These flows can be controlled so as to present a non-Poissonian distribution of single-occupied partitions while providing lower levels of unoccupied partitions. The above noted ranges of unoccupied partitions can be achieved while still providing any of the single occupancy rates described above. For example, in many cases, the use of the methods described herein can create resulting partitions that have multiple occupancy rates of less than about 25%, less than about 20%, less than about 15%, less than about 10%, and in many cases, less than about 5%, while having unoccupied partitions of less than about 50%, less than about 40%, less than about 30%, less than about 20%, less than about 10%, less than about 5%, or less.

As will be appreciated, the above-described occupancy rates are also applicable to partitions that include both biological particles (*e.g.,* cells, cell beads, or nuclei) and additional reagents, including, but not limited to, beads (*e.g.,* gel beads) carrying nucleic acid barcode molecules (*e.g.,* barcoded oligonucleotides) (described in relation to FIG. 13 and 16). The occupied partitions (*e.g.,* at least about 10%, 20%, 30%, 40%, 50%, 60%, 70%, 80%, 90%, 95%, or 99% of the occupied partitions) can include both a bead comprising nucleic acid barcode molecules and a biological particle (e.g., a cell or a nucleus).

In another aspect, in addition to or as an alternative to droplet-based partitioning, labelled biological particles (*e.g.,* cells or nuclei) may be encapsulated within a particulate material to form a "cell bead".

The cell bead can include other reagents. Encapsulation of biological particles, *e.g.,* labelled biological particles, such as engineered cells or nuclei, can be performed by a variety of processes. Such processes can combine an aqueous fluid containing the labelled biological particles with a polymeric precursor material that can be capable of being formed into a gel or other solid or semi-solid matrix upon application of a particular stimulus to the polymer precursor. Such stimuli can include, for example, thermal stimuli (*e.g.,* either heating or cooling), photo-stimuli (*e.g.,* through photo-curing), chemical stimuli (*e.g.,* through crosslinking, polymerization initiation of the precursor (*e.g.,* through added initiators)), mechanical stimuli, or a combination thereof.

Encapsulation of biological particles, *e.g.,* labelled cells, including engineered cells, B cells, memory B cells or nuclei, can be performed by a variety of methods. For example, air knife droplet or aerosol generators may be used to dispense droplets of precursor fluids into gelling solutions in order to form cell beads that include individual biological particles or small groups of biological particles. Likewise, membrane-based encapsulation systems may be used to generate cell beads comprising encapsulated biological particles as described herein. Microfluidic systems of the present disclosure, such as that shown in FIG. 13, may be readily used in encapsulating biological particles (*e.g.,* cells or nuclei, including labelled cells or nuclei) as described herein. Exemplary methods for encapsulating biological particles (*e.g.,* cells or nuclei) are also further described in U.S. Patent Application Pub. No. US 2015/0376609 and PCT/US2018/016019. In particular, and with reference to FIG. 13, the aqueous fluid 112 comprising (i) the biological particles 114 and (ii) the polymer precursor material (not shown) is flowed into channel junction 110, where it is partitioned into droplets 118, 120

through the flow of non-aqueous fluid 116. In the case of encapsulation methods, non-aqueous fluid 116 may also include an initiator (not shown) to cause polymerization and/or crosslinking of the polymer precursor to form the bead that includes the entrained biological particles. Examples of polymer precursor/initiator pairs include those described in U.S. Patent Application Publication No. 2014/0378345.

For example, in the case where the polymer precursor material comprises a linear polymer material, such as a linear polyacrylamide, PEG, or other linear polymeric material, the activation agent can include a cross-linking agent, or a chemical that activates a cross-linking agent within the formed droplets. Likewise, for polymer precursors that comprise polymerizable monomers, the activation agent can include a polymerization initiator. For example, in certain cases, where the polymer precursor comprises a mixture of acrylamide monomer with a N,N'-bis-(acryloyl)cystamine (BAC) comonomer, an agent such as tetraethylmethylenediamine (TEMED) can be provided within the second fluid streams 1216 in channel segments 1204 and 1206, which can initiate the copolymerization of the acrylamide and BAC into a cross-linked polymer network, or hydrogel.

Upon contact of the second fluid stream 116 with the first fluid stream 112 at junction 110, during formation of droplets, the TEMED can diffuse from the second fluid 116 into the aqueous fluid 112 comprising the linear polyacrylamide, which will activate the crosslinking of the polyacrylamide within the droplets 118, 120, resulting in the formation of gel (*e.g.,* hydrogel) cell beads, as solid or semi-solid beads or particles entraining the biological particles, including labelled biological particles (*e.g.,* cells such as B cells or nuclei) 114. Although described in terms of polyacrylamide encapsulation, other "activatable" encapsulation compositions can also be employed in the context of the methods described herein. For example, formation of alginate droplets followed by exposure to divalent metal ions (*e.g.,* Ca2+ ions), can be used as an encapsulation process using the described processes. Likewise, agarose droplets can also be transformed into capsules through temperature based gelling (*e.g.,* upon cooling, etc.).

In some cases, encapsulated biological particles (e.g., labelled cells or nuclei) can be selectively releasable from the cell bead, such as through passage of time or upon application of a particular stimulus, that degrades the encapsulating material sufficiently to allow the biological particles (*e.g.,* labelled cells including B cells or nuclei), or its other contents to be released from the encapsulating material, such as into a partition (*e.g.,* droplet). For example, in the case of the polyacrylamide polymer described above, degradation of the polymer can be accomplished through the introduction of an appropriate reducing agent, such as DTT or the like, to cleave disulfide bonds that cross-link the polymer matrix. See, for example, U.S. Patent Application Publication No. 2014/0378345.

In at least one embodiment, the partition containing the enzyme-decorated cell and linear polymers, and other reagent(s), can also include a barcode. The inclusion of a barcode in a partition along with a cell provides a unique identifier that allows data obtained from the result hydrogel-coated cell in the partition to be distinguished from data obtained from other partitions, and individually analyzed. The term "barcode," as used herein, generally refers to an identifier, that conveys, or is capable of conveying information about a cell, biological particle, or other analyte associated with the barcode in the partition. A barcode can be part of an analyte or can be independent of an analyte. A barcode can be a tag attached to an analyte (*e.g.,* a nucleic acid molecule) or a combination of the tag in addition to an endogenous characteristic of the analyte (*e.g.,* size of the analyte or end sequence(s)). A barcode may be unique. Barcodes can have a variety of different formats. For example, barcodes can include polynucleotide barcodes; random nucleic acid and/or amino acid sequences; and synthetic nucleic acid and/or amino acid sequences. A barcode can be attached to an analyte in a reversible or irreversible manner. A barcode can be added to, for example, a fragment of a deoxyribonucleic acid (DNA) or ribonucleic acid (RNA) sample before, during, and/or after sequencing of the sample. Barcodes can allow for identification and/or quantification of individual sequencing-reads. In some embodiments, a barcode can be configured for use as a fluorescent barcode. For example, in some embodiments, a barcode can be configured for hybridization to fluorescently labeled oligonucleotide probes. Barcodes can be configured to spatially resolve molecular components found in biological samples, for example, at single-cell resolution (*e.g.,* a barcode can be or can include a "spatial barcode"). In some embodiments, a barcode includes two or more sub-barcodes that together function as a single barcode. For example, a polynucleotide barcode can include two or more polynucleotide sequences (*e.g.,* sub-barcodes). In some embodiments, the two or more sub-barcodes are separated by one or more non-barcode sequences. In some embodiments, the two or more sub-barcodes are not separated by non-barcode sequences.

In some embodiments, a barcode can include one or more unique molecular identifiers (UMIs). Generally, a unique molecular identifier is a contiguous nucleic acid segment or two or more non-contiguous nucleic acid segments that function as a label or identifier for a particular analyte, or for a nucleic acid barcode molecule that binds a particular analyte (*e.g.,* mRNA) via the capture sequence. A UMI can include one or more specific polynucleotides sequences, one or more random nucleic acid and/or amino acid sequences, and/or one or more synthetic nucleic acid and/or amino acid sequences. In some embodiments, the UMI is a nucleic acid sequence that does not substantially hybridize to analyte nucleic acid molecules in a biological sample. In some embodiments, the UMI has less than 80% sequence identity (*e.g.,* less than 70%, 60%, 50%, or less than 40% sequence identity) to the nucleic acid sequences across a substantial part (*e.g.,* 80% or more) of the nucleic acid molecules in the biological sample. These nucleotides can be completely contiguous, *i.e.,* in a single stretch of adjacent nucleotides, or they can be separated into two or more separate

subsequences that are separated by 1 or more nucleotides.

A "barcoded nucleic acid molecule" refers to a nucleic acid molecule that results from, for example, the processing of a barcode molecule with a nucleic acid sequence (*e.g.,* nucleic acid sequence complementary to a nucleic acid primer sequence encompassed by the nucleic acid barcode molecule). The nucleic acid sequence may be a targeted sequence (*e.g.,* targeted by a primer sequence) or a non-targeted sequence. For example, in the methods, compositions, kits, and systems described herein, hybridization and reverse transcription of the nucleic acid molecule (*e.g.,* an mRNA molecule) of a cell contained in a partition with a nucleic acid barcode molecule (*e.g.,* a nucleic acid barcode molecule containing a barcode sequence and a nucleic acid primer sequence complementary to a nucleic acid sequence of the mRNA molecule) results in a barcoded nucleic acid molecule that has a sequence corresponding to the nucleic acid sequence of the mRNA and the barcode sequence (or a reverse complement thereof). A barcoded nucleic acid molecule may serve as a template, such as a template polynucleotide, that can be further processed (*e.g.,* amplified) and sequenced to obtain the target nucleic acid sequence. For example, in the methods described herein, a barcoded nucleic acid molecule may be further processed (*e.g.,* amplified) and sequenced to obtain the nucleic acid sequence of the mRNA.

It is contemplated that in the methods of the present disclosure that barcodes can be delivered into the partition previous to, subsequent to, or concurrent with the enzyme-decorated cell, linear polymers, co-substrate, and/or other assay reagents. For example, barcodes may be injected into an aqueous mixture used to form a discrete droplet previous to droplet formation in an immiscible oil phase.

Barcodes useful in the methods of the present disclosure typically comprise a nucleic acid molecule (*e.g.,* an oligonucleotide). The nucleic acid molecules typically are delivered to a partition via a solid or semi-solid phase, such as a bead, to which the barcode molecules are linked. In some cases, the barcode nucleic acid molecules are initially associated with a bead upon generation of the partition and then released from the bead upon application of a stimulus to droplet. Beads comprising barcodes useful in the methods of the present disclosure are described in further detail elsewhere herein. The beads are generally particles and can include solid or semi-solid particles, such as a gel bead. In some embodiments, a gel bead can include a polymer matrix formed by polymerization or cross-linking of linear polymers. The polymer matrix may be made up of one or more different polymers (*e.g.,* polymers having different functional groups or repeat units), and the polymers in the matrix may be randomly arranged, such as in random copolymers, and/or have ordered structures, such as in block copolymers. Cross-linking between polymers in the polymer matrix can be via covalent, ionic, or inductive, interactions, or physical entanglement. The bead may be a macromolecule. The bead may be formed of nucleic acid molecules bound together. The bead may be formed via covalent or non-covalent assembly of molecules (*e.g.,* macromolecules), such as monomers or polymers, which may be natural or synthetic. Such polymers or monomers may be or include nucleic acid molecules (*e.g.,* DNA or RNA). The bead may be magnetic or non-magnetic. The bead may be rigid. The bead may be flexible and/or compressible. The bead may be disruptable or dissolvable. The bead may be a solid particle (*e.g.,* a metal-based particle including but not limited to iron oxide, gold or silver) covered with a coating comprising one or more polymers. Such a coating may be disruptable, degradable, or dissolvable. Beads useful with the methods can be of materials that are porous, non-porous, solid, semi-solid, semi-fluidic, fluidic, and/or a combination thereof. In some embodiments, the bead is a gel bead comprising a hydrogel matrix. Such gel beads can be formed from polymeric or monomeric precursor molecules that undergo a crosslinking reaction to form a hydrogel matrix. Another semi-solid bead useful in the present disclosure is a liposomal bead. In some embodiments, beads used can be solid beads that comprise a metal including iron oxide, gold, and silver. In some cases, the bead may be a silica bead. In some cases, the bead can be rigid. In other cases, the bead may be flexible and/or compressible. Generally, the beads can be of any suitable shape. Examples of bead shapes include, but are not limited to, spherical, non-spherical, oval, oblong, amorphous, circular, cylindrical, and variations thereof.

The biological particle (*e.g.,* labelled cells, such as B cells, or nuclei), can be subjected to other conditions sufficient to polymerize or gel the precursors. The conditions sufficient to polymerize or gel the precursors can include exposure to heating, cooling, electromagnetic radiation, and/or light. The conditions sufficient to polymerize or gel the precursors can include any conditions sufficient to polymerize or gel the precursors. Following polymerization or gelling, a polymer or gel can be formed around the biological particle (*e.g.,* labelled cells, such as B cells, or nuclei). The polymer or gel can be diffusively permeable to chemical or biochemical reagents. The polymer or gel can be diffusively impermeable to macromolecular constituents (*e.g.,* secreted antibodies or antigen-binding fragments thereof) of the biological particle (*e.g.,* labelled cells, such as B cells, or nuclei). In this manner, the polymer or gel can act to allow the biological particle (*e.g.,* labelled cells, such as B cells, or nuclei) to be subjected to chemical or biochemical operations while spatially confining the macromolecular constituents to a region of the droplet defined by the polymer or gel. The polymer or gel can include one or more of disulfide cross-linked polyacrylamide, agarose, alginate, polyvinyl alcohol, polyethylene glycol (PEG)-diacrylate, PEG-acrylate, PEG-thiol, PEG-azide, PEG-alkyne, other acrylates, chitosan, hyaluronic acid, collagen, fibrin, gelatin, or elastin. The polymer or gel can include any other polymer or gel.

The polymer or gel can be functionalized (*e.g.,* coupled to a capture agent) to bind to targeted analytes (*e.g.,* secreted antibodies or antigen-binding fragment thereof), such as nucleic acids, proteins, carbohydrates, lipids or other analytes. The polymer or gel can be polymerized or gelled via a passive mechanism. The polymer or gel can be stable

in alkaline conditions or at elevated temperature. The polymer or gel can have mechanical properties similar to the mechanical properties of the bead. For instance, the polymer or gel can be of a similar size to the bead. The polymer or gel can have a mechanical strength (*e.g.,* tensile strength) similar to that of the bead. The polymer or gel can be of a lower density than an oil. The polymer or gel can be of a density that is roughly similar to that of a buffer. The polymer or gel can have a tunable pore size. The pore size can be chosen to, for instance, retain denatured nucleic acids. The pore size can be chosen to maintain diffusive permeability to exogenous chemicals such as sodium hydroxide (NaOH) and/or endogenous chemicals such as inhibitors. The polymer or gel can be biocompatible. The polymer or gel can maintain or enhance cell viability. The polymer or gel can be biochemically compatible. The polymer or gel can be polymerized and/or depolymerized thermally, chemically, enzymatically, and/or optically.

The polymer can include poly(acrylamide-co-acrylic acid) crosslinked with disulfide linkages. The preparation of the polymer can include a two-step reaction. In the first activation step, poly(acrylamide-co-acrylic acid) can be exposed to an acylating agent to convert carboxylic acids to esters. For instance, the poly(acrylamide-co-acrylic acid) can be exposed to 4-(4,6-dimethoxy-1,3,5-triazin-2-yl)-4-methylmorpholinium chloride (DMTMM). The polyacrylamide-co-acrylic acid can be exposed to other salts of 4-(4,6-dimethoxy-1,3,5-triazin-2-yl)-4-methylmorpholinium. In the second crosslinking step, the ester formed in the first step can be exposed to a disulfide crosslinking agent. For instance, the ester can be exposed to cystamine (2,2'-dithiobis(ethylamine)). Following the two steps, the biological particle (e.g., the labelled biological particle, including a labelled cell or nucleus) can be surrounded by polyacrylamide strands linked together by disulfide bridges. In this manner, the biological particle can be encased inside of or comprise a gel or matrix (*e.g.,* polymer matrix) to form a "cell bead." A cell bead can contain biological particles (*e.g.,* labelled cells such as B cells or nuclei) or macromolecular constituents (*e.g.,* RNA, DNA, proteins, secreted antibodies or antigen-binding fragments thereof *etc.*) of biological particles. A cell bead can include a single cell/nucleus or multiple cells/nuclei, or a derivative of the single cell/nucleus or multiple cells/nuclei. For example after lysing and washing the cells, inhibitory components from cell or nucleus lysates can be washed away and the macromolecular constituents can be bound as cell beads. Methods disclosed herein can be applicable to both (i) cell beads (and/or droplets or other partitions) containing biological particles and (ii) cell beads (and/or droplets or other partitions) containing macromolecular constituents of biological particles.

Encapsulated biological particles (*e.g.,* labelled cells such as B cells or nuclei) can provide certain potential advantages of being more storable and more portable than droplet-based partitioned biological particles. Furthermore, in some cases, it can be desirable to allow biological particles (*e.g.,* labelled cells such as B cells or nuclei) to incubate for a select period of time before analysis, such as in order to characterize changes in such biological particles over time, either in the presence or absence of different stimuli (*e.g.,* cytokines, antigens, *etc.*). In such cases, encapsulation can allow for longer incubation than partitioning in emulsion droplets, although in some cases, droplet partitioned biological particles can also be incubated for different periods of time, *e.g.,* at least 10 seconds, at least 30 seconds, at least 1 minute, at least 5 minutes, at least 10 minutes, at least 30 minutes, at least 1 hour, at least 2 hours, at least 5 hours, or at least 10 hours or more. The encapsulation of biological particles (*e.g.,* labelled cells such as B cells or nuclei) can constitute the partitioning of the biological particles into which other reagents are co-partitioned. Alternatively or in addition, encapsulated biological particles can be readily deposited into other partitions (*e.g.,* droplets) as described above.

### *Microwells*

As described herein, one or more processes can be performed in a partition, which can be a well. The well can be a well of a plurality of wells of a substrate, such as a microwell of a microwell array or plate, or the well can be a microwell or microchamber of a device (*e.g.,* microfluidic device) comprising a substrate. The well can be a well of a well array or plate, or the well can be a well or chamber of a device (*e.g.,* fluidic device). Accordingly, the wells or microwells can assume an "open" configuration, in which the wells or microwells are exposed to the environment (*e.g.,* contain an open surface) and are accessible on one planar face of the substrate, or the wells or microwells can assume a "closed" or "sealed" configuration, in which the microwells are not accessible on a planar face of the substrate. In some instances, the wells or microwells can be configured to toggle between "open" and "closed" configurations. For instance, an "open" microwell or set of microwells can be "closed" or "sealed" using a membrane (*e.g.,* semi-permeable membrane), an oil (*e.g.,* fluorinated oil to cover an aqueous solution), or a lid, as described elsewhere herein. The wells or microwells can be initially provided in a "closed" or "sealed" configuration, wherein they are not accessible on a planar surface of the substrate without an external force. For instance, the "closed" or "sealed" configuration can include a substrate such as a sealing film or foil that is puncturable or pierceable by pipette tip(s). Suitable materials for the substrate include, without limitation, polyester, polypropylene, polyethylene, vinyl, and aluminum foil.

In some embodiments, the well can have a volume of less than 1 milliliter (mL). For example, the well can be configured to hold a volume of at most 1000 microliters (μL), at most 100 μL, at most 10 μL, at most 1 μL, at most 100 nanoliters (nL), at most 10 nL, at most 1 nL, at most 100 picoliters (pL), at most 10 (pL), or less. The well can be configured to hold a volume of about 1000 μL, about 100 μL, about 10 μL, about 1 μL, about 100 nL, about 10 nL, about 1 nL,

about 100 pL, about 10 pL, *etc.* The well can be configured to hold a volume of at least 10 pL, at least 100 pL, at least 1 nL, at least 10 nL, at least 100 nL, at least 1 μL, at least 10 μL, at least 100 μL, at least 1000 μL, or more. The well can be configured to hold a volume in a range of volumes listed herein, for example, from about 5 nL to about 20 nL, from about 1 nL to about 100 nL, from about 500 pL to about 100 μL, *etc.* The well can be of a plurality of wells that have varying volumes and can be configured to hold a volume appropriate to accommodate any of the partition volumes described herein.

In some instances, a microwell array or plate includes a single variety of microwells. In some instances, a microwell array or plate includes a variety of microwells. For instance, the microwell array or plate can include one or more types of microwells within a single microwell array or plate. The types of microwells can have different dimensions (*e.g.,* length, width, diameter, depth, cross-sectional area, *etc.),* shapes *(e.g.,* circular, triangular, square, rectangular, pentagonal, hexagonal, heptagonal, octagonal, nonagonal, decagonal, *etc.),* aspect ratios, or other physical characteristics. The microwell array or plate can include any number of different types of microwells. For example, the microwell array or plate can include 1, 2, 3, 4, 5, 6, 7, 8, 9, 10, 20, 30, 40, 50, 60, 70, 80, 90, 100, 200, 300, 400, 500, 600, 700, 800, 900, 1000 or more different types of microwells. A well can have any dimension *(e.g.,* length, width, diameter, depth, cross-sectional area, volume, *etc.),* shape (*e.g.,* circular, triangular, square, rectangular, pentagonal, hexagonal, heptagonal, octagonal, nonagonal, decagonal, other polygonal, *etc.),* aspect ratios, or other physical characteristics described herein with respect to any well.

In certain instances, the microwell array or plate includes different types of microwells that are located adjacent to one another within the array or plate. For example, a microwell with one set of dimensions can be located adjacent to and in contact with another microwell with a different set of dimensions. Similarly, microwells of different geometries can be placed adjacent to or in contact with one another. The adjacent microwells can be configured to hold different articles; for example, one microwell can be used to contain a biological particle (e.g., cell, nuclei, cell bead), or other sample (*e.g.,* cellular components, nucleic acid molecules, *etc.)* while the adjacent microwell can be used to contain a droplet, bead, or other reagent. In some cases, the adjacent microwells can be configured to merge the contents held within, *e.g.,* upon application of a stimulus, or spontaneously, upon contact of the articles in each microwell.

As is described elsewhere herein, a plurality of partitions can be used in the methods described herein. For example, any suitable number of partitions (*e.g.,* wells or droplets) can be generated or otherwise provided. For example, in the case when wells are used, at least about 1,000 wells, at least about 5,000 wells, at least about 10,000 wells, at least about 50,000 wells, at least about 100,000 wells, at least about 500,000 wells, at least about 1,000,000 wells, at least about 5,000,000 wells at least about 10,000,000 wells, at least about 50,000,000 wells, at least about 100,000,000 wells, at least about 500,000,000 wells, at least about 1,000,000,000 wells, or more wells can be generated or otherwise provided. Moreover, the plurality of wells can include both unoccupied wells (*e.g.,* empty wells) and occupied wells.

A well can include any of the reagents described herein, or combinations thereof. These reagents can include, for example, barcode molecules, enzymes, adapters, and combinations thereof. The reagents can be physically separated from a sample (for example, a cell, nucleus, cell bead, or cellular components, *e.g.,* proteins, nucleic acid molecules, *etc.)* that is placed in the well. This physical separation can be accomplished by containing the reagents within, or coupling to, a bead that is placed within a well. The physical separation can also be accomplished by dispensing the reagents in the well and overlaying the reagents with a layer that is, for example, dissolvable, meltable, or permeable prior to introducing the polynucleotide sample into the well. This layer can be, for example, an oil, wax, membrane (*e.g.,* semi-permeable membrane), or the like. The well can be sealed at any point, for example, after addition of the bead, after addition of the reagents, or after addition of either of these components. The sealing of the well can be useful for a variety of purposes, including preventing escape of beads or loaded reagents from the well, permitting select delivery of certain reagents (*e.g.,* via the use of a semi-permeable membrane), for storage of the well prior to or following further processing, *etc.*

A well can include free reagents and/or reagents encapsulated in, or otherwise coupled to or associated with, beads or droplets. In some embodiments, any of the reagents described in this disclosure can be encapsulated in, or otherwise coupled to, a droplet or bead, with any chemicals, particles, and elements suitable for sample processing reactions involving biomolecules, such as, but not limited to, nucleic acid molecules and proteins. For example, a bead or droplet used in a sample preparation reaction for DNA sequencing can include one or more of the following reagents: enzymes, restriction enzymes (*e.g.,* multiple cutters), ligase, polymerase, fluorophores, oligonucleotide barcodes, adapters, buffers, nucleotides (*e.g.,* dNTPs, ddNTPs) and the like.

Additional examples of reagents include, but are not limited to: buffers, acidic solution, basic solution, temperature-sensitive enzymes, pH-sensitive enzymes, light-sensitive enzymes, metals, metal ions, magnesium chloride, sodium chloride, manganese, aqueous buffer, mild buffer, ionic buffer, inhibitor, enzyme, protein, polynucleotide, antibodies, saccharides, lipid, oil, salt, ion, detergents, ionic detergents, non-ionic detergents, oligonucleotides, nucleotides, deoxyribonucleotide triphosphates (dNTPs), dideoxyribonucleotide triphosphates (ddNTPs), DNA, RNA, peptide polynucleotides, complementary DNA (cDNA), double stranded DNA (dsDNA), single stranded DNA (ssDNA), plasmid DNA, cosmid DNA, chromosomal DNA, genomic DNA, viral DNA, bacterial DNA, mtDNA (mitochondrial DNA), mRNA, rRNA,

tRNA, nRNA, siRNA, snRNA, snoRNA, scaRNA, microRNA, dsRNA, ribozyme, riboswitch and viral RNA, polymerase, ligase, restriction enzymes, proteases, nucleases, protease inhibitors, nuclease inhibitors, chelating agents, reducing agents, oxidizing agents, fluorophores, probes, chromophores, dyes, organics, emulsifiers, surfactants, stabilizers, polymers, water, small molecules, pharmaceuticals, radioactive molecules, preservatives, antibiotics, aptamers, and pharmaceutical drug compounds. As described herein, one or more reagents in the well can be used to perform one or more reactions, including but not limited to: cell lysis, cell fixation, permeabilization, nucleic acid reactions, *e.g.,* nucleic acid extension reactions, amplification, reverse transcription, transposase reactions *(e.g.,* tagmentation), *etc.*

The wells disclosed herein can be provided as a part of a kit. For example, a kit can include instructions for use, a microwell array or device, and reagents (*e.g.,* beads). The kit can include any useful reagents for performing the processes described herein, *e.g.,* nucleic acid reactions, barcoding of nucleic acid molecules, sample processing (*e.g.,* for cell lysis, fixation, and/or permeabilization).

In some cases, a well includes a bead or droplet that includes a set of reagents that has a similar attribute, for example, a set of enzymes, a set of minerals, a set of oligonucleotides, a mixture of different barcode molecules, a mixture of identical barcode molecules. In other cases, a bead or droplet includes a heterogeneous mixture of reagents. In some cases, the heterogeneous mixture of reagents can include all components necessary to perform a reaction. In some cases, such mixture can include all components necessary to perform a reaction, except for 1, 2, 3, 4, 5, or more components necessary to perform a reaction. In some cases, such additional components are contained within, or otherwise coupled to, a different droplet or bead, or within a solution within a partition (*e.g.,* microwell) of the system.

A non-limiting example of a microwell array in accordance with some embodiments of the disclosure is schematically presented in **FIG. 22.** In this example, the array can be contained within a substrate **1700.** The substrate **1700** includes a plurality of wells **1702.** The wells **1702** can be of any size or shape, and the spacing between the wells, the number of wells per substrate, as well as the density of the wells on the substrate **1700** can be modified, depending on the particular application. In one such example application, a sample molecule **1706,** which can include a biological particle, including a cell/nucleus or cellular/nuclear components (*e.g.,* nucleic acid molecules) is co-partitioned with a bead **1704,** which can include a nucleic acid barcode molecule coupled thereto. The wells **1702** can be loaded using gravity or other loading technique (*e.g.,* centrifugation, liquid handler, acoustic loading, optoelectronic, *etc.).* In some instances, at least one of the wells **1702** contains a single sample molecule **1706** (*e.g.,* cell, cell bead, or nucleus) and a single bead **1704.**

Reagents can be loaded into a well either sequentially or concurrently. In some cases, reagents are introduced to the device either before or after a particular operation. In some cases, reagents (which can be provided, in certain instances, in droplets or beads) are introduced sequentially such that different reactions or operations occur at different steps. The reagents (or droplets, or beads) can also be loaded at operations interspersed with a reaction or operation step. For example, or droplets or beads including reagents for fragmenting polynucleotides (*e.g.,* restriction enzymes) and/or other enzymes (*e.g.,* transposases, ligases, polymerases, *etc.)* can be loaded into the well or plurality of wells, followed by loading of droplets or beads including reagents for attaching nucleic acid barcode molecules to a sample nucleic acid molecule. Reagents can be provided concurrently or sequentially with a sample, *e.g.,* a cell/nucleus or cellular/nuclear components (*e.g.,* organelles, proteins, nucleic acid molecules, carbohydrates, lipids, *etc.).* Accordingly, use of wells can be useful in performing multi-step operations or reactions.

As described elsewhere herein, the nucleic acid barcode molecules and other reagents can be contained within a bead or droplet. These beads or droplets can be loaded into a partition (*e.g.,* a microwell) before, after, or concurrently with the loading of a biological particle (e.g., a cell, cell bead, or nucleus), such that each biological particle is contacted with a different bead or droplet. This technique can be used to attach a unique nucleic acid barcode molecule to nucleic acid molecules obtained from each biological particle (e.g., cell, cell bead, or nucleus). Alternatively or in addition, the sample nucleic acid molecules can be attached to a support. For example, the partition (*e.g.,* microwell) can include a bead which has coupled thereto a plurality of nucleic acid barcode molecules. The sample nucleic acid molecules, or derivatives thereof, can couple or attach to the nucleic acid barcode molecules attached on the support. The resulting barcoded nucleic acid molecules can then be removed from the partition, and in some instances, pooled and sequenced. In such cases, the nucleic acid barcode sequences can be used to trace the origin of the sample nucleic acid molecule. For example, polynucleotides with identical barcodes can be determined to originate from the same biological particle (e.g., cell, cell bead, or nucleus) or partition, while polynucleotides with different barcodes can be determined to originate from different biological particles (e.g., cells, cell beads or nuclei) or partitions.

The samples or reagents can be loaded in the wells or microwells using a variety of approaches. For example, the samples (*e.g.,* a cell, a nucleus, a cell bead, or cellular/nuclear component) or reagents (as described herein) can be loaded into the well or microwell using an external force, *e.g.,* gravitational force, electrical force, magnetic force, or using mechanisms to drive the sample or reagents into the well, for example, via pressure-driven flow, centrifugation, optoelectronics, acoustic loading, electrokinetic pumping, vacuum, capillary flow, etc. In certain cases, a fluid handling system can be used to load the samples or reagents into the well. The loading of the samples or reagents can follow a Poissonian distribution or a non-Poissonian distribution, *e.g.,* super Poisson or sub-Poisson. The geometry, spacing

between wells, density, and size of the microwells can be modified to accommodate a useful sample or reagent distribution; for example, the size and spacing of the microwells can be adjusted such that the sample or reagents can be distributed in a super-Poissonian fashion.

In one non-limiting example, the microwell array or plate includes pairs of microwells, in which each pair of microwells is configured to hold a droplet (*e.g.,* including a single biological particle such as a cell, cell bead or nucleus) and a single bead (such as those described herein, which can, in some instances, also be encapsulated in a droplet). The droplet and the bead (or droplet containing the bead) can be loaded simultaneously or sequentially, and the droplet and the bead can be merged, *e.g.,* upon contact of the droplet and the bead, or upon application of a stimulus (*e.g.,* external force, agitation, heat, light, magnetic or electric force, etc.). In some cases, the loading of the droplet and the bead is super-Poissonian. In other examples of pairs of microwells, the wells are configured to hold two droplets including different reagents and/or samples, which are merged upon contact or upon application of a stimulus. In such instances, the droplet of one microwell of the pair can include reagents that can react with an agent in the droplet of the other microwell of the pair. For example, one droplet can include reagents that are configured to release the nucleic acid barcode molecules of a bead contained in another droplet, located in the adjacent microwell. Upon merging of the droplets, the nucleic acid barcode molecules can be released from the bead into the partition (*e.g.,* the microwell or microwell pair that are in contact), and further processing can be performed (*e.g.,* barcoding, nucleic acid reactions, etc.). In cases where intact or live cells are loaded in the microwells, one of the droplets can include lysis reagents for lysing the cell upon droplet merging.

In some embodiments, a droplet or bead can be partitioned into a well. The droplets can be selected or subjected to pre-processing prior to loading into a well. For instance, the droplets can include biological particles (e.g., cells, cell beads or nuclei), and only certain droplets, such as those containing a single biological particle (or at least one biological particle), can be selected for use in loading of the wells. Such a pre-selection process can be useful in efficient loading of single biological particles (e.g., cells, cell beads, or nuclei), such as to obtain a non-Poissonian distribution, or to pre-filter cells for a selected characteristic prior to further partitioning in the wells. Additionally, the technique can be useful in obtaining or preventing biological particle (e.g., cell, cell bead or nucleus) doublet or multiplet formation prior to or during loading of the microwell.

In some embodiments, the wells can include nucleic acid barcode molecules attached thereto. The nucleic acid barcode molecules can be attached to a surface of the well (*e.g.,* a wall of the well). The nucleic acid barcode molecule (*e.g.,* a partition barcode sequence) of one well can differ from the nucleic acid barcode molecule of another well, which can permit identification of the contents contained with a single partition or well. In some embodiments, the nucleic acid barcode molecule can include a spatial barcode sequence that can identify a spatial coordinate of a well, such as within the well array or well plate. In some embodiments, the nucleic acid barcode molecule can include a unique molecular identifier for individual molecule identification. In some instances, the nucleic acid barcode molecules can be configured to attach to or capture a nucleic acid molecule within a sample or biological particle (e.g., a cell, cell bead or nucleus) distributed in the well. For example, the nucleic acid barcode molecules can include a capture sequence that can be used to capture or hybridize to a nucleic acid molecule (*e.g.,* RNA, DNA) within the sample. In some embodiments, the nucleic acid barcode molecules can be releasable from the microwell. For example, the nucleic acid barcode molecules can include a chemical cross-linker which can be cleaved upon application of a stimulus (*e.g.,* photo-, magnetic, chemical, biological, stimulus). The released nucleic acid barcode molecules, which can be hybridized or configured to hybridize to a sample nucleic acid molecule, can be collected and pooled for further processing, which can include nucleic acid processing (*e.g.,* amplification, extension, reverse transcription, *etc.)* and/or characterization (*e.g.,* sequencing). In such cases, the unique partition barcode sequences can be used to identify the biological particle (e.g., cell, cell bead or nucleus) or partition from which a nucleic acid molecule originated.

Characterization of samples within a well can be performed. Such characterization can include, in non-limiting examples, imaging of the sample (*e.g.,* cell, nucleus, cell bead, or cellular/nuclear components) or derivatives thereof. Characterization techniques such as microscopy or imaging can be useful in measuring sample profiles in fixed spatial locations. For example, when biological particles (e.g., cells, cell beads, or nuclei) are partitioned, optionally with beads, imaging of each microwell and the contents contained therein can provide useful information on biological particle (e.g., cell, cell bead, or nucleus) doublet formation *(e.g.,* frequency, spatial locations, *etc.),* cell-bead pair efficiency, cell viability, cell size, cell morphology, expression level of a biomarker (*e.g.,* a surface marker, a fluorescently labeled molecule therein, *etc.),* cell or bead loading rate, number of cell-bead pairs, *etc.* In some instances, imaging can be used to characterize live cells in the wells, including, but not limited to: dynamic live-cell tracking, cell-cell interactions (when two or more cells are co-partitioned), cell proliferation, *etc.* Alternatively or in addition to, imaging can be used to characterize a quantity of amplification products in the well.

In operation, a well can be loaded with a sample and reagents, simultaneously or sequentially. When biological particles (e.g., cells, nuclei or cell beads) are loaded, the well can be subjected to washing, *e.g.,* to remove excess cells from the well, microwell array, or plate. Similarly, washing can be performed to remove excess beads or other reagents from the well, microwell array, or plate. In the instances where live cells are used, the cells can be lysed in the individual

partitions to release the intracellular components or cellular analytes. Alternatively, the cells or nuclei can be fixed or permeabilized in the individual partitions. The intracellular components or cellular analytes can couple to a support, *e.g.,* on a surface of the microwell, on a solid support (*e.g.,* bead), or they can be collected for further downstream processing. For example, after cell or nucleus lysis, the intracellular components or cellular analytes can be transferred to individual droplets or other partitions for barcoding. Alternatively, or in addition, the intracellular components or cellular analytes (*e.g.,* nucleic acid molecules) can couple to a bead including a nucleic acid barcode molecule; subsequently, the bead can be collected and further processed, *e.g.,* subjected to nucleic acid reaction such as reverse transcription, amplification, or extension, and the nucleic acid molecules thereon can be further characterized, *e.g.,* via sequencing. Alternatively, or in addition, the intracellular components or cellular analytes can be barcoded in the well (*e.g.,* using a bead including nucleic acid barcode molecules that are releasable or on a surface of the microwell including nucleic acid barcode molecules). The barcoded nucleic acid molecules or analytes can be further processed in the well, or the barcoded nucleic acid molecules or analytes can be collected from the individual partitions and subjected to further processing outside the partition. Further processing can include nucleic acid processing (*e.g.,* performing an amplification, extension) or characterization (*e.g.,* fluorescence monitoring of amplified molecules, sequencing). At any suitable or useful step, the well (or microwell array or plate) can be sealed (*e.g.,* using an oil, membrane, wax, etc.), which enables storage of the assay or selective introduction of additional reagents.

Once sealed, the well may be subjected to conditions for further processing of a biological particle (e.g., a cell, a cell bead or a nucleus) in the well. For instance, reagents in the well may allow further processing of the biological particle, e.g., lysis of the cell or nucleus, as further described herein. Alternatively, the well (or wells such as those of a well-based array) comprising the biological particle (e.g., cell, cell bead, or nucleus) may be subjected to freeze-thaw cycling to process the biological particle(s), e.g., lysis of a cell or nucleus. The well containing the biological particle (e.g., cell, cell bead, or nucleus) may be subjected to freezing temperatures (e.g., 0°C, below 0°C, -5°C, -10°C, -15°C, -20°C, -25°C, - 30°C, -35°C, -40°C, -45°C, -50°C, -55°C, -60°C, -65°C, -70°C, -80°C, or -85°C). Freezing may be performed in a suitable manner, e.g., sub-zero freezer or a dry ice/ethanol bath. Following an initial freezing, the well (or wells) comprising the biological particle(s) (e.g., cell(s), cell bead(s), nucleus or nuclei) may be subjected to freeze thaw cycles to lyse biological particle(s). In one embodiment, the initially frozen well (or wells) are thawed to a temperature above freezing (e.g., room temperature or 25°C). In another embodiment, the freezing is performed for less than 10 minutes (e.g., 5 minutes or 7 minutes) followed by thawing at room temperature for less than 10 minutes (e.g., 5 minutes or 7 minutes). This freeze-thaw cycle may be repeated a number of times, e.g., 2, 3, or 4 times, to obtain lysis of the biological particle(s) (e.g., cell(s), cell bead(s), nucleus, or nuclei) in the well (or wells). In one embodiment, the freezing, thawing and/or freeze/thaw cycling is performed in the absence of a lysis buffer.

*Beads*

In some embodiments of the disclosure, a partition can include one or more unique identifiers, such as barcodes (*e.g.,* a plurality of nucleic acid barcode molecules which can be, for example, a plurality of partition barcode sequences). Barcodes can be previously, subsequently or concurrently delivered to the partitions that hold the compartmentalized or partitioned biological particle (*e.g.,* labelled cells such as B cells or nuclei). For example, barcodes can be injected into droplets previous to, subsequent to, or concurrently with droplet generation. In some embodiments, the delivery of the barcodes to a particular partition allows for the later attribution of the characteristics of the individual biological particle (*e.g.,* labelled cells, such as B cells, nuclei, or cell beads) to the particular partition. Barcodes can be delivered, for example on a nucleic acid molecule (*e.g.,* a barcoded oligonucleotide), to a partition via any suitable mechanism. In some embodiments, nucleic acid barcode molecules can be delivered to a partition via a bead. Beads are described in further detail below.

In some embodiments, nucleic acid barcode molecules can be initially associated with the bead and then released from the bead. In some embodiments, release of the nucleic acid barcode molecules can be passive (*e.g.,* by diffusion out of the bead). In addition or alternatively, release from the bead can be upon application of a stimulus which allows the barcoded nucleic acid nucleic acid molecules to dissociate or to be released from the bead. Such stimulus can disrupt the bead, an interaction that couples the nucleic acid barcode molecules to or within the bead, or both. Such stimulus can include, for example, a thermal stimulus, photo-stimulus, chemical stimulus (*e.g.,* change in pH or use of a reducing agent), a mechanical stimulus, a radiation stimulus; a biological stimulus (*e.g.,* enzyme), or any combination thereof. Methods and systems for partitioning barcode carrying beads into droplets are provided in US. Patent Publication Nos. 2019/0367997 and 2019/0064173, and International Application Nos. PCT/US20/17785 and PCT/US20/020486.

Beneficially, a discrete droplet partitioning a biological particle and a barcode carrying bead can effectively allow the attribution of the barcode to macromolecular constituents of the biological particle within the partition. The contents of a partition can remain discrete from the contents of other partitions.

In operation, the barcoded oligonucleotides can be released (*e.g.,* in a partition), as described elsewhere herein. Alternatively, the nucleic acid molecules bound to the bead (*e.g.,* gel bead) can be used to hybridize and capture analytes

(*e.g.,* one or more types of analytes) on the solid phase of the bead.

In some examples, beads, biological particles (*e.g.,* labelled cells, such as B cells, cell beads or nuclei) and droplets can flow along channels (*e.g.,* the channels of a microfluidic device), in some cases at substantially regular flow profiles (*e.g.,* at regular flow rates). Such regular flow profiles can permit a droplet to include a single bead and a single biological particle. Such regular flow profiles can permit the droplets to have an occupancy (*e.g.,* droplets having beads and biological particles) greater than 5%, 10%, 20%, 30%, 40%, 50%, 60%, 70%, 80%, 90%, or 95%. Such regular flow profiles and devices that can be used to provide such regular flow profiles are provided in, for example, U.S. Patent Publication No. 2015/0292988.

A bead can be porous, non-porous, solid, semi-solid, semi-fluidic, fluidic, and/or a combination thereof. In some instances, a bead can be dissolvable, disruptable, and/or degradable. In some cases, a bead cannot be degradable. In some cases, the bead can be a gel bead. A gel bead can be a hydrogel bead. A gel bead can be formed from molecular precursors, such as a polymeric or monomeric species. A semi-solid bead can be a liposomal bead. Solid beads can include metals including iron oxide, gold, and silver. In some cases, the bead can be a silica bead. In some cases, the bead can be rigid. In other cases, the bead can be flexible and/or compressible.

A bead can be of any suitable shape. Examples of bead shapes include, but are not limited to, spherical, non-spherical, oval, oblong, amorphous, circular, cylindrical, and variations thereof.

Beads can be of uniform size or heterogeneous size. In some cases, the diameter of a bead can be at least about 10 nanometers (nm), 100 nm, 500 nm, 1 micrometer (μm), 5μm, 10μm, 20μm, 30μm, 40μm, 50μm, 60μm, 70μm, 80μm, 90μm, 100μm, 250μm, 500μm, 1mm, or greater. In some cases, a bead can have a diameter of less than about 10 nm, 100 nm, 500 nm, 1μm, 5μm, 10μm, 20μm, 30μm, 40μm, 50μm, 60μm, 70μm, 80μm, 90μm, 100μm, 250μm, 500μm, 1mm, or less. In some cases, a bead can have a diameter in the range of about 40-75μm, 30-75μm, 20-75μm, 40-85μm, 40-95μm, 20-100μm, 10-100μm, 1-100μm, 20-250μm, or 20-500μm.

In certain aspects, beads can be provided as a population or plurality of beads having a relatively monodisperse size distribution. Where it may be desirable to provide relatively consistent amounts of reagents within partitions, maintaining relatively consistent bead characteristics, such as size, can contribute to the overall consistency. In some embodiments, the beads described herein can have size distributions that have a coefficient of variation in their cross-sectional dimensions of less than 50%, less than 40%, less than 30%, less than 20%, and in some cases less than 15%, less than 10%, less than 5%, or less.

The beads useful in the methods of the present disclosure can comprise a range of natural and/or synthetic polymeric materials. For example, a bead can comprise a natural polymer, a synthetic polymer or both natural and synthetic polymers. Examples of natural polymers include proteins and sugars such as deoxyribonucleic acid, rubber, cellulose, starch (e.g., amylose, amylopectin), proteins, enzymes, polysaccharides, silks, polyhydroxyalkanoates, chitosan, dextran, collagen, carrageenan, ispaghula, acacia, agar, gelatin, shellac, sterculia gum, xanthan gum, corn sugar gum, guar gum, gum karaya, agarose, alginic acid, alginate, or natural polymers thereof. Examples of synthetic polymers include acrylics, nylons, silicones, spandex, viscose rayon, polycarboxylic acids, polyvinyl acetate, polyacrylamide, polyacrylate, polyethylene glycol, polyurethanes, polylactic acid, silica, polystyrene, polyacrylonitrile, polybutadiene, polycarbonate, polyethylene, polyethylene terephthalate, poly(chlorotrifluoroethylene), poly(ethylene oxide), poly(ethylene terephthalate), polyethylene, polyisobutylene, poly(methyl methacrylate), poly(oxymethylene), polyformaldehyde, polypropylene, polystyrene, poly(tetrafluoroethylene), poly(vinyl acetate), poly(vinyl alcohol), poly(vinyl chloride), poly(vinylidene dichloride), poly(vinylidene difluoride), poly(vinyl fluoride) and/or combinations (*e.g.,* co-polymers) thereof. Beads may also be formed from materials other than polymers, including lipids, micelles, ceramics, glass-ceramics, material composites, metals, other inorganic materials, and others.

In some embodiments, the bead can contain molecular precursors (*e.g.,* monomers or polymers), which can form a polymer network via polymerization of the molecular precursors. In some cases, a precursor can be an already polymerized species capable of undergoing further polymerization via, for example, a chemical cross-linkage. In some embodiments, a precursor can include one or more of an acrylamide or a methacrylamide monomer, oligomer, or polymer. In some cases, the bead can include prepolymers, which are oligomers capable of further polymerization. For example, polyurethane beads can be prepared using prepolymers. In some embodiments, the bead can contain individual polymers that can be further polymerized together. In some cases, beads can be generated via polymerization of different precursors, such that they include mixed polymers, copolymers, and/or block co-polymers. In some embodiments, the bead can include covalent or ionic bonds between polymeric precursors (*e.g.,* monomers, oligomers, linear polymers), nucleic acid molecules (*e.g.,* oligonucleotides), primers, and other entities. In some embodiments, the covalent bonds can be carbon-carbon bonds, thioether bonds, or carbon-heteroatom bonds.

Cross-linking can be permanent or reversible, depending upon the particular cross-linker used. Reversible cross-linking can allow for the polymer to linearize or dissociate under appropriate conditions. In some embodiments, reversible cross-linking can also allow for reversible attachment of a material bound to the surface of a bead. In some embodiments, a cross-linker can form disulfide linkages. In some embodiments, the chemical cross-linker forming disulfide linkages can be cystamine or a modified cystamine.

In some embodiments, disulfide linkages can be formed between molecular precursor units (*e.g.,* monomers, oligomers, or linear polymers) or precursors incorporated into a bead and nucleic acid molecules (*e.g.,* oligonucleotides). Cystamine (including modified cystamines), for example, is an organic agent including a disulfide bond that can be used as a crosslinker agent between individual monomeric or polymeric precursors of a bead. Polyacrylamide can be polymerized in the presence of cystamine or a species including cystamine (*e.g.,* a modified cystamine) to generate polyacrylamide gel beads including disulfide linkages (*e.g.,* chemically degradable beads including chemically-reducible cross-linkers). The disulfide linkages can permit the bead to be degraded (or dissolved) upon exposure of the bead to a reducing agent.

In some embodiments, chitosan, a linear polysaccharide polymer, can be crosslinked with glutaraldehyde via hydrophilic chains to form a bead. Crosslinking of chitosan polymers can be achieved by chemical reactions that are initiated by heat, pressure, change in pH, and/or radiation.

In some embodiments, a bead can include an acrydite moiety, which in certain aspects can be used to attach one or more nucleic acid molecules (*e.g.,* barcode sequence, barcoded nucleic acid molecule, barcoded oligonucleotide, primer, or other oligonucleotide) to the bead. In some cases, an acrydite moiety can refer to an acrydite analogue generated from the reaction of acrydite with one or more species, such as, the reaction of acrydite with other monomers and cross-linkers during a polymerization reaction. Acrydite moieties can be modified to form chemical bonds with a species to be attached, such as a nucleic acid molecule (e.g., barcode sequence, barcoded nucleic acid molecule, barcoded oligonucleotide, primer, or other oligonucleotide). Acrydite moieties can be modified with thiol groups capable of forming a disulfide bond or can be modified with groups already including a disulfide bond. The thiol or disulfide (via disulfide exchange) can be used as an anchor point for a species to be attached or another part of the acrydite moiety can be used for attachment. In some cases, attachment can be reversible, such that when the disulfide bond is broken (e.g., in the presence of a reducing agent), the attached species is released from the bead. In other cases, an acrydite moiety can include a reactive hydroxyl group that can be used for attachment.

Functionalization of beads for attachment of nucleic acid molecules (e.g., oligonucleotides) can be achieved through a wide range of different approaches, including activation of chemical groups within a polymer, incorporation of active or activatable functional groups in the polymer structure, or attachment at the pre-polymer or monomer stage in bead production.

For example, precursors (*e.g.*, monomers, cross-linkers) that are polymerized to form a bead can include acrydite moieties, such that when a bead is generated, the bead also includes acrydite moieties. The acrydite moieties can be attached to a nucleic acid molecule (*e.g.*, oligonucleotide), which can include a priming sequence (*e.g.*, a primer for amplifying target nucleic acids, random primer, primer sequence for messenger RNA) and/or one or more barcode sequences. The one or more barcode sequences can include sequences that are the same for all nucleic acid molecules coupled to a given bead and/or sequences that are different across all nucleic acid molecules coupled to the given bead. The nucleic acid molecule can be incorporated into the bead.

In some embodiments, the nucleic acid molecule can include a functional sequence, for example, for attachment to a sequencing flow cell, such as, for example, a P5 sequence for Illumina® sequencing. In some cases, the nucleic acid molecule or derivative thereof (*e.g.*, oligonucleotide or polynucleotide generated from the nucleic acid molecule) can include another functional sequence, such as, for example, a P7 sequence for attachment to a sequencing flow cell for Illumina sequencing. In some cases, the nucleic acid molecule can include a barcode sequence. In some cases, the primer can further include a unique molecular identifier (UMI). In some cases, the primer can include an R1 primer sequence for Illumina sequencing. In some cases, the primer can include an R2 primer sequence for Illumina sequencing. Examples of such nucleic acid molecules (*e.g.*, oligonucleotides, polynucleotides, etc.) and uses thereof, as can be used with compositions, devices, methods and systems of the present disclosure, are provided in U.S. Patent Pub. Nos. 2014/0378345 and 2015/0376609.

**FIG. 23** illustrates an example of a barcode carrying bead. A nucleic acid molecule **1502**, such as an oligonucleotide, can be coupled to a bead **1504** by a releasable linkage **1506**, such as, for example, a disulfide linker. The same bead **1504** can be coupled (*e.g.*, via releasable linkage) to one or more other nucleic acid molecules **1518, 1520.** The nucleic acid molecule **1502** can be or include a barcode. As noted elsewhere herein, the structure of the barcode can include a number of sequence elements. The nucleic acid molecule **1502** can include a functional sequence **1508** that can be used in subsequent processing. For example, the functional sequence **1508** can include one or more of a sequencer specific flow cell attachment sequence (*e.g.*, a P5 sequence for Illumina® sequencing systems) and a sequencing primer sequence (*e.g.*, a R1 primer for Illumina® sequencing systems). The nucleic acid molecule **1502** can include a barcode sequence **1510** for use in barcoding the sample (*e.g.*, DNA, RNA, protein, *etc*.). In some cases, the barcode sequence **1510** can be bead-specific such that the barcode sequence **1510** is common to all nucleic acid molecules (*e.g.*, including nucleic acid molecule **1502**) coupled to the same bead **1504.** Alternatively or in addition, the barcode sequence **1510** can be partition-specific such that the barcode sequence **1510** is common to all nucleic acid molecules coupled to one or more beads that are partitioned into the same partition. The nucleic acid molecule **1502** can include a specific priming sequence **1512**, such as an mRNA specific priming sequence (*e.g.*, poly-T sequence), a

targeted priming sequence, and/or a random priming sequence. The nucleic acid molecule **1502** can include an anchoring sequence **1514** to ensure that the specific priming sequence **1512** hybridizes at the sequence end (*e.g.*, of the mRNA). For example, the anchoring sequence **1514** can include a random short sequence of nucleotides, such as a 1-mer, 2-mer, 3-mer or longer sequence, which can ensure that a poly-T segment is more likely to hybridize at the sequence end of the poly-A tail of the mRNA.

The nucleic acid molecule **1502** can include a unique molecular identifying sequence **1516** (*e.g.*, unique molecular identifier (UMI)). In some cases, the unique molecular identifying sequence **1516** can include from about 5 to about 8 nucleotides. Alternatively, the unique molecular identifying sequence **1516** can compress less than about 5 or more than about 8 nucleotides. The unique molecular identifying sequence **1516** can be a unique sequence that varies across individual nucleic acid molecules (e.g., **1502, 1518, 1520,** *etc.)* coupled to a single bead (*e.g.*, bead **1504**). In some cases, the unique molecular identifying sequence **1516** can be a random sequence (*e.g.*, such as a random N-mer sequence). For example, the UMI can provide a unique identifier of the starting mRNA molecule that was captured, in order to allow quantitation of the number of original expressed RNA. As will be appreciated, although **FIG. 23** shows three nucleic acid molecules **1502, 1518, 1520** coupled to the surface of the bead **1504**, an individual bead can be coupled to any number of individual nucleic acid molecules, for example, from one to tens to hundreds of thousands or even millions of individual nucleic acid molecules. The respective barcodes for the individual nucleic acid molecules can include both common sequence segments or relatively common sequence segments *(e.g.,* **1508**, **1510**, **1512**, *etc.*) and variable or unique sequence segments (*e.g.*, **1516**) between different individual nucleic acid molecules coupled to the same bead.

In operation, a biological particle *(e.g.,* cell, cell bead, nucleus, DNA, RNA, *etc.*) can be co-partitioned along with a barcode bearing bead **1504.** The nucleic acid barcode molecules **1502, 1518, 1520** can be released from the bead **1504** in the partition. By way of example, in the context of analyzing sample RNA, the poly-T segment (*e.g.*, **1512**) of one of the released nucleic acid molecules (*e.g.*, **1502**) can hybridize to the poly-A tail of a mRNA molecule. Reverse transcription can result in a cDNA transcript of the mRNA, but which transcript includes each of the sequence segments **1508, 1510, 1516** of the nucleic acid molecule **1502.** Because the nucleic acid molecule **1502** includes an anchoring sequence **1514**, it will more likely hybridize to and prime reverse transcription at the sequence end of the poly-A tail of the mRNA. Within any given partition, all of the cDNA transcripts of the individual mRNA molecules can include a common barcode sequence segment **1510.** However, the transcripts made from the different mRNA molecules within a given partition can vary at the unique molecular identifying sequence **1512** segment (*e.g.*, UMI segment). Beneficially, even following any subsequent amplification of the contents of a given partition, the number of different UMIs can be indicative of the quantity of mRNA originating from a given partition, and thus from the biological particle (*e.g.*, cell, nucleus, or cell bead). As noted above, the transcripts can be amplified, cleaned up and sequenced to identify the sequence of the cDNA transcript of the mRNA, as well as to sequence the barcode segment and the UMI segment. While a poly-T primer sequence is described, other targeted or random priming sequences can also be used in priming the reverse transcription reaction. Likewise, although described as releasing the barcoded oligonucleotides into the partition, in some cases, the nucleic acid molecules bound to the bead (*e.g.*, gel bead) can be used to hybridize and capture the mRNA on the solid phase of the bead, for example, in order to facilitate the separation of the RNA from other cell or nuclear contents. In such cases, further processing can be performed, in the partitions or outside the partitions (*e.g.*, in bulk). For instance, the RNA molecules on the beads can be subjected to reverse transcription or other nucleic acid processing, additional adapter sequences can be added to the barcoded nucleic acid molecules, or other nucleic acid reactions (*e.g.*, amplification, nucleic acid extension) can be performed. The beads or products thereof (*e.g.*, barcoded nucleic acid molecules) can be collected from the partitions, and/or pooled together and subsequently subjected to clean up and further characterization (*e.g.*, sequencing).

The operations described herein can be performed at any useful or suitable step. For instance, the beads including nucleic acid barcode molecules can be introduced into a partition (*e.g.*, well or droplet) prior to, during, or following introduction of a sample into the partition. The nucleic acid molecules of a sample can be subjected to barcoding, which can occur on the bead (in cases where the nucleic acid molecules remain coupled to the bead) or following release of the nucleic acid barcode molecules into the partition. In cases where the nucleic acid molecules from the sample remain attached to the bead, the beads from various partitions can be collected, pooled, and subjected to further processing (*e.g.*, reverse transcription, adapter attachment, amplification, clean up, and/or sequencing). In other instances, the processing can occur in the partition. For example, conditions sufficient for barcoding, adapter attachment, reverse transcription, or other nucleic acid processing operations can be provided in the partition and performed prior to clean up and sequencing.

In some instances, a bead can include a capture sequence or binding sequence configured to bind to a corresponding capture sequence or binding sequence. In some instances, a bead can include a plurality of different capture sequences or binding sequences configured to bind to different respective corresponding capture sequences or binding sequences. For example, a bead can include a first subset of one or more capture sequences each configured to bind to a first corresponding capture sequence, a second subset of one or more capture sequences each configured to bind

to a second corresponding capture sequence, a third subset of one or more capture sequences each configured to bind to a third corresponding capture sequence, and etc. A bead can include any number of different capture sequences. In some instances, a bead can include at least 2, 3, 4, 5, 6, 7, 8, 9, 10 or more different capture sequences or binding sequences configured to bind to different respective capture sequences or binding sequences, respectively. Alternatively or in addition, a bead can include at most about 10, 9, 8, 7, 6, 5, 4, 3, or 2 different capture sequences or binding sequences configured to bind to different respective capture sequences or binding sequences. In some instances, the different capture sequences or binding sequences can be configured to facilitate analysis of a same type of analyte. In some instances, the different capture sequences or binding sequences can be configured to facilitate analysis of different types of analytes (with the same bead). The capture sequence can be designed to attach to a corresponding capture sequence. Beneficially, such corresponding capture sequence can be introduced to, or otherwise induced in, a biological particle (*e.g.*, cell, nucleus, cell bead, etc.) for performing different assays in various formats (*e.g.*, barcoded antibodies including the corresponding capture sequence, barcoded MHC dextramers including the corresponding capture sequence, barcoded guide RNA molecules including the corresponding capture sequence, etc.), such that the corresponding capture sequence can later interact with the capture sequence associated with the bead. In some instances, a capture sequence coupled to a bead (or other support) can be configured to attach to a linker molecule, such as a splint molecule, wherein the linker molecule is configured to couple the bead (or other support) to other molecules through the linker molecule, such as to one or more analytes or one or more other linker molecules.

**FIG. 24** illustrates a non-limiting example of a barcode carrying bead in accordance with some embodiments of the disclosure. A nucleic acid molecule **1605,** such as an oligonucleotide, can be coupled to a bead **1604** by a releasable linkage **1606,** such as, for example, a disulfide linker. The nucleic acid molecule **1605** can include a first capture sequence **1660.** The same bead **1604** can be coupled, *e.g.*, via releasable linkage, to one or more other nucleic acid molecules **1603, 1607** including other capture sequences. The nucleic acid molecule **1605** can be or include a barcode. As described elsewhere herein, the structure of the barcode can include a number of sequence elements, such as a functional sequence **1608** (*e.g.*, flow cell attachment sequence, sequencing primer sequence, *etc.*), a barcode sequence **1610** (*e.g.*, bead-specific sequence common to bead, partition-specific sequence common to partition, *etc.*), and a unique molecular identifier **1612** (*e.g.*, unique sequence within different molecules attached to the bead), or partial sequences thereof. The capture sequence **1660** can be configured to attach to a corresponding capture sequence **1665** (*e.g.*, capture handle). In some instances, the corresponding capture sequence **1665** can be coupled to another molecule that can be an analyte or an intermediary carrier. For example, as illustrated in **FIG. 24**, the corresponding capture sequence **1665** is coupled to a guide RNA molecule **1662** including a target sequence **1664**, wherein the target sequence

**1664** is configured to attach to the analyte. Another oligonucleotide molecule **1607** attached to the bead **1604** includes a second capture sequence **1680** which is configured to attach to a second corresponding capture sequence (*e.g.*, capture handle) **1685.** As illustrated in **FIG. 24**, the second corresponding capture sequence **1685** is coupled to an antibody **1682.** In some cases, the antibody **1682** can have binding specificity to an analyte (*e.g.*, surface protein). Alternatively, the antibody **1682** cannot have binding specificity. Another oligonucleotide molecule **1603** attached to the bead **1604** includes a third capture sequence **470** which is configured to attach to a second corresponding capture sequence **1675**. As illustrated in **FIG. 24**, the third corresponding capture sequence (*e.g.*, capture handle) **1675** is coupled to a molecule **1672**. The molecule **1672** may or may not be configured to target an analyte. The other oligonucleotide molecules **1603**, **1607** can include the other sequences (*e.g.*, functional sequence, barcode sequence, UMI, *etc.*) described with respect to oligonucleotide molecule **1605**. While a single oligonucleotide molecule including each capture sequence is illustrated in **FIG. 24**, it will be appreciated that, for each capture sequence, the bead can include a set of one or more oligonucleotide molecules each

including the capture sequence. For example, the bead can include any number of sets of one or more different capture sequences. Alternatively or in addition, the bead **1604** can include other capture sequences. Alternatively or in addition, the bead **1604** can include fewer types of capture sequences (*e.g.*, two capture sequences). Alternatively or in addition, the bead **1604** can include oligonucleotide molecule(s) including a priming sequence, such as a specific priming sequence such as an mRNA specific priming sequence (*e.g.*, poly-T sequence), a targeted priming sequence, and/or a random priming sequence, for example, to facilitate an assay for gene expression.

The generation of a barcoded sequence, see, *e.g.*, **FIG. 23**, is described herein.

In some embodiments, precursors including a functional group that is reactive or capable of being activated such that it becomes reactive can be polymerized with other precursors to generate gel beads including the activated or activatable functional group. The functional group can then be used to attach additional species (*e.g.*, disulfide linkers, primers, other oligonucleotides, *etc.*) to the gel beads. For example, some precursors including a carboxylic acid (COOH) group can co-polymerize with other precursors to form a gel bead that also includes a COOH functional group. In some cases, acrylic acid (a species including free COOH groups), acrylamide, and bis(acryloyl)cystamine can be co-polymerized together to generate a gel bead including free COOH groups. The COOH groups of the gel bead can be activated (*e.g.*, via 1-Ethyl-3-(3-dimethylaminopropyl)carbodiimide (EDC) and N-Hydroxysuccinimide (NHS) or 4-(4,6-Dimethoxy-1,3,5-triazin-2-yl)-4-methylmorpholinium chloride (DMTMM)) such that they are reactive (*e.g.*, reactive to amine functional

groups where EDC/NHS or DMTMM are used for activation). The activated COOH groups can then react with an appropriate species (*e.g.*, a species including an amine functional group where the carboxylic acid groups are activated to be reactive with an amine functional group) including a moiety to be linked to the bead.

Beads including disulfide linkages in their polymeric network can be functionalized with additional species via reduction of some of the disulfide linkages to free thiols. The disulfide linkages can be reduced via, for example, the action of a reducing agent (*e.g.*, DTT, TCEP, etc.) to generate free thiol groups, without dissolution of the bead. Free thiols of the beads can then react with free thiols of a species or a species including another disulfide bond (*e.g.*, via thiol-disulfide exchange) such that the species can be linked to the beads (*e.g.*, via a generated disulfide bond). In some cases, free thiols of the beads can react with any other suitable group. For example, free thiols of the beads can react with species including an acrydite moiety. The free thiol groups of the beads can react with the acrydite via Michael addition chemistry, such that the species including the acrydite is linked to the bead. In some cases, uncontrolled reactions can be prevented by inclusion of a thiol capping agent such as N-ethylmaleimide or iodoacetate.

Activation of disulfide linkages within a bead can be controlled such that only a small number of disulfide linkages are activated. Control can be exerted, for example, by controlling the concentration of a reducing agent used to generate free thiol groups and/or concentration of reagents used to form disulfide bonds in bead polymerization. In some cases, a low concentration (*e.g.*, molecules of reducing agent:gel bead ratios of less than or equal to about 1:100,000,000,000, less than or equal to about 1:10,000,000,000, less than or equal to about 1:1,000,000,000, less than or equal to about 1:100,000,000, less than or equal to about 1:10,000,000, less than or equal to about 1:1,000,000, less than or equal to about 1:100,000, less than or equal to about 1:10,000) of reducing agent can be used for reduction. Controlling the number of disulfide linkages that are reduced to free thiols can be useful in ensuring bead structural integrity during functionalization. In some cases, optically-active agents, such as fluorescent dyes can be coupled to beads via free thiol groups of the beads and used to quantify the number of free thiols present in a bead and/or track a bead.

In some embodiments, addition of moieties to a gel bead after gel bead formation can be advantageous. For example, addition of an oligonucleotide (*e.g.*, barcoded oligonucleotide, such as a barcoded nucleic acid molecule) after gel bead formation can avoid loss of the species during chain transfer termination that can occur during polymerization. Moreover, smaller precursors (*e.g.*, monomers or cross linkers that do not include side chain groups and linked moieties) can be used for polymerization and can be minimally hindered from growing chain ends due to viscous effects. In some cases, functionalization after gel bead synthesis can minimize exposure of species (*e.g.*, oligonucleotides) to be loaded with potentially damaging agents (*e.g.*, free radicals) and/or chemical environments. In some cases, the generated gel can possess an upper critical solution temperature (UCST) that can permit temperature driven swelling and collapse of a bead. Such functionality can aid in oligonucleotide (*e.g.*, a primer) infiltration into the bead during subsequent functionalization of the bead with the oligonucleotide. Post-production functionalization can also be useful in controlling loading ratios of species in beads, such that, for example, the variability in loading ratio is minimized. Species loading can also be performed in a batch process such that a plurality of beads can be functionalized with the species in a single batch.

A bead injected or otherwise introduced into a partition can include releasably, cleavably, or reversibly attached barcodes (*e.g.*, partition barcode sequences). A bead injected or otherwise introduced into a partition can include activatable barcodes. A bead injected or otherwise introduced into a partition can be degradable, disruptable, or dissolvable beads.

Barcodes can be releasably, cleavably or reversibly attached to the beads such that barcodes can be released or be releasable through cleavage of a linkage between the barcode molecule and the bead, or released through degradation of the underlying bead itself, allowing the barcodes to be accessed or be accessible by other reagents, or both. In non-limiting examples, cleavage can be achieved through reduction of di-sulfide bonds, use of restriction enzymes, photo-activated cleavage, or cleavage via other types of stimuli (*e.g.*, chemical, thermal, pH, enzymatic, etc.) and/or reactions, such as described elsewhere herein. Releasable barcodes can sometimes be referred to as being activatable, in that they are available for reaction once released. Thus, for example, an activatable barcode can be activated by releasing the barcode from a bead (or other suitable type of partition described herein). Other activatable configurations are also envisioned in the context of the described methods and systems.

In addition to, or as an alternative to the cleavable linkages between the beads and the associated molecules, such as barcode containing nucleic acid molecules (*e.g.*, barcoded oligonucleotides), the beads can be degradable, disruptable, or dissolvable spontaneously or upon exposure to one or more stimuli (*e.g.*, temperature changes, pH changes, exposure to particular chemical species or phase, exposure to light, reducing agent, etc.). In some cases, a bead can be dissolvable, such that material components of the beads are solubilized when exposed to a particular chemical species or an environmental change, such as a change temperature or a change in pH. In some cases, a gel bead can be degraded or dissolved at elevated temperature and/or in basic conditions. In some cases, a bead can be thermally degradable such that when the bead is exposed to an appropriate change in temperature (*e.g.*, heat), the bead degrades. Degradation or dissolution of a bead bound to a species (*e.g.*, a nucleic acid molecule, *e.g.*, barcoded oligonucleotide) can result in release of the species from the bead.

As will be appreciated from the above disclosure, the degradation of a bead can refer to the disassociation of a bound (*e.g.*, capture agent configured to couple to a secreted antibody or antigen-binding fragment thereof) or entrained species (*e.g.*, labelled B cells, or memory B cells, or secreted antibody or antigen-binding fragment thereof) from a bead, both with and without structurally degrading the physical bead itself. For example, the degradation of the bead can involve cleavage of a cleavable linkage via one or more species and/or methods described elsewhere herein. In another example, entrained species can be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead can cause a bead to better retain an entrained species due to pore size contraction.

A degradable bead can be introduced into a partition, such as a droplet of an emulsion or a well, such that the bead degrades within the partition and any associated species (*e.g.*, oligonucleotides) are released within the droplet when the appropriate stimulus is applied. The free species (*e.g.*, oligonucleotides, nucleic acid molecules) can interact with other reagents contained in the partition. For example, a polyacrylamide bead including cystamine and linked, via a disulfide bond, to a barcode sequence, can be combined with a reducing agent within a droplet of a water-in-oil emulsion. Within the droplet, the reducing agent can break the various disulfide bonds, resulting in bead degradation and release of the barcode sequence into the aqueous, inner environment of the droplet. In another example, heating of a droplet including a bead-bound barcode sequence in basic solution can also result in bead degradation and release of the attached barcode sequence into the aqueous, inner environment of the droplet.

Any suitable number of molecular tag molecules (*e.g.*, primer, barcoded oligonucleotide) can be associated with a bead such that, upon release from the bead, the molecular tag molecules (*e.g.*, primer, *e.g.*, barcoded oligonucleotide) are present in the partition at a pre-defined concentration. Such pre-defined concentration can be selected to facilitate certain reactions for generating a sequencing library, *e.g.*, amplification, within the partition. In some cases, the pre-defined concentration of the primer can be limited by the process of producing nucleic acid molecule (*e.g.*, oligonucleotide) bearing beads.

In some cases, beads can be non-covalently loaded with one or more reagents. The beads can be non-covalently loaded by, for instance, subjecting the beads to conditions sufficient to swell the beads, allowing sufficient time for the reagents to diffuse into the interiors of the beads, and subjecting the beads to conditions sufficient to de-swell the beads. The swelling of the beads can be accomplished, for instance, by placing the beads in a thermodynamically favorable solvent, subjecting the beads to a higher or lower temperature, subjecting the beads to a higher or lower ion concentration, and/or subjecting the beads to an electric field. The swelling of the beads can be accomplished by various swelling methods. The de-swelling of the beads can be accomplished, for instance, by transferring the beads in a thermodynamically unfavorable solvent, subjecting the beads to lower or high temperatures, subjecting the beads to a lower or higher ion concentration, and/or removing an electric field. The de-swelling of the beads can be accomplished by various de-swelling methods. Transferring the beads can cause pores in the bead to shrink. The shrinking can then hinder reagents within the beads from diffusing out of the interiors of the beads. The hindrance can be due to steric interactions between the reagents and the interiors of the beads. The transfer can be accomplished microfluidically. For instance, the transfer can be achieved by moving the beads from one co-flowing solvent stream to a different co-flowing solvent stream. The swellability and/or pore size of the beads can be adjusted by changing the polymer composition of the bead.

In some cases, an acrydite moiety linked to a precursor, another species linked to a precursor, or a precursor itself can include a labile bond, such as chemically, thermally, or photo-sensitive bond *e.g.*, disulfide bond, UV sensitive bond, or the like. Once acrydite moieties or other moieties including a labile bond are incorporated into a bead, the bead can also include the labile bond. The labile bond can be, for example, useful in reversibly linking (*e.g.*, covalently linking) species (*e.g.*, barcodes, primers, etc.) to a bead. In some cases, a thermally labile bond can include a nucleic acid hybridization based attachment, *e.g.*, where an oligonucleotide is hybridized to a complementary sequence that is attached to the bead, such that thermal melting of the hybrid releases the oligonucleotide, *e.g.*, a barcode containing sequence, from the bead.

The addition of multiple types of labile bonds to a gel bead can result in the generation of a bead capable of responding to varied stimuli. Each type of labile bond can be sensitive to an associated stimulus (*e.g.*, chemical stimulus, light, temperature, enzymatic, etc.) such that release of species attached to a bead via each labile bond can be controlled by the application of the appropriate stimulus. Such functionality can be useful in controlled release of species from a gel bead. In some cases, another species including a labile bond can be linked to a gel bead after gel bead formation via, for example, an activated functional group of the gel bead as described above. As will be appreciated, barcodes that are releasably, cleavably or reversibly attached to the beads described herein include barcodes that are released or releasable through cleavage of a linkage between the barcode molecule and the bead, or that are released through degradation of the underlying bead itself, allowing the barcodes to be accessed or accessible by other reagents, or both.

The barcodes that are releasable as described herein can sometimes be referred to as being activatable, in that they are available for reaction once released. Thus, for example, an activatable barcode can be activated by releasing

the barcode from a bead (or other suitable type of partition described herein). Other activatable configurations are also envisioned in the context of the described methods and systems.

In addition to thermally cleavable bonds, disulfide bonds and UV sensitive bonds, other non-limiting examples of labile bonds that can be coupled to a precursor or bead include an ester linkage (*e.g.*, cleavable with an acid, a base, or hydroxylamine), a vicinal diol linkage (*e.g.*, cleavable via sodium periodate), a Diels-Alder linkage (*e.g.*, cleavable via heat), a sulfone linkage (*e.g.*, cleavable via a base), a silyl ether linkage (*e.g.*, cleavable via an acid), a glycosidic linkage (*e.g.*, cleavable via an amylase), a peptide linkage (*e.g.*, cleavable via a protease), or a phosphodiester linkage (*e.g.*, cleavable via a nuclease (*e.g.*, DNAse)). A bond can be cleavable via other nucleic acid molecule targeting enzymes, such as restriction enzymes (*e.g.*, restriction endonucleases), as described further below.

Species can be encapsulated in beads (*e.g.*, capture agent) during bead generation (e.g., during polymerization of precursors). Such species may or may not participate in polymerization. Such species can be entered into polymerization reaction mixtures such that generated beads include the species upon bead formation. In some cases, such species can be added to the gel beads after formation. Such species can include, for example, nucleic acid molecules (*e.g.*, oligonucleotides), reagents for a nucleic acid amplification reaction (*e.g.*, primers, polymerases, dNTPs, co-factors (*e.g.*, ionic co-factors, buffers) including those described herein, reagents for enzymatic reactions (*e.g.*, enzymes, co-factors, substrates, buffers), reagents for nucleic acid modification reactions such as polymerization, ligation, or digestion, and/or reagents for template preparation (*e.g.*, tagmentation) for one or more sequencing platforms (*e.g.*, Nextera® for Illumina®). Such species can include one or more enzymes described herein, including without limitation, polymerase, reverse transcriptase, restriction enzymes (*e.g.*, endonuclease), transposase, ligase, proteinase K, DNAse, etc. Such species can include one or more reagents described elsewhere herein (*e.g.*, lysis agents, inhibitors, inactivating agents, chelating agents, stimulus). Trapping of such species can be controlled by the polymer network density generated during polymerization of precursors, control of ionic charge within the gel bead (*e.g.*, via ionic species linked to polymerized species), or by the release of other species. Encapsulated species can be released from a bead upon bead degradation and/or by application of a stimulus capable of releasing the species from the bead. Alternatively or in addition, species can be partitioned in a partition (*e.g.*, droplet) during or subsequent to partition formation. Such species can include, without limitation, the abovementioned species that can also be encapsulated in a bead.

A degradable bead can include one or more species with a labile bond such that, when the bead/species is exposed to the appropriate stimuli, the bond is broken and the bead degrades. The labile bond can be a chemical bond (*e.g.*, covalent bond, ionic bond) or can be another type of physical interaction (*e.g.*, van der Waals interactions, dipole-dipole interactions, etc.). In some cases, a crosslinker used to generate a bead can include a labile bond. Upon exposure to the appropriate conditions, the labile bond can be broken and the bead degraded. For example, upon exposure of a polyacrylamide gel bead including cystamine crosslinkers to a reducing agent, the disulfide bonds of the cystamine can be broken and the bead degraded.

A degradable bead can be useful in more quickly releasing an attached species (*e.g.*, a nucleic acid molecule, a barcode sequence, a primer, etc.) from the bead when the appropriate stimulus is applied to the bead as compared to a bead that does not degrade. For example, for a species bound to an inner surface of a porous bead or in the case of an encapsulated species, the species can have greater mobility and accessibility to other species in solution upon degradation of the bead. In some cases, a species can also be attached to a degradable bead via a degradable linker (*e.g.*, disulfide linker). The degradable linker can respond to the same stimuli as the degradable bead or the two degradable species can respond to different stimuli. For example, a barcode sequence can be attached, via a disulfide bond, to a polyacrylamide bead including cystamine. Upon exposure of the barcoded-bead to a reducing agent, the bead degrades and the barcode sequence is released upon breakage of both the disulfide linkage between the barcode sequence and the bead and the disulfide linkages of the cystamine in the bead.

As will be appreciated from the above disclosure, while referred to as degradation of a bead, in many instances as noted above, that degradation can refer to the disassociation of a bound or entrained species from a bead, both with and without structurally degrading the physical bead itself. For example, entrained species can be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead can cause a bead to better retain an entrained species due to pore size contraction.

Where degradable beads are provided, it can be beneficial to avoid exposing such beads to the stimulus or stimuli that cause such degradation prior to a given time, in order to, for example, avoid premature bead degradation and issues that arise from such degradation, including for example poor flow characteristics and aggregation. By way of example, where beads include reducible cross-linking groups, such as disulfide groups, it will be desirable to avoid contacting such beads with reducing agents, *e.g.*, DTT or other disulfide cleaving reagents. In such cases, treatment to the beads described herein will, in some cases be provided free of reducing agents, such as DTT. Because reducing agents are often provided in commercial enzyme preparations, it can be desirable to provide reducing agent free (or

DTT free) enzyme preparations in treating the beads described herein. Examples of such enzymes include, *e.g.*, polymerase enzyme preparations, reverse transcriptase enzyme preparations, ligase enzyme preparations, as well as many other enzyme preparations that can be used to treat the beads described herein. The terms "reducing agent free" or "DTT free" preparations can refer to a preparation having less than about 1/10th, less than about 1/50th, or even less than about 1/100th of the lower ranges for such materials used in degrading the beads. For example, for DTT, the reducing agent free preparation can have less than about 0.01 millimolar (mM), 0.005 mM, 0.001 mM DTT, 0.0005 mM DTT, or even less than about 0.0001 mM DTT. In many cases, the amount of DTT can be undetectable.

Numerous chemical triggers can be used to trigger the degradation of beads. Examples of these chemical changes can include, but are not limited to pH-mediated changes to the integrity of a component within the bead, degradation of a component of a bead via cleavage of cross-linked bonds, and depolymerization of a component of a bead.

In some embodiments, a bead can be formed from materials that include degradable chemical crosslinkers, such as BAC or cystamine. Degradation of such degradable crosslinkers can be accomplished through a number of mechanisms. In some examples, a bead can be contacted with a chemical degrading agent that can induce oxidation, reduction or other chemical changes. For example, a chemical degrading agent can be a reducing agent, such as dithiothreitol (DTT). Additional examples of reducing agents can include β-mercaptoethanol, (2S)-2-amino-1,4-dimercaptobutane (dithiobutylamine or DTBA), tris(2-carboxyethyl) phosphine (TCEP), or combinations thereof. A reducing agent can degrade the disulfide bonds formed between gel precursors forming the bead, and thus, degrade the bead. In other cases, a change in pH of a solution, such as an increase in pH, can trigger degradation of a bead. In other cases, exposure to an aqueous solution, such as water, can trigger hydrolytic degradation, and thus degradation of the bead. In some cases, any combination of stimuli can trigger degradation of a bead. For example, a change in pH can enable a chemical agent (*e.g.*, DTT) to become an effective reducing agent.

Beads can also be induced to release their contents upon the application of a thermal stimulus. A change in temperature can cause a variety of changes to a bead. For example, heat can cause a solid bead to liquefy. A change in heat can cause melting of a bead such that a portion of the bead degrades. In other cases, heat can increase the internal pressure of the bead components such that the bead ruptures or explodes. Heat can also act upon heat-sensitive polymers used as materials to construct beads.

Any suitable agent can degrade beads. In some embodiments, changes in temperature or pH can be used to degrade thermo-sensitive or pH-sensitive bonds within beads. In some embodiments, chemical degrading agents can be used to degrade chemical bonds within beads by oxidation, reduction or other chemical changes. For example, a chemical degrading agent can be a reducing agent, such as DTT, wherein DTT can degrade the disulfide bonds formed between a crosslinker and gel precursors, thus degrading the bead. In some embodiments, a reducing agent can be added to degrade the bead, which may or may not cause the bead to release its contents. Examples of reducing agents can include dithiothreitol (DTT), β-mercaptoethanol, (2S)-2-amino-1,4-dimercaptobutane (dithiobutylamine or DTBA), tris(2-carboxyethyl) phosphine (TCEP), or combinations thereof. The reducing agent can be present at a concentration of about 0.1mM, 0.5mM, 1mM, 5mM, or 10mM. The reducing agent can be present at a concentration of at least about 0.1mM, 0.5mM, 1mM, 5mM, 10mM, or greater than 10 mM. The reducing agent can be present at concentration of at most about 10mM, 5mM, 1mM, 0.5mM, 0.1mM, or less.

Any suitable number of molecular tag molecules (*e.g.,* primer, barcoded oligonucleotide) can be associated with a bead such that, upon release from the bead, the molecular tag molecules (*e.g.*, primer, *e.g.*, barcoded oligonucleotide) are present in the partition at a pre-defined concentration. Such pre-defined concentration can be selected to facilitate certain reactions for generating a sequencing library, *e.g.*, amplification, within the partition. In some cases, the pre-defined concentration of the primer can be limited by the process of producing oligonucleotide bearing beads.

Although **FIG. 13** and **FIG. 16** have been described in terms of providing substantially singly occupied partitions, above, in certain cases, it may be desirable to provide multiply occupied partitions, *e.g.*, containing two, three, four or more biological particles (*e.g.*, cells, cell beads, or nuclei) and/or beads including nucleic acid barcode molecules (*e.g.*, oligonucleotides) within a single partition (*e.g.*, multi-omics method described elsewhere, herein). Accordingly, as noted above, the flow characteristics of the biological particle and/or bead containing fluids and partitioning fluids can be controlled to provide for such multiply occupied partitions. In particular, the flow parameters can be controlled to provide a given occupancy rate at greater than about 50% of the partitions, greater than about 75%, and in some cases greater than about 80%, 90%, 95%, or higher.

In some cases, additional beads can be used to deliver additional reagents to a partition. In such cases, it can be advantageous to introduce different beads into a common channel or droplet generation junction, from different bead sources (*e.g.*, containing different associated reagents) through different channel inlets into such common channel or droplet generation junction (*e.g.*, junction **1210**). In such cases, the flow and frequency of the different beads into the channel or junction can be controlled to provide for a certain ratio of beads from each source, while ensuring a given pairing or combination of such beads into a partition with a given number of biological particles (*e.g.*, one biological particle and one bead per partition).

The partitions described herein can include small volumes, for example, less than about 10 microliters (μL),

5μL, 1μL, 900 picoliters (pL), 800 pL, 700 pL, 600 pL, 500 pL, 400pL, 300 pL, 200 pL, 100pL, 50 pL, 20 pL, 10 pL, 1 pL, 500 nanoliters (nL), 100 nL, 50 nL, or less.

For example, in the case of droplet based partitions, the droplets can have overall volumes that are less than about 1000 pL, 900 pL, 800 pL, 700 pL, 600 pL, 500 pL, 400pL, 300 pL, 200 pL, 100pL, 50 pL, 20 pL, 10 pL, 1 pL, or less. Where co-partitioned with beads, it will be appreciated that the sample fluid volume, *e.g.*, including co-partitioned biological particles and/or beads, within the partitions can be less than about 90% of the above described volumes, less than about 80%, less than about 70%, less than about 60%, less than about 50%, less than about 40%, less than about 30%, less than about 20%, or less than about 10% of the above described volumes.

As is described elsewhere herein, partitioning species can generate a population or plurality of partitions. In such cases, any suitable number of partitions can be generated or otherwise provided. For example, at least about 1,000 partitions, at least about 5,000 partitions, at least about 10,000 partitions, at least about 50,000 partitions, at least about 100,000 partitions, at least about 500,000 partitions, at least about 1,000,000 partitions, at least about 5,000,000 partitions at least about 10,000,000 partitions, at least about 50,000,000 partitions, at least about 100,000,000 partitions, at least about 500,000,000 partitions, at least about 1,000,000,000 partitions, or more partitions can be generated or otherwise provided. Moreover, the plurality of partitions can include both unoccupied partitions (*e.g.*, empty partitions) and occupied partitions.

*Reagents*

In accordance with certain aspects, biological particles can be partitioned along with lysis reagents in order to release the contents of the biological particles within the partition. See, *e.g.*, U.S. Pat. Pub. 2018/0216162 (now U.S. Pat. 10,428,326), U.S. Pat. Pub. 2019/0100632 (now U.S. Pat. 10,590,244), and U.S. Pat. Pub. 2019/0233878. Biological particles (*e.g.*, cells, cell beads, cell nuclei, organelles, and the like) can be partitioned together with nucleic acid barcode molecules and the nucleic acid molecules of or derived from the biological particle (*e.g.,* mRNA, cDNA, gDNA, *etc*.,) can be barcoded as described elsewhere herein. In some embodiments, biological particles are co-partitioned with barcode carrying beads (*e.g.*, gel beads) and the nucleic acid molecules of or derived from the biological particle are barcoded as described elsewhere herein. In such cases, the lysis agents can be contacted with the biological particle suspension concurrently with, or immediately prior to, the introduction of the biological particles into the partitioning junction/droplet generation zone (*e.g.*, junction **1210**), such as through an additional channel or channels upstream of the channel junction. In accordance with other aspects, additionally or alternatively, biological particles can be partitioned along with other reagents, as will be described further below.

Beneficially, when lysis reagents and biological particles are co-partitioned, the lysis reagents can facilitate the release of the contents of the biological particles within the partition. The contents released in a partition can remain discrete from the contents of other partitions.

As will be appreciated, the channel segments described herein can be coupled to any of a variety of different fluid sources or receiving components, including reservoirs, tubing, manifolds, or fluidic components of other systems. As will be appreciated, the microfluidic channel structures can have other geometries and/or configurations. For example, a microfluidic channel structure can have more than two channel junctions.

For example, a microfluidic channel structure can have 2, 3, 4, 5 channel segments or more each carrying the same or different types of beads, reagents, and/or biological particles that meet at a channel junction. Fluid flow in each channel segment can be controlled to control the partitioning of the different elements into droplets. Fluid can be directed flow along one or more channels or reservoirs via one or more fluid flow units. A fluid flow unit can include compressors (*e.g.*, providing positive pressure), pumps (*e.g.*, providing negative pressure), actuators, and the like to control flow of the fluid. Fluid can also or otherwise be controlled via applied pressure differentials, centrifugal force, electrokinetic pumping, vacuum, capillary or gravity flow, or the like.

Examples of lysis agents include bioactive reagents, such as lysis enzymes that are used for lysis of different cell types, *e.g.*, gram positive or negative bacteria, plants, yeast, mammalian, etc., such as lysozymes, achromopeptidase, lysostaphin, labiase, kitalase, lyticase, and a variety of other lysis enzymes available from, *e.g.*, Sigma-Aldrich, Inc. (St Louis, MO), as well as other commercially available lysis enzymes. Other lysis agents can additionally or alternatively be co-partitioned with the biological particles to cause the release of the biological particle's contents into the partitions. For example, in some cases, surfactant-based lysis solutions can be used to lyse cells (*e.g.*, labelled engineered cells), although these can be less desirable for emulsion based systems where the surfactants can interfere with stable emulsions. In some cases, lysis solutions can include non-ionic surfactants such as, for example, Triton X-100 and Tween 20. In some cases, lysis solutions can include ionic surfactants such as, for example, sarcosyl and sodium dodecyl sulfate (SDS). Electroporation, thermal, acoustic or mechanical cellular disruption can also be used in certain cases, *e.g.*, non-emulsion based partitioning such as encapsulation of biological particles that can be in addition to or in place of droplet partitioning, where any pore size of the encapsulate is sufficiently small to retain nucleic acid fragments of a given size, following cellular disruption.

Alternatively or in addition to the lysis agents co-partitioned with the biological particles (*e.g.*, labelled engineered cells) described above, other reagents can also be co-partitioned with the biological particles, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated biological particles (*e.g.*, cell beads comprising labelled engineered cells), the biological particles can be exposed to an appropriate stimulus to release the biological particles or their contents from a co-partitioned cell bead. For example, in some cases, a chemical stimulus can be co-partitioned along with an encapsulated biological particle to allow for the degradation of the encapsulating material and release of the cell or its contents into the larger partition. In some cases, this stimulus can be the same as the stimulus described elsewhere herein for release of nucleic acid molecules (*e.g.*, oligonucleotides) from their respective bead. In alternative aspects, this can be a different and non-overlapping stimulus, in order to allow an encapsulated biological particle to be released into a partition at a different time from the release of nucleic acid molecules into the same partition.

Additional reagents can also be co-partitioned with the biological particles (*e.g.*, labelled engineered cells), such as endonucleases to fragment a biological particle's DNA, DNA polymerase enzymes and dNTPs used to amplify the biological particle's nucleic acid fragments and to attach the barcode molecular tags to the amplified fragments. Other enzymes can be co-partitioned, including without limitation, polymerase, transposase, ligase, proteinase K, DNAse, etc. Additional reagents can also include reverse transcriptase enzymes, including enzymes with terminal transferase activity, primers and oligonucleotides, and switch oligonucleotides (also referred to herein as "switch oligos" or "template switching oligonucleotides") which can be used for template switching. In some cases, template switching can be used to increase the length of a cDNA. In some cases, template switching can be used to append a predefined nucleic acid sequence to the cDNA. In an example of template switching, cDNA can be generated from reverse transcription of a template, *e.g.*, cellular mRNA, where a reverse transcriptase with terminal transferase activity can add additional nucleotides, *e.g.*, polyC, to the cDNA in a template independent manner. Switch oligos can include sequences complementary to the additional nucleotides, *e.g.*, polyG. The additional nucleotides (*e.g.*, polyC) on the cDNA can hybridize to the additional nucleotides (*e.g.*, polyG) on the switch oligo, whereby the switch oligo can be used by the reverse transcriptase as template to further extend the cDNA. Template switching oligonucleotides can include a hybridization region and a template region. The hybridization region can include any sequence capable of hybridizing to the target. In some cases, as previously described, the hybridization region includes a series of G bases to complement the overhanging C bases at the 3' end of a cDNA molecule. The series of G bases can include 1 G base, 2 G bases, 3 G bases, 4 G bases, 5 G bases or more than 5 G bases. The template sequence can include any sequence to be incorporated into the cDNA. In some cases, the template region includes at least 1 (*e.g.*, at least 2, 3, 4, 5 or more) tag sequences and/or functional sequences. Switch oligos can include deoxyribonucleic acids; ribonucleic acids; modified nucleic acids including 2-Aminopurine, 2,6-Diaminopurine (2-Amino-dA), inverted dT, 5-Methyl dC, 2'-deoxylnosine, Super T (5-hydroxybutynl-2'-deoxyuridine), Super G (8-aza-7-deazaguanosine), locked nucleic acids (LNAs), unlocked nucleic acids (UNAs, *e.g.*, UNA-A, UNA-U, UNA-C, UNA-G), Iso-dG, Iso-dC, 2' Fluoro bases (*e.g.*, Fluoro C, Fluoro U, Fluoro A, and Fluoro G), or any combination.

In some cases, the length of a switch oligo can be at least about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197 , 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides or longer.

In some cases, the length of a switch oligo can be at most about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197 , 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249

or 250 nucleotides.

Once the contents of the biological particles ((*e.g.*, cells such as B cells, cell beads, or nuclei) are released into their respective partitions, the macromolecular components (e.g., macromolecular constituents of biological particles, such as RNA, DNA, proteins, or secreted antibodies or antigen-binding fragments thereof) contained therein can be further processed within the partitions. In accordance with the methods described herein, the macromolecular component contents of individual biological particles (*e.g.*, cells such as B cells, cell beads, or nuclei) can be provided with unique identifiers such that, upon characterization of those macromolecular components they can be attributed as having been derived from the same biological particle or particles. The ability to attribute characteristics to individual biological particles or groups of biological particles is provided by the assignment of unique identifiers specifically to an individual biological particle or groups of biological particles. Unique identifiers, *e.g.*, in the form of nucleic acid barcodes can be assigned or associated with individual biological particles or populations of biological particles, in order to tag or label the biological particle's macromolecular components (and as a result, its characteristics) with the unique identifiers. These unique identifiers can then be used to attribute the biological particle's components and characteristics to an individual biological particle or group of biological particles.

In some aspects, this is performed by co-partitioning the individual biological particle (*e.g.*, cells such as B cells, cell beads, or nuclei) or groups of biological particles (*e.g.*, cells such as B cells, cell beads or nuclei) with the unique identifiers, such as described above (with reference to FIGS. 12 and 13). In some aspects, the unique identifiers are provided in the form of nucleic acid molecules (*e.g.*, oligonucleotides) that include nucleic acid barcode sequences that can be attached to or otherwise associated with the nucleic acid contents of individual biological particle, or to other components of the biological particle, and particularly to fragments of those nucleic acids. The nucleic acid molecules are partitioned such that as between nucleic acid molecules in a given partition, the nucleic acid barcode sequences contained therein are the same, but as between different partitions, the nucleic acid molecule can, and do have differing barcode sequences, or at least represent a large number of different barcode sequences across all of the partitions in a given analysis. In some aspects, only one nucleic acid barcode sequence can be associated with a given partition, although in some cases, two or more different barcode sequences can be present.

The nucleic acid barcode sequences can include from about 6 to about 20 or more nucleotides within the sequence of the nucleic acid molecules (*e.g.*, oligonucleotides). The nucleic acid barcode sequences can include from about 6 to about 20, 30, 40, 50, 60, 70, 80, 90, 100 or more nucleotides. In some cases, the length of a barcode sequence can be about 6, 7, 8, 9, 10, 11, 12, 13, 14, 15, 16, 17, 18, 19, 20 nucleotides or longer. In some cases, the length of a barcode sequence can be at least about 6, 7, 8, 9, 10, 11, 12, 13, 14, 15, 16, 17, 18, 19, 20 nucleotides or longer. In some cases, the length of a barcode sequence can be at most about 6, 7, 8, 9, 10, 11, 12, 13, 14, 15, 16, 17, 18, 19, 20 nucleotides or shorter. These nucleotides can be completely contiguous, i.e., in a single stretch of adjacent nucleotides, or they can be separated into two or more separate subsequences that are separated by 1 or more nucleotides. In some cases, separated barcode subsequences can be from about 4 to about 16 nucleotides in length. In some cases, the barcode subsequence can be about 4, 5, 6, 7, 8, 9, 10, 11, 12, 13, 14, 15, 16 nucleotides or longer. In some cases, the barcode subsequence can be at least about 4, 5, 6, 7, 8, 9, 10, 11, 12, 13, 14, 15, 16 nucleotides or longer. In some cases, the barcode subsequence can be at most about 4, 5, 6, 7, 8, 9, 10, 11, 12, 13, 14, 15, 16 nucleotides or shorter.

The co-partitioned nucleic acid molecules can also include other functional sequences useful in the processing of the nucleic acids from the co-partitioned biological particles (*e.g.,* labelled B cells). These sequences include, *e.g.*, targeted or random/universal amplification primer sequences for amplifying the genomic DNA from the individual biological particles within the partitions while attaching the associated barcode sequences, sequencing primers or primer recognition sites, hybridization or probing sequences, *e.g.*, for identification of presence of the sequences or for pulling down barcoded nucleic acids, or any of a number of other potential functional sequences. Other mechanisms of co-partitioning oligonucleotides can also be employed, including, *e.g.*, coalescence of two or more droplets, where one droplet contains oligonucleotides, or microdispensing of oligonucleotides into partitions, *e.g.*, droplets within microfluidic systems.

In an example, beads are provided that each include large numbers of the above described nucleic acid barcode molecules (*e.g.*, barcoded oligonucleotides) releasably attached to the beads, where all of the nucleic acid molecules attached to a particular bead will include the same nucleic acid barcode sequence, but where a large number of diverse barcode sequences are represented across the population of beads used. In some embodiments, hydrogel beads, *e.g.*, including polyacrylamide polymer matrices, are used as a solid support and delivery vehicle for the nucleic acid molecules into the partitions, as they are capable of carrying large numbers of nucleic acid molecules, and can be configured to release those nucleic acid molecules upon exposure to a particular stimulus, as described elsewhere herein. In some cases, the population of beads provides a diverse barcode sequence library that includes at least about 1,000 different barcode sequences, at least about 5,000 different barcode sequences, at least about 10,000 different barcode sequences, at least about 50,000 different barcode sequences, at least about 100,000 different barcode sequences, at least about 1,000,000 different barcode sequences, at least about 5,000,000 different barcode sequences, or at least about 10,000,000 different barcode sequences, or more. Additionally, each bead can be provided with large numbers of nucleic

acid (*e.g.*, oligonucleotide) molecules attached. In particular, the number of molecules of nucleic acid molecules including the barcode sequence on an individual bead can be at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules, or more. Nucleic acid molecules of a given bead can include identical (or common) barcode sequences, different barcode sequences, or a combination of both. Nucleic acid molecules of a given bead can include multiple sets of nucleic acid molecules. Nucleic acid molecules of a given set can include identical barcode sequences. The identical barcode sequences can be different from barcode sequences of nucleic acid molecules of another set.

Moreover, when the population of beads is partitioned, the resulting population of partitions can also include a diverse barcode library that includes at least about 1,000 different barcode sequences, at least about 5,000 different barcode sequences, at least about 10,000 different barcode sequences, at least at least about 50,000 different barcode sequences, at least about 100,000 different barcode sequences, at least about 1,000,000 different barcode sequences, at least about 5,000,000 different barcode sequences, or at least about 10,000,000 different barcode sequences. Additionally, each partition of the population can include at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules.

In some cases, it may be desirable to incorporate multiple different barcodes within a given partition, either attached to a single or multiple beads within the partition. For example, in some cases, a mixed, but known set of barcode sequences can provide greater assurance of identification in the subsequent processing, *e.g.*, by providing a stronger address or attribution of the barcodes to a given partition, as a duplicate or independent confirmation of the output from a given partition.

The nucleic acid molecules (*e.g.*, oligonucleotides) are releasable from the beads upon the application of a particular stimulus to the beads. In some cases, the stimulus can be a photo-stimulus, *e.g.*, through cleavage of a photo-labile linkage that releases the nucleic acid molecules. In other cases, a thermal stimulus can be used, where elevation of the temperature of the beads environment will result in cleavage of a linkage or other release of the nucleic acid molecules from the beads. In still other cases, a chemical stimulus can be used that cleaves a linkage of the nucleic acid molecules to the beads, or otherwise results in release of the nucleic acid molecules from the beads. In one case, such compositions include the polyacrylamide matrices described above for encapsulation of biological particles, and can be degraded for release of the attached nucleic acid molecules through exposure to a reducing agent, such as DTT.

*Methods for controlled partitioning*

In some aspects, provided are methods for controlled partitioning. Droplet size can be controlled by adjusting certain geometric features in channel architecture (*e.g.*, microfluidics channel architecture). For example, an expansion angle, width, and/or length of a channel can be adjusted to control droplet size.

**FIG. 14** shows an exemplary microfluidic channel structure **200** for generating discrete droplets containing a barcode carrying bead **214** along with an enzyme-decorated cell **216**. The channel structure **200** includes channel segments **201, 202**, **204**, **206** and **208** in fluid communication at a channel junction **210.** In operation, the channel segment **201** transports an aqueous fluid **212** that can include a plurality of beads **214** (*e.g.*, gel beads carrying barcode oligonucleotides) along the channel segment **201** into junction **210.** The plurality of beads **214** may be sourced from a suspension of beads. For example, the channel segment **201** can be connected to a reservoir comprising an aqueous suspension of beads **214.** The channel segment **202** transports the aqueous fluid **212** that includes a plurality of enzyme-decorated cells **216** along the channel segment **202** into junction **210.** The plurality of cells **216** may be sourced from a suspension. For example, the channel segment **202** may be connected to a reservoir comprising an aqueous suspension of a biological sample comprising the plurality of cells **216**. In some instances, the aqueous fluid **212** in either the first channel segment **201** or the second channel segment **202**, or in both segments, can include one or more reagents, as further described elsewhere herein. For example, in some embodiments of the present disclosure, the aqueous fluid in the first and/or second channel segments that delivers the enzyme-decorated cells can include linear polymers modified with a crosslink precursor moiety, and/or a co-substrate. The second fluid **218** that is immiscible with the aqueous fluid **212 (*e.g.* oil)** is delivered to the junction **210** from each of channel segments **204** and **206.** Upon meeting of the aqueous fluid **212** from each of channel segments **201** and **202** and the second fluid **218** (*e.g.*, a fluorinated oil) from each of channel segments **204** and **206** at the channel junction **210**, the aqueous fluid **212** is partitioned into discrete droplets **220** in the second

fluid **218** and flow away from the junction **210** along channel segment **208.** The channel segment **208** can then deliver the discrete droplets encapsulating the enzyme-decorated cell and a barcode bead to an outlet reservoir fluidly coupled to the channel segment **208**, where they can be collected.

As an alternative, the channel segments **201** and **202** may meet at another junction upstream of the junction **210**. At such junction, beads and enzyme-decorated cells may form a mixture that is directed along another channel to the junction **210** to yield droplets **220**. The mixture may provide the beads and enzyme-decorated cells in an alternating fashion, such that, for example, a droplet comprises a single bead and a single cell.

Using such a channel system as exemplified in **FIG. 14**, partitions **220** can be generated that encapsulate an individual enzyme-decorated cell, linear polymers, and one bead, wherein the bead can carry a barcode, and/or the bead can carry other reagents. It is also contemplated, that in some instances, a partition may be generated using the channel system of **FIG. 14**, wherein the partition includes more than one biological particle (e.g., cell, cell bead, or nucleus) or includes no biological particles. Similarly, in some embodiments, the partition may include more than one bead or no bead. A discrete droplet also may be completely unoccupied (*e.g.*, no bead or cell or nucleus or cell bead).

In some embodiments, it is desired that the enzyme-decorated biological particles (*e.g.*, cells or nuclei), linear polymers, and beads, generated discrete droplets flow along channels at substantially regular flow rates that generate a discrete droplet containing a single bead and a single biological particle (e.g., a cell or nucleus). Regular flow rates and devices that may be used to provide such regular flow rates are known in the art, and described in e.g., US Pat. Publ. No. 2015/0292988A1. In some embodiments, the flow rates are set to provide discrete droplets containing a single bead and a single enzyme-decorated biological particle (e.g., cell or nucleus) with a yield rate of greater than 5%, 10%, 20%, 30%, 40%, 50%, 60%, 70%, 80%, 90%, or 95%.

One of ordinary skill will recognize that numerous different microfluidic channel designs and methods described herein for generating a discrete droplet containing an enzyme-decorated biological particle (e.g., cell, cell bead, or nucleus), linear polymers, a co-substrate, and other crosslinking reagents, also can be used with the methods of the present disclosure to generate discrete droplets that further contain barcodes and/or assay reagents.

Although the exemplary embodiments of **FIG. 13** and **FIG. 14** have been described in terms of providing partitions, such as discrete droplets, that are predominantly singly occupied, it is also contemplated in certain embodiments that it is desirable to provide multiply occupied discrete droplets, *e.g.*, a single droplet that contains two, three, four or more enzyme-decorated biological particles (e.g., cells, cell beads, or nuclei). Accordingly, as noted elsewhere herein, the flow characteristics of the biological particle (e.g., cells, cell beads, or nuclei) can be controlled to provide for such multiply occupied droplets. In particular, the flow parameters of the liquids used in the channel structures may be controlled to provide a given droplet occupancy rate greater than about 50%, greater than about 75%, and in some cases greater than about 80%, 90%, 95%, or higher.

In some embodiments, a plurality of different reagents and/or beads can be introduced from different sources into different inlets leading to a common droplet generation junction (*e.g.*, junction **210**). In such cases, the flow and frequency of the different reagents and/or beads into the channel or junction may be controlled to provide for a certain ratio from each source, while ensuring a given ratio in a partition with a given number of biological particles, such as cells, cell beads or nuclei (e.g., one biological particle, such as a cell, cell bead or nucleus, and one bead per partition).

The discrete droplets described herein generally comprise small volumes, for example, less than about 10 microliters ($\mu$L), 5 $\mu$L, 1 $\mu$L, 900 picoliters (pL), 800 pL, 700 pL, 600 pL, 500 pL, 400pL, 300 pL, 200 pL, 100pL, 50 pL, 20 pL, 10 pL, 1 pL, 500 nanoliters (nL), 100 nL, 50 nL, or less. In some embodiments, the discrete droplets generated that encapsulate a biological particle (e.g., a cell, cell bead, or nucleus) have overall volumes that are less than about 1000 pL, 900 pL, 800 pL, 700 pL, 600 pL, 500 pL, 400pL, 300 pL, 200 pL, 100pL, 50 pL, 20 pL, 10 pL, 1 pL, or less. It will be appreciated that the sample fluid volume, *e.g.*, including co-partitioned biological particles (e.g., cells, cell beads, or nuclei), linear polymers, and/or beads, within the droplets may be less than about 90% of the above described volumes, less than about 80%, less than about 70%, less than about 60%, less than about 50%, less than about 40%, less than about 30%, less than about 20%, or less than about 10% of the above described volumes.

The methods of generating discrete droplets useful with the methods of the present disclosure, result in the generation of a population or plurality of discrete droplets containing a biological particle (e.g., a cell, a cell bead, or a nucleus), linear polymers, other reagents, and/or a barcode bead. Generally, the methods are easily controlled to provide for any suitable number of droplets. For example, at least about 1,000 discrete droplets, at least about 5,000 discrete droplets, at least about 10,000 discrete droplets, at least about 50,000 discrete droplets, at least about 100,000 discrete droplets, at least about 500,000 discrete droplets, at least about 1,000,000 discrete droplets, at least about 5,000,000 discrete droplets, at least about 10,000,000 discrete droplets, or more discrete droplets can be generated or otherwise provided. Moreover, the plurality of discrete droplets may comprise both unoccupied and occupied droplets.

As described elsewhere herein, in some embodiments of the methods of the present disclosure, the generated discrete droplets containing a biological particle (e.g., a cell, a cell bead, or a nucleus), linear polymers, and optionally, one or more different beads, also contain other reagents. In some embodiments, the other reagents encapsulated or contained in the droplet include a co-substrate and/or linker cleavage reagent within the droplet. In some embodiments,

the co-substrate and/or linker cleaving reagent can be contacted with the suspension of biological particles (e.g., cells, cell beads, or nuclei) concurrently with, or immediately prior to, the introduction into the droplet generation junction of the microfluidic system (*e.g.*, junction **210**). In some embodiments, the reagents are introduced through an additional channel or channels upstream of the channel junction.

**FIG. 15** shows an example of a microfluidic channel structure **300** for co-partitioning enzyme-decorated cells and other reagents, such as linear polymers, co-substrate, and/or linker cleaving agents. The channel structure **300** can include channel segments **301, 302**, **304**, **306** and **308.** Channel segments **301** and **302** communicate at a first channel junction **309.** Channel segments **302**, **304, 306**, and **308** communicate at a second channel junction **310.** In exemplary co-partitioning operation, the channel segment **301** may transport an aqueous fluid **312** that includes a plurality of enzyme-decorated cells **314** along the channel segment **301** into the second junction **310.** As an alternative or in addition to, channel segment **301** may transport a fluid carrying linear polymers modified with a crosslinking precursor moiety. For example, the channel segment **301** may be connected to a reservoir comprising an aqueous suspension of the enzyme-decorated cells **314**. Upstream of, and immediately prior to reaching, the second junction **310**, the channel segment **301** may meet the channel segment **302** at the first junction **309**. The channel segment **302** can transport a plurality of reagents **315** (*e.g.*, linear polymers) in the aqueous fluid **312** along the channel segment **302** into the first junction **309**. For example, the channel segment **302** may be connected to a reservoir comprising other reagents **315**. After the first junction **309**, the aqueous fluid **312** in the channel segment **301** can carry both the enzyme decorated cells **314** and the other reagents **315** towards the second junction **310**. In some instances, the aqueous fluid **312** in the channel segment **301** can include one or more reagents, which can be the same or different reagents as the reagents **315**. A second fluid **316** that is immiscible with the aqueous fluid **312** (*e.g.*, a fluorinated oil) can be delivered to the second junction **310** from each of channel segments **304** and **306**. Upon meeting of the aqueous fluid **312** from the channel segment **301** and the second fluid **316** from each of channel segments **304** and **306** at the second channel junction **310**, the aqueous fluid **312** is partitioned as discrete droplets **318** in the second fluid **316** and flow away from the second junction **310** along channel segment **308.** The channel segment **308** may deliver the discrete droplets **318** to an outlet reservoir fluidly coupled to the channel segment **308**, where they may be collected for further analysis.

Discrete droplets generated can include an individual enzyme-decorated cell **314** and/or one or more reagents **315**, depending on what reagents are included in channel segment **302**. The discrete droplet generated may also include a barcoding bead (not shown), such as can be added via other channel structures described elsewhere herein.

Generally, the channel segments described herein may be coupled to any of a variety of different fluid sources or receiving components, including reservoirs, tubing, manifolds, or fluidic components of other systems. As will be appreciated, the microfluidic channel structure **300** may have other geometries. For example, a microfluidic channel structure can have more than two channel junctions. For example, a microfluidic channel structure can have 2, 3, 4, 5 channel segments or more each carrying the same or different types of biological particles (e.g., cells, cell beads, or nuclei), linear polymers, reagents, and/or beads that meet at a channel junction. Fluid flow in each channel segment may be controlled to control the partitioning of the different elements into droplets. Fluid may be directed flow along one or more channels or reservoirs via one or more fluid flow units. A fluid flow unit can comprise compressors (*e.g.*, providing positive pressure), pumps (*e.g.*, providing negative pressure), actuators, and the like to control flow of the fluid. Fluid may also or otherwise be controlled via applied pressure differentials, centrifugal force, electrokinetic pumping, vacuum, capillary or gravity flow, or the like.

**FIG. 16** shows an example of a microfluidic channel structure for the controlled partitioning of enzyme-decorated cells and/or modified linear polymers into discrete droplets. A channel structure **400** can include a channel segment **402** communicating at a channel junction **406** (or intersection) with a reservoir **404.** The reservoir **404** can be a chamber. Any reference to "reservoir," as used herein, can also refer to a "chamber." In operation, an aqueous fluid **408** that includes suspended beads **412** may be transported along the channel segment **402** into the junction **406** to meet a second fluid **410** that is immiscible with the aqueous fluid **408** in the reservoir **404** to create droplets **416, 418** of the aqueous fluid **408** flowing into the reservoir **404**. At the junction **406** where the aqueous fluid **408** and the second fluid **410** meet, droplets can form based on factors such as the hydrodynamic forces at the junction **406**, flow rates of the two fluids **408, 410**, fluid properties, and certain geometric parameters (*e.g.,* w, $h_0$, $\alpha$, etc.) of the channel structure **400**. A plurality of droplets can be collected in the reservoir **404** by continuously injecting the aqueous fluid **408** from the channel segment **402** through the junction **406**.

A discrete droplet generated can include a bead (*e.g.*, as in occupied droplets **416**). Alternatively, a discrete droplet generated can include more than one bead. Alternatively, a discrete droplet generated cannot include any beads (*e.g.*, as in unoccupied droplet **418**). In some instances, a discrete droplet generated can contain one or more biological particles, as described elsewhere herein. In some instances, a discrete droplet generated can include one or more reagents, as described elsewhere herein.

In some instances, the aqueous fluid **408** can have a substantially uniform concentration or frequency of beads **412.** The beads **412** can be introduced into the channel segment 402 from a separate channel (not shown in **FIG. 16**). The frequency of beads **412** in the channel segment **402** can be controlled by controlling the frequency in which the

beads **412** are introduced into the channel segment **402** and/or the relative flow rates of the fluids in the channel segment **402** and the separate channel. In some instances, the beads can be introduced into the channel segment **402** from a plurality of different channels, and the frequency controlled accordingly.

In some instances, the aqueous fluid **408** in the channel segment **402** can include biological particles (*e.g.*, described with reference to **FIG. 16**). In some instances, the aqueous fluid **408** can have a substantially uniform concentration or frequency of biological particles. As with the beads, the biological particles (*e.g.*, labelled engineered cells, cell beads, or nuclei) can be introduced into the channel segment **402** from a separate channel. The frequency or concentration of the biological particles in the aqueous fluid **408** in the channel segment **402** can be controlled by controlling the frequency in which the biological particles are introduced into the channel segment **402** and/or the relative flow rates of the fluids in the channel segment **402** and the separate channel. In some instances, the biological particles can be introduced into the channel segment **402** from a plurality of different channels, and the frequency controlled accordingly. In some instances, a first separate channel can introduce beads and a second separate channel can introduce biological particles into the channel segment **402**. The first separate channel introducing the beads can be upstream or downstream of the second separate channel introducing the biological particles.

The second fluid **410** can include an oil, such as a fluorinated oil, that includes a fluorosurfactant for stabilizing the resulting droplets, for example, inhibiting subsequent coalescence of the resulting droplets.

In some instances, the second fluid **410** cannot be subjected to and/or directed to any flow in or out of the reservoir **404**. For example, the second fluid **410** can be substantially stationary in the reservoir 404. In some instances, the second fluid **410** can be subjected to flow within the reservoir **404**, but not in or out of the reservoir **404**, such as via application of pressure to the reservoir **404** and/or as affected by the incoming flow of the aqueous fluid 1308 at the junction **406**. Alternatively, the second fluid **410** can be subjected and/or directed to flow in or out of the reservoir **404**. For example, the reservoir **404** can be a channel directing the second fluid **410** from upstream to downstream, transporting the generated droplets.

The channel structure **400** at or near the junction **406** can have certain geometric features that at least partly determine the sizes of the droplets formed by the channel structure 400. The channel segment **402** can have a height, h0 and width, w, at or near the junction 406. By way of example, the channel segment **402** can include a rectangular cross-section that leads to a reservoir **404** having a wider cross-section (such as in width or diameter). Alternatively, the cross-section of the channel segment **402** can be other shapes, such as a circular shape, trapezoidal shape, polygonal shape, or any other shapes. The top and bottom walls of the reservoir 404 at or near the junction **406** can be inclined at an expansion angle, $\alpha$. The expansion angle, $\alpha$, allows the tongue (portion of the aqueous fluid 408 leaving channel segment **402** at junction **406** and entering the reservoir **404** before droplet formation) to increase in depth and facilitate decrease in curvature of the intermediately formed droplet. Droplet size can decrease with increasing expansion angle. The resulting droplet radius, Rd, can be predicted by the following equation for the aforementioned geometric parameters of $h_0$, w, and $\alpha$:

$$R_d \approx 0.44\left(1 + 2.2\sqrt{\tan\alpha}\,\frac{w}{h_0}\right)\frac{h_0}{\sqrt{\tan\alpha}}$$

By way of example, for a channel structure with w = 21 μm, h = 21 μm, and $\alpha$ = 3°, the predicted droplet size is 121 μm. In another example, for a channel structure with w = 25 μm, h = 25 μm, and $\alpha$ = 5°, the predicted droplet size is 123 μm. In another example, for a channel structure with w = 28 μm, h = 28 μm, and $\alpha$ = 7°, the predicted droplet size is 124 μm.

In some instances, the expansion angle, $\alpha$, can be between a range of from about 0.5° to about 4°, from about 0.1° to about 10°, or from about 0° to about 90°. For example, the expansion angle can be at least about 0.01°, 0.1°, 0.2°, 0.3°, 0.4°, 0.5°, 0.6°, 0.7°, 0.8°, 0.9°, 1°, 2°, 3°, 4°, 5°, 6°, 7°, 8°, 9°, 10°, 15°, 20°, 25°, 30°, 35°, 40°, 45°, 50°, 55°, 60°, 65°, 70°, 75°, 80°, 85°, or higher. In some instances, the expansion angle can be at most about 89°, 88°, 87°, 86°, 85°, 84°, 83°, 82°, 81°, 80°, 75°, 70°, 65°, 60°, 55°, 50°, 45°, 40°, 35°, 30°, 25°, 20°, 15°, 10°, 9°, 8°, 7°, 6°, 5°, 4°, 3°, 2°, 1°, 0.1°, 0.01°, or less. In some instances, the width, w, can be between a range of from about 100 micrometers (μm) to about 500 μm. In some instances, the width, w, can be between a range of from about 10 μm to about 200 μm. Alternatively, the width can be less than about 10 μm. Alternatively, the width can be greater than about 500 μm. In some instances, the flow rate of the aqueous fluid 1308 entering the junction 1306 can be between about 0.04 microliters (μL)/minute (min) and about 40 μL/min. In some instances, the flow rate of the aqueous fluid 1308 entering the junction 1306 can be between about 0.01 microliters (μL)/minute (min) and about 100 μL/min. Alternatively, the flow rate of the aqueous fluid 1308 entering the junction 1306 can be less than about 0.01 μL/min. Alternatively, the flow rate of the aqueous fluid 1308 entering the junction 1306 can be greater than about 40 μL/min, such as 45 μL/min, 50 μL/min, 55 μL/min, 60 μL/min, 65 μL/min, 70 μL/min, 75 μL/min, 80 μL/min, 85 μL/min, 90 μL/min, 95 μL/min, 100 μL/min, 110 μL/min , 120 μL/min , 130 μL/min , 140 μL/min , 150 μL/min, or greater. At lower flow rates, such as flow rates of about

less than or equal to 10 microliters/minute, the droplet radius cannot be dependent on the flow rate of the aqueous fluid 1308 entering the junction 1306.

In some instances, at least about 50% of the droplets generated can have uniform size. In some instances, at least about 55%, 60%, 65%, 70%, 75%, 80%, 85%, 90%, 95%, 96%, 97%, 98%, 99%, or greater of the droplets generated can have uniform size. Alternatively, less than about 50% of the droplets generated can have uniform size.

The throughput of droplet generation can be increased by increasing the points of generation, such as increasing the number of junctions (e.g., junction 406) between aqueous fluid 408 channel segments (e.g., channel segment 402) and the reservoir 404. Alternatively or in addition, the throughput of droplet generation can be increased by increasing the flow rate of the aqueous fluid 408 in the channel segment 402. The methods described herein can be used to greatly increase the efficiency of single cell (or single cell bead or single nucleus) applications and/or other applications receiving droplet-based input.

The throughput of droplet generation can also be increased by using other suitable methods of droplet generations known in the art such as, piezoelectric droplet generators, dispensers, or actuators (see e.g., PCT/US2020/062195).

Subsequent operations that can be performed can include generation of amplification products, purification (*e.g.*, via solid phase reversible immobilization (SPRI)), further processing (*e.g.*, shearing, ligation of functional sequences, and subsequent amplification (*e.g.*, via PCR)). These operations can occur in bulk (*e.g.*, outside the partition). In the case where a partition is a droplet in an emulsion, the emulsion can be broken and the contents of the droplet pooled for additional operations. Additional reagents that can be co-partitioned along with the barcode bearing bead can include oligonucleotides to block ribosomal RNA (rRNA) and nucleases to digest genomic DNA from cells. Alternatively, rRNA removal agents can be applied during additional processing operations. The configuration of the constructs generated by such a method can help minimize (or avoid) sequencing of the poly-T sequence during sequencing and/or sequence the 5' end of a polynucleotide sequence. The amplification products, for example, first amplification products and/or second amplification products, can be subject to sequencing for sequence analysis. In some cases, amplification can be performed using the Partial Hairpin Amplification for Sequencing (PHASE) method.

A variety of applications require the evaluation of the presence and quantification of different biological particle or organism types within a population of biological particles, including, for example, microbiome analysis and characterization, environmental testing, food safety testing, epidemiological analysis, *e.g.*, in tracing contamination or the like.

Partitions including a barcode bead (*e.g.*, a gel bead) associated with barcode molecules and a bead encapsulating cellular constituents (*e.g.*, a cell bead) such as cellular nucleic acids can be useful in constituent analysis as is described in U.S. Patent Publication No. 2018/0216162.

**FIG. 17** shows an example of a microfluidic channel structure for increased droplet generation throughput. A microfluidic channel structure **500** can comprise a plurality of channel segments **502** and a reservoir **504**. Each of the plurality of channel segments 502 may be in fluid communication with the reservoir **504**. The channel structure **500** can comprise a plurality of channel junctions **506** between the plurality of channel segments **502** and the reservoir **504**. Each channel junction can be a point of droplet generation. The channel segment **402** from the channel structure **400** in **FIG. 6** and any description to the components thereof may correspond to a given channel segment of the plurality of channel segments **502** in channel structure **500** and any description to the corresponding components thereof. The reservoir **404** from the channel structure **400** and any description to the components thereof may correspond to the reservoir **504** from the channel structure **500** and any description to the corresponding components thereof.

**FIG. 18** shows another example of a microfluidic channel structure for increased droplet generation throughput. A microfluidic channel structure **600** can comprise a plurality of channel segments **602** arranged generally circularly around the perimeter of a reservoir **604**. Each of the plurality of channel segments **602** may be in fluid communication with the reservoir **604**. The channel structure **600** can comprise a plurality of channel junctions **606** between the plurality of channel segments **602** and the reservoir **604.** Each channel junction can be a point of droplet generation. The channel segment **402** from the channel structure **400** in **FIG. 6** and any description to the components thereof may correspond to a given channel segment of the plurality of channel segments **602** in channel structure **600** and any description to the corresponding components thereof. The reservoir **404** from the channel structure **400** and any description to the components thereof may correspond to the reservoir **604** from the channel structure **600** and any description to the corresponding components thereof. Additional aspects of the microfluidic structures depicted in **FIGS. 16-18**, including systems and methods implementing the same, are provided in US Pat. Publ. No. 2019/0323088A1.

Subsequent operations that can be performed can include generation of amplification products, purification (*e.g.*, via solid phase reversible immobilization (SPRI)), further processing (*e.g.*, shearing, ligation of functional sequences, and subsequent amplification (*e.g.*, via PCR)). These operations can occur in bulk (*e.g.*, outside the partition). In the case where a partition is a droplet in an emulsion, the emulsion can be broken and the contents of the droplet pooled for additional operations. Additional reagents that can be co-partitioned along with the barcode bearing bead can include oligonucleotides to block ribosomal RNA (rRNA) and nucleases to digest genomic DNA from cells. Alternatively, rRNA removal agents can be applied during additional processing operations. The configuration of the constructs generated

by such a method can help minimize (or avoid) sequencing of the poly-T sequence during sequencing and/or sequence the 5' end of a polynucleotide sequence. The amplification products, for example, first amplification products and/or second amplification products, can be subject to sequencing for sequence analysis. In some cases, amplification can be performed using the Partial Hairpin Amplification for Sequencing (PHASE) method.

A variety of applications require the evaluation of the presence and quantification of different biological particle or organism types within a population of biological particles, including, for example, microbiome analysis and characterization, environmental testing, food safety testing, epidemiological analysis, *e.g.*, in tracing contamination or the like.

Partitions including a barcode bead (*e.g.*, a gel bead) associated with barcode molecules and a bead encapsulating cellular constituents (*e.g.*, a cell bead) such as cellular nucleic acids can be useful in constituent analysis as is described in U.S. Patent Publication No. 2018/0216162.

*Sample and biological particle processing*

A sample can be derived from any useful source including any subject, such as a human subject. A sample can include material (*e.g.*, one or more cells or nuclei) from one or more different sources, such as one or more different subjects. Multiple samples, such as multiple samples from a single subject (*e.g.*, multiple samples obtained in the same or different manners from the same or different bodily locations, and/or obtained at the same or different times (*e.g.*, seconds, minutes, hours, days, weeks, months, or years apparat)), or multiple samples from different subjects, can be obtained for analysis as described herein. For example, a first sample can be obtained from a subject at a first time and a second sample can be obtained from the subject at a second time later than the first time. The first time can be before a subject undergoes a treatment regimen or procedure (*e.g.*, to address a disease or condition), and the second time can be during or after the subject undergoes the treatment regimen or procedure. In another example, a first sample can be obtained from a first bodily location or system of a subject (*e.g.*, using a first collection technique) and a second sample can be obtained from a second bodily location or system of the subject (*e.g.*, using a second collection technique), which second bodily location or system can be different than the first bodily location or system. In another example, multiple samples can be obtained from a subject at a same time from the same or different bodily locations. Different samples, such as different samples collected from different bodily locations of a same subject, at different times, from multiple different subjects, and/or using different collection techniques, can undergo the same or different processing (*e.g.*, as described herein). For example, a first sample can undergo a first processing protocol and a second sample can undergo a second processing protocol.

A sample can be a biological sample, such as a cell sample (*e.g.*, as described herein). A sample can include one or more biological particles, such as one or more cells and/or cellular constituents, such as one or more cell nuclei. For example, a sample can include a plurality of cells and/or cellular constituents. Components (*e.g.*, cells or cellular constituents, such as cell nuclei) of a sample can be of a single type or a plurality of different types. For example, cells of a sample can include one or more different types of blood cells.

A biological sample can include a plurality of cells having different dimensions and features. In some cases, processing of the biological sample, such as cell separation and sorting (*e.g.*, as described herein), can affect the distribution of dimensions and cellular features included in the sample by depleting cells having certain features and dimensions and/or isolating cells having certain features and dimensions.

A sample may undergo one or more processes in preparation for analysis (*e.g.*, as described herein), including, but not limited to, filtration, selective precipitation, purification, centrifugation, permeabilization, isolation, agitation, heating, and/or other processes. For example, a sample may be filtered to remove a contaminant or other materials. In an example, a filtration process can include the use of microfluidics (*e.g.*, to separate biological particles of different sizes, types, charges, or other features).

In an example, a sample including one or more cells can be processed to separate the one or more cells from other materials in the sample (*e.g.*, using centrifugation and/or another process). In some cases, cells and/or cellular constituents of a sample can be processed to separate and/or sort groups of cells and/or cellular constituents, such as to separate and/or sort cells and/or cellular constituents of different types. Examples of cell separation include, but are not limited to, separation of white blood cells or immune cells from other blood cells and components, separation of circulating tumor cells from blood, and separation of bacteria from bodily cells and/or environmental materials. A separation process can include a positive selection process (*e.g.*, targeting of a cell type of interest for retention for subsequent downstream analysis, such as by use of a monoclonal antibody that targets a surface marker of the cell type of interest), a negative selection process (*e.g.*, removal of one or more cell types and retention of one or more other cell types of interest), and/or a depletion process (*e.g.*, removal of a single cell type from a sample, such as removal of red blood cells from peripheral blood mononuclear cells).

Separation of one or more different types of cells can include, for example, centrifugation, filtration, microfluidic-based sorting, flow cytometry, fluorescence-activated cell sorting (FACS), magnetic-activated cell sorting (MACS), buoyancy-activated cell sorting (BACS), or any other useful method. For example, a flow cytometry method can be used to

detect cells and/or cellular constituents based on a parameter such as a size, morphology, or protein expression. Flow cytometry-based cell sorting can include injecting a sample into a sheath fluid that conveys the cells and/or cellular constituents of the sample into a measurement region one at a time. In the measurement region, a light source such as a laser can interrogate the cells and/or cellular constituents and scattered light and/or fluorescence can be detected and converted into digital signals. A nozzle system (*e.g.*, a vibrating nozzle system) can be used to generate droplets (*e.g.*, aqueous droplets) including individual cells and/or cellular constituents. Droplets including cells and/or cellular constituents of interest (*e.g.*, as determined via optical detection) can be labeled with an electric charge (*e.g.*, using an electrical charging ring), which charge can be used to separate such droplets from droplets including other cells and/or cellular constituents. For example, FACS can include labeling cells and/or cellular constituents with fluorescent markers (*e.g.*, using internal and/or external biomarkers). Cells and/or cellular constituents can then be measured and identified one by one and sorted based on the emitted fluorescence of the marker or absence thereof. MACS can use micro- or nano-scale magnetic particles to bind to cells and/or cellular constituents (*e.g.*, via an antibody interaction with cell surface markers) to facilitate magnetic isolation of cells and/or cellular constituents of interest from other components of a sample (*e.g.*, using a column-based analysis). BACS can use microbubbles (*e.g.*, glass microbubbles) labeled with antibodies to target cells of interest. Cells and/or cellular components coupled to microbubbles can float to a surface of a solution, thereby separating target cells and/or cellular components from other components of a sample. Cell separation techniques can be used to enrich for populations of cells of interest (*e.g.*, prior to partitioning, as described herein). For example, a sample including a plurality of cells including a plurality of cells of a given type can be subjected to a positive separation process. The plurality of cells of the given type can be labeled with a fluorescent marker (*e.g.*, based on an expressed cell surface marker or another marker) and subjected to a FACS process to separate these cells from other cells of the plurality of cells. The selected cells can then be subjected to subsequent partition-based analysis (*e.g.*, as described herein) or other downstream analysis. The fluorescent marker can be removed prior to such analysis or can be retained. The fluorescent marker can include an identifying feature, such as a nucleic acid barcode sequence and/or unique molecular identifier.

In another example, a first sample including a first plurality of cells including a first plurality of cells of a given type (*e.g.*, immune cells expressing a particular marker or combination of markers) and a second sample including a second plurality of cells including a second plurality of cells of the given type can be subjected to a positive separation process. The first and second samples can be collected from the same or different subjects, at the same or different types, from the same or different bodily locations or systems, using the same or different collection techniques. For example, the first sample can be from a first subject and the second sample can be from a second subject different than the first subject. The first plurality of cells of the first sample can be provided a first plurality of fluorescent markers configured to label the first plurality of cells of the given type. The second plurality of cells of the second sample can be provided a second plurality of fluorescent markers configured to label the second plurality of cells of the given type. The first plurality of fluorescent markers can include a first identifying feature, such as a first barcode, while the second plurality of fluorescent markers can include a second identifying feature, such as a second barcode, that is different than the first identifying feature. The first plurality of fluorescent markers and the second plurality of fluorescent markers can fluoresce at the same intensities and over the same range of wavelengths upon excitation with a same excitation source (*e.g.*, light source, such as a laser). The first and second samples can then be combined and subjected to a FACS process to separate cells of the given type from other cells based on the first plurality of fluorescent markers labeling the first plurality of cells of the given type and the second plurality of fluorescent markers labeling the second plurality of cells of the given type. Alternatively, the first and second samples can undergo separate FACS processes and the positively selected cells of the given type from the first sample and the positively selected cells of the given type from the second sample can then be combined for subsequent analysis. The encoded identifying features of the different fluorescent markers can be used to identify cells originating from the first sample and cells originating from the second sample. For example, the first and second identifying features can be configured to interact (*e.g.*, in partitions, as described herein) with nucleic acid barcode molecules (*e.g.*, as described herein) to generate barcoded nucleic acid products detectable using, *e.g.*, nucleic acid sequencing.

**FIG. 25** schematically shows an example workflow for processing nucleic acid molecules within a sample. A substrate **1800** including a plurality of microwells **1802** can be provided. A sample **1806** which can include a cell, cell bead, cellular components or analytes (*e.g.*, proteins and/or nucleic acid molecules) can be co-partitioned, in a plurality of microwells **1802,** with a plurality of beads **1804** including nucleic acid barcode molecules. During a partitioning process, the sample **1806** can be processed within the partition. For instance, in the case of live cells, the cell can be subjected to conditions sufficient to lyse the cells and release the analytes contained therein. In process **1820**, the bead **1804** can be further processed. By way of example, processes **1820a** and **1820b** schematically illustrate different workflows, depending on the properties of the bead **1804.**

In **1820a**, the bead includes nucleic acid barcode molecules that are attached thereto, and sample nucleic acid molecules (*e.g.*, RNA, DNA) can attach, *e.g.*, via hybridization of ligation, to the nucleic acid barcode molecules. Such attachment can occur on the bead. In process **1830**, the beads **1804** from multiple wells **1802** can be collected and

pooled. Further processing can be performed in process **1840**. For example, one or more nucleic acid reactions can be performed, such as reverse transcription, nucleic acid extension, amplification, ligation, transposition, *etc.* In some instances, adapter sequences are ligated to the nucleic acid molecules, or derivatives thereof, as described elsewhere herein. For instance, sequencing primer sequences can be appended to each end of the nucleic acid molecule. In process **1850**, further characterization, such as sequencing can be performed to generate sequencing reads. The sequencing reads can yield information on individual cells or populations of cells, which can be represented visually or graphically, *e.g.*, in a plot.

In **1820b**, the bead includes nucleic acid barcode molecules that are releasably attached thereto, as described below. The bead can degrade or otherwise release the nucleic acid barcode molecules into the well **1802**; the nucleic acid barcode molecules can then be used to barcode nucleic acid molecules within the well **1802**. Further processing can be performed either inside the partition or outside the partition. For example, one or more nucleic acid reactions can be performed, such as reverse transcription, nucleic acid extension, amplification, ligation, transposition, *etc.* In some instances, adapter sequences are ligated to the nucleic acid molecules, or derivatives thereof, as described elsewhere herein. For instance, sequencing primer sequences can be appended to each end of the nucleic acid molecule. In process **1850**, further characterization, such as sequencing can be performed to generate sequencing reads. The sequencing reads can yield information on individual cells or populations of cells, which can be represented visually or graphically, *e.g.*, in a plot.

*Multiplexing methods*

In some embodiments of the disclosure, steps (a) and (b) of the methods described herein are performed in multiplex format. For example, in some embodiments, step (a) of the methods disclosed herein can include individually partitioning additional single biological particles, such as nuclei, cell beads, or cells (*e.g.*, B cells) of the plurality of biological particles (e.g., nuclei, cell beads, or cells) in additional partitions of the plurality of partitions, and step (b) can further include determining all or a part of the nucleic acid sequences encoding antibodies or antigen-binding fragments thereof produced by the additional biological particles, such as cells (*e.g.*, B cells), cell beads or nuclei.

Accordingly, in some embodiments, the present disclosure provides for multiplexing, and otherwise increasing throughput of samples for analysis. For example, a single or integrated process workflow may permit the processing, identification, and/or analysis of more or multiple analytes, more or multiple types of analytes, and/or more or multiple types of analyte characterizations.

For example, in the methods described herein, one or more labelling agents capable of binding to or otherwise coupling to one or more biological particles (e.g., cells or nuclei) or features of biological particles (e.g., cells or nuclei) can be used to characterize biological particles (e.g., cells/nuclei and/or cell/nucleus features. In some instances, cell features include cell surface features. Cell surface features can include, but are not limited to, a receptor, an antigen or antigen fragment (*e.g.*, an antigen or antigen fragment that binds to an antigen-binding molecule located on a cell surface), a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, a B-cell receptor, a chimeric antigen receptor, a gap junction, an adherens junction, or any combination thereof.

In some cases, the labelling agent (*e.g.*, an antigen, an antigen fragment, an antibody, an antibody fragment) is presented as a monomer. In some cases, the labelling agent is presented as a multimer. In some cases, a labelling agent (*e.g.*, an antigen, an antigen fragment, an antibody, an antibody fragment) is presented as a dimer. In some cases, a labelling agent (*e.g.*, an antigen, an antigen fragment, an antibody, an antibody fragment) is presented as a trimer. In some cases, a labelling agent (*e.g.*, an antigen, an antigen fragment, an antibody, an antibody fragment) is presented as a tetramer. In some cases, a labelling agent (*e.g.*, an antigen, an antigen fragment, an antibody, an antibody fragment) is presented as a pentamer. In some cases, a labelling agent (*e.g.*, an antigen, an antigen fragment, an antibody, an antibody fragment) is presented as a hexamer. In some cases, a labelling agent (*e.g.*, an antigen, an antigen fragment, an antibody, an antibody fragment) is presented as a heptamer. In some cases, a labelling agent (*e.g.*, an antigen, an antigen fragment, an antibody, an antibody fragment) is presented as an octamer. In some cases, a labelling agent (*e.g.*, an antigen, an antigen fragment, an antibody, an antibody fragment) is presented as a nonamer. In some cases, a labelling agent (*e.g.*, an antigen, an antigen fragment, an antibody, an antibody fragment) is presented as a decamer. In some cases, a labelling agent (*e.g.*, an antigen, an antigen fragment, an antibody, an antibody fragment) is presented as a 10+-mer.

In some cases, the labelling agent can include a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (*e.g.*, the label can be conjugated to a molecule that can bind to the labelling agent or reporter oligonucleotide). In some cases, a label is conjugated to an oligonucleotide that is complementary to a sequence of the

reporter oligonucleotide, and the oligonucleotide can be allowed to hybridize to the reporter oligonucleotide.

**FIG. 26** describes exemplary labelling agents (**1910**, **1920**, **1930**) including reporter oligonucleotides (**1940**) attached thereto. Labelling agent **1910** (*e.g.*, any of the labelling agents described herein) is attached (either directly, *e.g.*, covalently attached, or indirectly) to reporter oligonucleotide **1940**. Reporter oligonucleotide **1940** can include barcode sequence **1942** that identifies labelling agent **1910**. Reporter oligonucleotide **1940** can also include one or more functional sequences **1943** that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, or a sequencing primer or primer binding sequence (such as an R1, R2, or partial R1 or R2 sequence).

Referring to **FIG. 26**, in some instances, reporter oligonucleotide **1940** conjugated to a labelling agent (*e.g.*, **1910**, **1920**, **1930)** includes a functional sequence **1941**, a reporter barcode sequence **1942** that identifies the labelling agent (*e.g.*, **1910**, **1920**, **1930**), and reporter capture handle **1943.** Reporter capture handle sequence **1943** can be configured to hybridize to a complementary sequence, such as a complementary sequence present on a nucleic acid barcode molecule **1990** (not shown), such as those described elsewhere herein. In some instances, nucleic acid barcode molecule **1990** is attached to a support (*e.g.*, a bead, such as a gel bead), such as those described elsewhere herein. For example, nucleic acid barcode molecule **1990** can be attached to the support via a releasable linkage (*e.g.*, including a labile bond), such as those described elsewhere herein. In some instances, reporter oligonucleotide **1940** includes one or more additional functional sequences, such as those described above.

In some instances, the labelling agent **1910** is a protein or polypeptide (*e.g.*, an antigen or prospective antigen, or a fragment of an antigen or prospective antigen) including reporter oligonucleotide **1940.** Reporter oligonucleotide **1940** includes reporter barcode sequence **1942** that identifies polypeptide **1910** and can be used to infer the presence of an analyte, *e.g.*, a binding partner of polypeptide **1910** (i.e., a molecule or compound to which polypeptide **1910** can bind). In some instances, the labelling agent **1910** is a lipophilic moiety (*e.g.*, cholesterol) including reporter oligonucleotide **1940,** where the lipophilic moiety is selected such that labelling agent **710** integrates into a membrane of a cell or nucleus. Reporter oligonucleotide **740** includes reporter barcode sequence **742** that identifies lipophilic moiety **1910** which in some instances is used to tag biological particles, such cells or nuclei (*e.g.,* groups of cells or nuclei, cell samples, *etc.*) and can be used for multiplex analyses as described elsewhere herein. In some instances, the labelling agent is an antibody **1920** (or an epitope binding fragment thereof) including reporter oligonucleotide **1940**. Reporter oligonucleotide **1940** includes reporter barcode sequence **1942** that identifies antibody **1920** and can be used to infer the presence of, *e.g.*, a target of antibody **1920** (i.e., a molecule or compound to which antibody **1920** binds). In other embodiments, labelling agent **1930** includes an MHC molecule **1931** including peptide **1932** and reporter oligonucleotide **1940** that identifies peptide **1932**. In some instances, the MHC molecule is coupled to a support **1933.** In some instances, support **1933** can be a polypeptide, such as streptavidin, or a polysaccharide, such as dextran. In some instances, reporter oligonucleotide **1940** can be directly or indirectly coupled to MHC labelling agent **1930** in any suitable manner. For example, reporter oligonucleotide **1940** can be coupled to MHC molecule **1931**, support **1933**, or peptide **1932**. In some embodiments, labelling agent **1930** includes a plurality of MHC molecules, (*e.g.* is an MHC multimer, which can be coupled to a support (*e.g.*, **1933**)). There are many possible configurations of Class I and/or Class II MHC multimers that can be utilized with the methods disclosed herein, *e.g.*, MHC tetramers, MHC pentamers (MHC assembled via a coiled-coil domain, *e.g.*, Pro5® MHC Class I Pentamers, (Prolmmune, Ltd.), MHC octamers, MHC dodecamers, MHC decorated dextran molecules (*e.g.*, MHC Dextramer® (Immudex)), *etc.* For a description of exemplary labelling agents, including antibody and MHC-based labelling agents, reporter oligonucleotides, and methods of use, see, *e.g.*, U.S. Pat. 10,550,429 and U.S. Pat. Pub. 20190367969.

Exemplary barcode molecules attached to a support (*e.g.*, a bead) is shown in **FIG. 27**. In some embodiments, analysis of multiple analytes (*e.g.*, RNA and one or more analytes using labelling agents described herein) can include nucleic acid barcode molecules as generally depicted in **FIG. 27.** In some embodiments, nucleic acid barcode molecules **2010** and **2020** are attached to support **2030** via a releasable linkage **2040** (*e.g.*, including a labile bond) as described elsewhere herein. Nucleic acid barcode molecule **2010** can include functional sequence **2011**, barcode sequence **2012** and capture sequence **2013.** Nucleic acid barcode molecule **2020** can include adapter sequence **2021**, barcode sequence **2012**, and capture sequence **2023**, wherein capture sequence **2023** includes a different sequence than capture sequence **2013.** In some instances, adapter **2011** and adapter **2021** include the same sequence. In some instances, adapter **2011** and adapter **2021** include different sequences. Although support **2030** is shown including nucleic acid barcode molecules **2010** and **2020**, any suitable number of barcode molecules including common barcode sequence **2012** are contemplated herein. For example, in some embodiments, support **2030** further includes nucleic acid barcode molecule **2050.** Nucleic acid barcode molecule **2050** can include adapter sequence **2051**, barcode sequence **2012** and capture sequence **2053**, wherein capture sequence **2053** includes a different sequence than capture sequence **2013** and **2023**. In some instances, nucleic acid barcode molecules (*e.g.*, **2010**, **2020**, **2050)** include one or more additional functional sequences, such as a UMI or other sequences described herein. The nucleic acid barcode molecules **2010**, **2020** or **2050** can interact with analytes as described elsewhere herein, for example, as depicted in **FIGS. 28A-28C**.

Referring to **FIG. 28A,** in an instance where biological particles (e.g., cells or nuclei) are labelled with labeling agents, capture sequence **2123** can be complementary to an adapter sequence of a reporter oligonucleotide. Biological particles (e.g., cells or nuclei) can be contacted with one or more reporter oligonucleotide **2120** conjugated labelling agents **2110** (*e.g.*, polypeptide such as an antigen or fragment of an antigen, antibody, or others described elsewhere herein). In some cases, the biological particles (e.g., cells or nuclei) can be further processed prior to barcoding. For example, such processing steps can include one or more washing and/or cell sorting steps. In some instances, a cell that is bound to labelling agent **2110** which is conjugated to oligonucleotide **2120** and support **2130** (*e.g.*, a bead, such as a gel bead) including nucleic acid barcode molecule **2190** is partitioned into a partition amongst a plurality of partitions (*e.g.*, a droplet of a droplet emulsion or a well of a microwell array). In some instances, the partition includes at most a single cell bound to labelling agent **2110.** In some instances, reporter oligonucleotide **2120** conjugated to labelling agent **2110** (*e.g.*, polypeptide such as an antigen or fragment of an antigen, an antibody, pMHC molecule such as an MHC multimer, *etc.)* includes a first functional sequence **2111** (*e.g.*, a primer sequence), a barcode sequence **2112** that identifies the labelling agent **2110** (*e.g.*, the polypeptide such as an antigen or fragment of an antigen, antibody, or peptide of a pMHC molecule or complex), and a capture handle sequence **2113.** Capture handle sequence **2113** can be configured to hybridize to a complementary sequence, such as capture sequence **2123** present on a nucleic acid barcode molecule **2190** (*e.g.*, partition-specific barcode molecule). In some instances, oligonucleotide **2110** includes one or more additional functional sequences, such as those described elsewhere herein.

Barcoded nucleic acid molecules can be generated (*e.g.*, via a nucleic acid reaction, such as nucleic acid extension, reverse transcription, or ligation) from the constructs described in **FIGS. 28A-28C**. For example, capture handle sequence **2113** can then be hybridized to complementary capture sequence **2123** to generate (*e.g.*, via a nucleic acid reaction, such as nucleic acid extension or ligation) a barcoded nucleic acid molecule including cell barcode (*e.g.*, common barcode or partition-specific barcode) sequence **2122** (or a reverse complement thereof) and reporter barcode sequence **2112** (or a reverse complement thereof). In some embodiments, the nucleic acid barcode molecule **2190** (*e.g.*, partition-specific barcode molecule) further includes a UMI. Barcoded nucleic acid molecules can then be optionally processed as described elsewhere herein, *e.g.*, to amplify the molecules and/or append sequencing platform specific sequences to the fragments. See, *e.g.*, U.S. Pat. Pub. 2018/0105808. Barcoded nucleic acid molecules, or derivatives generated therefrom, can then be sequenced on a suitable sequencing platform.

In some instances, analysis of multiple analytes (*e.g.*, nucleic acids and one or more analytes using labelling agents described herein) can be performed. For example, the workflow can include a workflow as generally depicted in any of **FIGS. 28A-28C**, or a combination of workflows for an individual analyte, as described elsewhere herein. For example, by using a combination of the workflows as generally depicted in **FIGS. 28A-28C,** multiple analytes can be analyzed.

In some instances, analysis of an analyte (*e.g.* a nucleic acid, a polypeptide, a carbohydrate, a lipid, etc.) includes a workflow as generally depicted in **FIG. 28A**. A nucleic acid barcode molecule **2190** can be co-partitioned with the one or more analytes. In some instances, nucleic acid barcode molecule **2190** is attached to a support **2130** (*e.g.*, a bead, such as a gel bead), such as those described elsewhere herein. For example, nucleic acid barcode molecule **2190** can be attached to support **2130** via a releasable linkage **2140** (*e.g.*, including a labile bond), such as those described elsewhere herein. Nucleic acid barcode molecule **2190** can include a functional sequence **2121** and optionally include other additional sequences, for example, a barcode sequence **2122** (*e.g.*, common barcode, partition-specific barcode, or other functional sequences described elsewhere herein), and/or a UMI sequence **2125.** The nucleic acid barcode molecule **2190** can include a capture sequence **2123** that can be complementary to another nucleic acid sequence, such that it can hybridize to a particular sequence.

For example, capture sequence **2123** can include a poly-T sequence and can be used to hybridize to mRNA. Referring to **FIG. 28C**, in some embodiments, nucleic acid barcode molecule **2190** includes capture sequence **2123** complementary to a sequence of RNA molecule **2160** from a cell. In some instances, capture sequence **2123** includes a sequence specific for an RNA molecule. Capture sequence **2123** can include a known or targeted sequence or a random sequence. In some instances, a nucleic acid extension reaction can be performed, thereby generating a barcoded nucleic acid product including capture sequence **2123**, the functional sequence **2121**, UMI sequence **2125**, any other functional sequence, and a sequence corresponding to the RNA molecule **2160.**

In another example, capture sequence **2123** can be complementary to an overhang sequence or an adapter sequence that has been appended to an analyte. For example, referring to **FIG. 28B**, in some embodiments, primer **2150** includes a sequence complementary to a sequence of nucleic acid molecule **2160** (such as an RNA encoding for a BCR sequence) from a biological particle. In some instances, primer **2150** includes one or more sequences **2151** that are not complementary to RNA molecule **2160**. Sequence **2151** can be a functional sequence as described elsewhere herein, for example, an adapter sequence, a sequencing primer sequence, or a sequence the facilitates coupling to a flow cell of a sequencer. In some instances, primer **2150** includes a poly-T sequence. In some instances, primer **2150** includes a sequence complementary to a target sequence in an RNA molecule. In some instances, primer **2150** includes a sequence complementary to a region of an immune molecule, such as the constant region of a BCR sequence. Primer

**2150** is hybridized to nucleic acid molecule **2160** and complementary molecule **2170** is generated. For example, complementary molecule **2170** can be cDNA generated in a reverse transcription reaction. In some instances, an additional sequence can be appended to complementary molecule **2170.** For example, the reverse transcriptase enzyme can be selected such that several non-templated bases **2180** (*e.g.*, a poly-C sequence) are appended to the cDNA. In another example, a terminal transferase can also be used to append the additional sequence. Nucleic acid barcode molecule **2190** includes a sequence **2124** complementary to the non-templated bases, and the reverse transcriptase performs a template switching reaction onto nucleic acid barcode molecule **2190** to generate a barcoded nucleic acid molecule including cell (*e.g.*, partition specific) barcode sequence **2122** (or a reverse complement thereof) and a sequence of complementary molecule **2170** (or a portion thereof). In some instances, capture sequence **2123** includes a sequence complementary to a region of an immune molecule, such as the constant region of a BCR sequence. Capture sequence **2123** is hybridized to nucleic acid molecule **2160** and a complementary molecule **2170** is generated. For example, complementary molecule **2170** can be generated in a reverse transcription reaction generating a barcoded nucleic acid molecule including cell barcode (*e.g.*, common barcode or partition-specific barcode) sequence **2122** (or a reverse complement thereof) and a sequence of complementary molecule **2170** (or a portion thereof). Additional methods and compositions suitable for barcoding cDNA generated from mRNA transcripts including those encoding V(D)J regions of an immune cell receptor and/or barcoding methods and composition including a template switch oligonucleotide are described in International Patent Application WO2018/075693, U.S. Patent Publication No. 2018/0105808, U.S. Patent Publication No. 2015/0376609, filed June 26, 2015, and U.S. Patent Publication No. 2019/0367969.

In some embodiments, the methods disclosed herein further include generating, in the partition, barcoded nucleic acid molecules. In some embodiments, the barcoded nucleic acid molecules comprise: (i) a first barcoded nucleic acid molecule comprising a sequence of the first or second reporter oligonucleotide or a reverse complement thereof and the partition-specific barcode sequence or reverse complement thereof.

In some embodiments, the barcoded nucleic acid molecules further comprise: (ii) a second barcoded nucleic acid molecule comprising a nucleic acid sequence encoding at least a portion of the ABM, or antigen-binding fragment thereof, expressed by the immune cell or reverse complement thereof and the partition-specific barcode sequence or reverse complement thereof.

In some embodiments, the first and/or second barcoded nucleic molecule comprises a UMI sequence. In some embodiments, the methods disclosed herein further determining sequences of the first and the second barcoded nucleic acid molecules. In some embodiments, the determined sequence comprises a nucleotide sequence. In some embodiments, the determined sequence comprises an amino acid sequence. Sequencing may be by performed by any of a variety of approaches, systems, or techniques, including next-generation sequencing (NGS) methods. Sequencing may be performed using nucleic acid amplification, polymerase chain reaction (PCR) (*e.g.*, digital PCR and droplet digital PCR (ddPCR), quantitative PCR, real time PCR, multiplex PCR, PCR-based singleplex methods, emulsion PCR), and/or isothermal amplification. Non-limiting examples of nucleic acid sequencing methods include Maxam-Gilbert sequencing and chain-termination methods, *de novo* sequencing methods including shotgun sequencing and bridge PCR, next-generation methods including Polony sequencing, 454 pyrosequencing, Illumina sequencing, SOLiD™ sequencing, Ion Torrent semiconductor sequencing, HeliScope single molecule sequencing, nanopore sequencing (see, e.g., Oxford Nanopore Technologies), and SMRT® sequencing.

Further, sequence analysis of the nucleic acid molecules can be direct or indirect. Thus, the sequence analysis can be performed on a barcoded nucleic acid molecule or it can be a molecule which is derived therefrom (*e.g.*, a complement thereof).

Other examples of methods for sequencing include, but are not limited to, DNA hybridization methods, restriction enzyme digestion methods, Sanger sequencing methods, ligation methods, and microarray methods. Additional examples of sequencing methods that can be used include targeted sequencing, single molecule real-time sequencing, exon sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, co-amplification at lower denaturation temperature-PCR (COLD-PCR), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, Solexa Genome Analyzer sequencing, MS-PET sequencing, whole transcriptome sequencing, and any combinations thereof.

In some embodiments, the method further comprises identifying and/or characterizing the ABM or antigen-binding fragment thereof based on the determined sequence of the second barcoded nucleic acid molecule.

In some embodiments, the ABM or antigen-binding fragment is identified based on the determined sequence of the second barcoded nucleic acid molecule. In some embodiments, the method disclosed herein further comprises assessing affinity of the ABM or antigen-binding fragment thereof, based on the generated first barcoded nucleic acid

molecule. In some embodiments, the disclosed herein further comprises contacting the immune cell with (i) a negative control antigen having or being suspected of having little or no binding affinity for the immune cell; and/or (ii) a positive control agent having or being suspected of having a binding affinity for the immune cell.

## Use of hydrogel-coated biological particles in partition-based assays

As disclosed elsewhere herein, the methods of the present disclose allow individual biological particles (e.g., cells or nuclei) to be selectively embedded or entrapped in a hydrogel matrix. The containment of the biological particle (e.g., cell or nucleus) in a hydrogel matrix allows for the more facile use storage, growth, and assay of the embedded biological particles (e.g., cells or nuclei). As described elsewhere herein, the hydrogel matrix can be prepared with cleavable crosslinks that allow the matrix to be degraded via a stimulus, thereby allowing the contained biological particle or its contents (e.g., cell/nucleus or the cell's/nucleus' contents) to be released and assayed in solution at a selected point in time.

Biological particles (e.g., cells or nuclei) embedded or entrapped in a hydrogel-coating can provide certain potential advantages of being more storable and more portable than biological particles merely partitioned in a liquid droplet. The porous nature of the hydrogel matrix can retain the biological particle or it macromolecular contents while allowing reagents and metabolites to diffuse in and out. Thus, a hydrogel-coated biological particle (e.g., cell or nucleus) composition of the present disclosure can be incubated with reagents for a select period of time before analysis, such as in order to characterize changes in the biological particle over time, either in the presence or absence of different stimuli. The hydrogel-coating containing the biological particle (e.g., cell or nucleus) allows for longer incubation with reagents that can be achieved with cell partitioned in emulsion droplets. The hydrogel-coated biological particle (e.g., cell or nucleus) may also be released from a partition, collected, and then co-partitioned into another partition with selected reagents and/or other biological molecules.

In at least one embodiment, a hydrogel-coated biological particle (e.g., cell or nucleus) composition of the present disclosure can be partitioned into a discrete droplet with a barcode and assay reagents. In the case of a degradable hydrogel matrix, a stimulus can be applied to the partition to release the biological particle (e.g., cell or nucleus) and allow further assay of the biological particle free of the hydrogel matrix. Typically, the free biological particle (e.g., cell or nucleus) in the partition would then be treated with lysing reagents to release its cellular contents. Alternatively, the hydrogel-coated biological particle (e.g., cell or nucleus) in the partition can by treated with lysis reagents capable of diffusing through the hydrogel matrix and lysing the biological particle (e.g., cell or nucleus) to release its cellular contents (or "cellular analytes") while not degrading the hydrogel matrix. In one embodiment, the biological particle (e.g., cell or nucleus) is initially coated with a hydrogel layer using the selective membrane anchor moiety-based approach described herein and then subsequently subjected to the cell bead-based methods described herein, wherein the biological particle having the initial hydrogel layer is provided as part of a cell bead. Accordingly, the range of cellular analytes that can be assayed using with the hydrogel-coated biological particle (e.g., cell or nucleus) compositions and methods of the present disclosure include, without limitation, intracellular and partially intracellular analytes, including a protein, a metabolite, a metabolic byproduct, an antibody or antibody fragment, an enzyme, an antigen, a carbohydrate, a lipid, a macromolecule, or a combination thereof (*e.g.*, proteoglycan) or other biomolecule. The cellular analyte may be a nucleic acid molecule, such as a deoxyribonucleic acid (DNA) molecule (*e.g.*, genomic DNA) or a ribonucleic acid (RNA) molecule (*e.g.*, messenger RNA (mRNA), ribosomal RNA (rRNA) or transfer RNA (tRNA)).

In at least one embodiment of the present disclosure, the cellular analyte detected from a hydrogel-coated biological particle (e.g., cell or nucleus) can be an RNA transcript, such as in a gene expression profiling assay. The RNA may be small RNA that are less than 200 nucleic acid bases in length, or large RNA that are greater than 200 nucleic acid bases in length. Small RNAs may include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA) and small rDNA-derived RNA (srRNA). The RNA may be double-stranded RNA or single-stranded RNA. The RNA may be circular RNA.

In at least one embodiment, the cellular analyte detected is associated with an intermediary entity, wherein the intermediary entity is analyzed to provide information about the cellular analyte and/or the intermediary entity itself. For instance, an intermediary entity (*e.g.*, an antibody) may be bound to a partially intracellular analyte (*e.g.*, a cell surface receptor), where the intermediary entity is processed to provide information about the intermediary entity, the partially intracellular analyte, or both. In at least one embodiment, the intermediary entity comprises an identifier of the biological sample, such as a barcode oligonucleotide, as further described herein.

A wide range of partition-based materials, methods, assays, and systems suitable for use in the embodiments of the present disclosure are known in the art, and described in US Pat. Nos. 9,694,361, 10,357,771, 10,273,541, and 10,011,872, and US Pat. Publ. Nos. 2018/0105808A1, 2019/0367982A1, and 2019/0338353A1. It is contemplated that any assay that can be carried out using a biological particle (e.g., a cell or nucleus) contained in a partition, such as a single biological particle (e.g., a cell or nucleus) encapsulated in a droplet with a bead carrying a barcode, can also be

carried out using a partition containing a hydrogel-coated cell composition and the methods of the present disclosure. Exemplary assays include single-cell transcription profiling, single-cell sequence analysis, immune profiling of individual T and B cells, single-cell chromatin accessibility analysis (*e.g.*, ATAC seq analysis). These exemplary assays can be carried out using commercially available systems for encapsulating biological samples, gel beads, barcodes, and/or other compounds/materials in droplets, such as The Chromium System (10x Genomics, Pleasanton, CA, USA).

In some embodiments of the assay methods, the discrete droplet further comprises one or more beads. In some embodiments, the bead(s) can contain the assay reagents and/or the un-fixing agent. In some embodiments, a barcode is carried by or contained in a bead. Compositions, methods and systems for sample preparation, amplification, and sequencing of biomolecules from single cells encapsulated with barcodes in droplets are provided in *e.g.*, US Pat. Publ. No. 2018/0216162A1.

Assay reagents can include those used to perform one or more additional chemical or biochemical operations on a biological sample encapsulated in a droplet. Accordingly, assay reagents useful in the assay method include any reagents useful in performing a reaction such as nucleic acid modification (*e.g.*, ligation, digestion, methylation, random mutagenesis, bisulfite conversion, uracil hydrolysis, nucleic acid repair, capping, or decapping), nucleic acid amplification (*e.g.*, isothermal amplification or PCR), nucleic acid insertion or cleavage (*e.g.*, via CRISPR/Cas9-mediated or transposon-mediated insertion or cleavage), and/or reverse transcription. Additionally, useful assay reagents can include those that allow the preparation of a target sequence or sequencing reads that are specific to the macromolecular constituents of interest at a higher rate than to non-target sequence specific reads.

As noted elsewhere herein, it is contemplated that a hydrogel-coated biological particle (e.g., a cell, cell bead, or nucleus) may be formed in partitions or partitioned with reagents for carrying out assays of the biological particle (e.g., nucleus, cell, and/or its cellular contents. The partition containing a hydrogel-coated biological particle (e.g., cell or nucleus) as described herein may further comprise one or more of the following: a reverse transcriptase (RT), a bead, and reagents for a nucleic acid extension reaction. In an additional embodiment, the compositions of the present invention have or are provided at a temperature other than ambient temperature or non-ambient temperature. In one embodiment, the temperature is below ambient temperature or above ambient temperature. As described elsewhere herein, partitioning approaches may generate a population or plurality of partitions. In such cases, any suitable number of partitions can be generated or otherwise provided. For example, at least about 1,000 partitions, at least about 5,000 partitions, at least about 10,000 partitions, at least about 50,000 partitions, at least about 100,000 partitions, at least about 500,000 partitions, at least about 1,000,000 partitions, at least about 5,000,000 partitions at least about 10,000,000 partitions, at least about 50,000,000 partitions, at least about 100,000,000 partitions, at least about 500,000,000 partitions, at least about 1,000,000,000 partitions, or more partitions can be generated or otherwise provided. Moreover, the plurality of partitions may comprise both unoccupied partitions (*e.g.*, empty partitions) and occupied partitions. For example, an occupied partition according the present invention comprises a hydrogel-coated cell.

In another aspect, the present invention concerns methods for the partitioning of a plurality of hydrogel-coated biological particles (e.g., cells, cell beads, or nuclei) into individual partitions. In some cases, about 10, about 20, about 30, about 40, about 50, about 60, about 70, about 80, about 90, about 100, about 200, about 300, about 400, about 500, about 600, about 700, about 800, about 900, about 1000, about 2000, about 3000, about 4000, about 5000, about 6000, about 7000, about 8000, about 9000, about 10,000, about 15,000, about 20,000, about 25,000, about 30,000, about 35,000, about 40,000, about 50,000, about 60,000, about 70,000, about 80,000, about 90,000 or about 100,000 biological particles (e.g., cells, cell beads, or nuclei) may be partitioned into individual partitions. In some instances, the method further comprises partitioning about 50 to about 20,000 biological particles (e.g., cells, cell beads, or nuclei) with each of a plurality of supports comprising the adaptor comprising the barcode sequence, wherein the barcode sequence is unique among each of the plurality of supports.

The partition can be subjected to other conditions sufficient to polymerize or gel the precursors. The conditions sufficient to polymerize or gel the precursors may comprise exposure to heating, cooling, electromagnetic radiation, and/or light. The conditions sufficient to polymerize or gel the precursors may comprise any conditions sufficient to polymerize or gel the precursors. Following polymerization or gelling, a polymer or gel may be formed around the biological particle. The polymer or gel may be diffusively permeable to chemical or biochemical reagents. The polymer or gel may be diffusively impermeable to macromolecular constituents of the biological particle. In this manner, the polymer or gel may act to allow the biological particle to be subjected to chemical or biochemical operations while spatially confining the macromolecular constituents to a region of the droplet defined by the polymer or gel. The polymer or gel may include one or more of disulfide cross-linked polyacrylamide, agarose, alginate, polyvinyl alcohol, polyethylene glycol (PEG)-diacrylate, PEG-acrylate, PEG-thiol, PEG-azide, PEG-alkyne, other acrylates, chitosan, hyaluronic acid, collagen, fibrin, gelatin, or elastin. The polymer or gel may comprise any other polymer or gel.

The polymer or gel may be functionalized (*e.g.* coupled to a capture agent) to bind to targeted analytes (*e.g.*, antibodies or antigen-binding fragment thereof), such as nucleic acids, proteins, carbohydrates, lipids or other analytes. The polymer or gel may be polymerized or gelled via a passive mechanism. The polymer or gel may be stable in alkaline conditions or at elevated temperature. The polymer or gel may have mechanical properties similar to the mechanical

properties of the bead. For instance, the polymer or gel may be of a similar size to the bead. The polymer or gel may have a mechanical strength (*e.g.* tensile strength) similar to that of the bead. The polymer or gel may be of a lower density than an oil. The polymer or gel may be of a density that is roughly similar to that of a buffer. The polymer or gel may have a tunable pore size. The pore size may be chosen to, for instance, retain denatured nucleic acids. The pore size may be chosen to maintain diffusive permeability to exogenous chemicals such as sodium hydroxide (NaOH) and/or endogenous chemicals such as inhibitors. The polymer or gel may be biocompatible. The polymer or gel may maintain or enhance cell viability. The polymer or gel may be biochemically compatible. The polymer or gel may be polymerized and/or depolymerized thermally, chemically, enzymatically, and/or optically.

The polymer may comprise poly(acrylamide-co-acrylic acid) crosslinked with disulfide linkages. The preparation of the polymer may comprise a two-step reaction. In the first activation step, poly(acrylamide-co-acrylic acid) may be exposed to an acylating agent to convert carboxylic acids to esters. For instance, the poly(acrylamide-co-acrylic acid) may be exposed to 4-(4,6-dimethoxy-1,3,5-triazin-2-yl)-4-methylmorpholinium chloride (DMTMM). The polyacrylamide-co-acrylic acid may be exposed to other salts of 4-(4,6-dimethoxy-1,3,5-triazin-2-yl)-4-methylmorpholinium. In the second cross-linking step, the ester formed in the first step may be exposed to a disulfide crosslinking agent. For instance, the ester may be exposed to cystamine (2,2'-dithiobis(ethylamine)). Following the two steps, the biological particle (e.g., cell or nucleus) may be surrounded by polyacrylamide strands linked together by disulfide bridges. In this manner, the biological particle (e.g., cell or nucleus) may be encased inside of or comprise a gel or matrix (*e.g.*, polymer matrix) to form coated biological particle (e.g., a coated cell or nucleus), or a "cell bead." A coated cell or cell bead can contain biological particles (*e.g.*, a cell or nucleus) or macromolecular constituents (*e.g.*, RNA, DNA, proteins, etc.) of biological particles. A cell bead may include a single biological particle (e.g., cell or nucleus) or multiple biological particles (e.g., cells or nuclei), or a derivative of the single biological particle(s) (e.g., cell/nucleus or multiple cells/nuclei). For example, after lysing and washing the cells, inhibitory components from cell lysates can be washed away and the macromolecular constituents can be bound as cell beads. Methods disclosed herein can be applicable to both cell beads (and/or droplets or other partitions) containing biological particles and cell beads (and/or droplets or other partitions) containing macromolecular constituents of biological particles.

Encapsulated biological particles (*e.g.*, labelled B cells, cell beads, or nuclei) can provide certain potential advantages of being more storable and more portable than droplet-based partitioned biological particles. Furthermore, in some cases, it may be desirable to allow biological particles (*e.g.* labelled B cells, cell beads, or nuclei) to incubate for a select period of time before analysis, such as in order to characterize changes in such biological particles over time, either in the presence or absence of different stimuli (*e.g.*, cytokines, antigens, etc.). In such cases, encapsulation may allow for longer incubation than partitioning in emulsion droplets, although in some cases, droplet partitioned biological particles may also be incubated for different periods of time, *e.g.*, at least 10 seconds, at least 30 seconds, at least 1 minute, at least 5 minutes, at least 10 minutes, at least 30 minutes, at least 1 hour, at least 2 hours, at least 5 hours, or at least 10 hours or more. The encapsulation of biological particles (*e.g.* labelled B cells, cell beads, or nuclei) may constitute the partitioning of the biological particles into which other reagents are co-partitioned. Alternatively, or in addition, encapsulated biological particles may be readily deposited into other partitions (*e.g.*, droplets) as described above.

## EXAMPLES

Various features and embodiments of the disclosure are illustrated in the following representative examples, which are intended to be illustrative, and not limiting. Those skilled in the art will readily appreciate that the specific examples are only illustrative of the invention as described more fully in the claims which follow thereafter. Every embodiment and feature described in the application should be understood to be interchangeable and combinable with every embodiment contained within.

**Example 1**: Enzyme-mediated preparation of hydrogel-coated cells

This example illustrates preparation of hydrogel-coated cells using a BAM-HRP cell decoration and phenol-modified DTT-degradable polyacrylamide hydrogel.

Materials and Methods

A. Preparation of BAM-HRP

Horseradish peroxidase (HRP) (Sigma-Aldrich, St. Louis, USA) was modified with a BAM linker as shown by the general reaction scheme in **FIG. 19.**

The BAM linker used was SUNBRIGHT® OE-080CS purchased from NOF EUROPE GmbH (Frankfurt, Germany). This BAM linker includes a BAM moiety linked to an N-hydroxy-succinimide moiety through a 8000 MW PEG polymer. The reaction coupling HRP and the BAM linker was carried out as follows: A solution of HRP in PBS, pH 7.4 (3 μL, 75 μM) was added to a solution of the BAM linker in PBS (1000 μL, 2.27 μM). The resulting mixture was shaken for 30 min at room temp and then purified with a 20 kD molecular weight cut-off (MWCO) Amicon Ultra centrifugal filters (EMD Millipore, Billerica, MA, USA), and concentrated to 0.1 mL in PBS by the same filters.

B. Jurkats cell preparation

Jurkats cells had previously been fixed by resuspending in 4% PFA at 4°C overnight, and the next day quenching fixation with 10% FBS and spinning down 300g for 5 min. Fresh cells can also be used according to the following cell decoration and gelation protocols. The cells were twice washed with PBS + 2% FBS. Cells were then stained with PE-cy7 CD45 (2 μL per 200 μL) on ice for 30 min in the dark. The staining was quenched with PBS + 10% FBS, and cells were spun down at 300g for 5 min. Cells were washed with 1X PBS, spun down at 300 g for 5 min and resuspended in 10 mL PBS. A total of 28M cells were counted and split into tubes of 1 million cells per tube.

C. Cell decoration with BAM-HRP

An aliquot of 1 million of the Jurkats cells prepared as described above were spun at 450 rpm for 5 min at 4C. The supernatant was removed and the cells were then incubated with 100 μL of the BAM-HRP solution (1μM in PBS) prepared as described above for 10 min. The solution volume was brought up to 2 mL with 0.04% BSA in PBS. The solution was spun at 450 rpm for 5 min, supernatant was removed. This BSA wash was repeated 3 times.

D. Preparation phenol-modified DTT-degradable polyacrylic acid linear polymer

**1. *Synthesis of phenol-modified DTT degradable linker***

**Scheme 3**

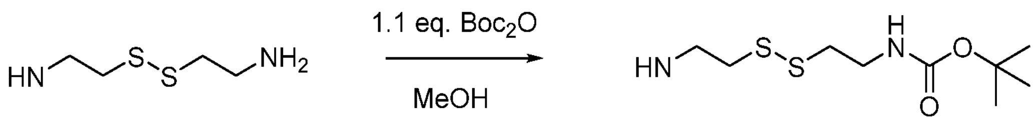


A mono-Boc-protected cystamine intermediate was prepared according to Scheme 3 (above) as follows: A solution of 10g cystamine in 150 mL MeOH was stirred at 0 C. 1.1 eq. $Boc_2O$ was added dropwise to the solution and subsequently stirred overnight. The solvent was removed *in vacuo* and the crude oil was dissolved in 1M $NaHPO_3$. The aqueous mixture was washed with 3 x 50 mL $Et_2O$ and the resulting aqueous was neutralized with 2M NaOH and extracted with 3 x 50 mL dichloromethane (DCM). The combined organic phase was dried over $MgSO_4$ and then filtered before removing solvent. The mono-Boc-cystamine product was confirmed by $^1H$ NMR integration of the 9 alkyl Boc protons with the cystamine multiplets.

**Scheme 4**

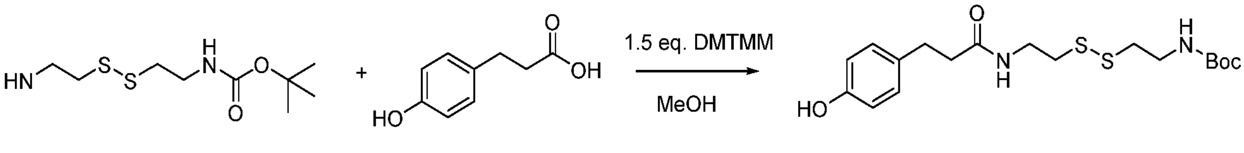


Phenol modification of the mono-Boc-cystamine intermediate was prepared according to Scheme 4 (above) as follows: mono-Boc-cystamine was dissolved in 100 mL MeOH and stirred at RT. To the solution was added 1.5 eq of 4-(4,6-dimethoxy-1,3,5-triazin-2-yl)-4-methyl-morpholinium chloride (DMTMM) before dropwise addition of phenol in 20 mL MeOH. The solution was stirred for 16 h before solvent was removed and the crude product was dissolved in 100 mL DCM. The organic phase was washed with 3 x 50 mL $H_2O$, 2M HCl and 2M NaOH before drying over $MgSO_4$ and evaporation to dryness. Product and purity was confirmed by 1H NMR integration of Boc peaks with aryl 4H.

**Scheme 5**

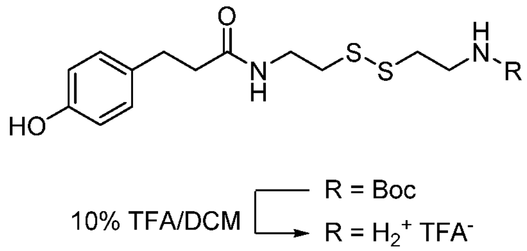


Deprotection to provide compound of Scheme 5 (above) was carried out as follows: the Boc protected phenol-modified compound of Scheme 4 was dissolved in 10% TFA in DCM and stirred for 16 h. The solvent was removed under vacuum and the final TFA salt was used without further purification. The target Boc-deprotected compound of Scheme 3 was confirmed by presence of phenol and total absence of alkyl Boc peak at δ = 0.9 - 1.1 ppm.

**2. *Synthesis of polyacrylamide 10% co-acrylic acid***

**Scheme 6**

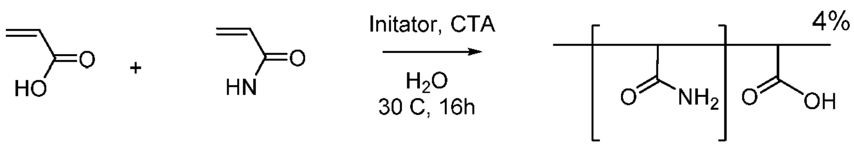


Preparation of linear polymers of polyacrylamide 10% co-acrylic acid according to Scheme 6 was carried out as follows: 49 mL of 40% aq. acrylamide solution was stirred with 2.17 mL acrylic acid and 10.8 g sodium formate in milliQ water. The solution was degassed with Ar for min and heated to 30°C. In a separate container was weighed out 200 mg thermal initiator VA-044 which was then dissolved in 1.96 mL degassed $H_2O$. 1 mL of the initiator solution was added by syringe to the stirring monomer solution and the reaction was allowed to proceed for 16 h under Ar. The final polymer solution was dialyzed using a 3.5K MWCO cassette with the resulting solution lyophilized to recover the target polyacrylic acid. Polymer was analyzed by GPC to determine polymer Mn and polydispersity.

**3. *Coupling of phenol-modified DTTdegradable linker to polyacrylic acid***

2g of the polyacrylic acid (prepared as above) was weighed out into a 100 mL roundbottom flask and dissolved in 30 mL milliQ $H_2O$. The TFA salt of the phenol-modified linker (prepared as described above) was dissolved in 10 mL milliQ water and added to the polymer solution which was then stirred at 300 rpm before addition of DMTMM (1 eq. with respect to acid). Once the solution is homogenous the pH is tested with pH strips and adjusted to 7 with 2M NaOH. Once neutralized, the solution is protected from light and stirred at room temperature for 16 h. The crude polymer is dialyzed using 3.5K MWCO snakeskin dialysis tubing and 0.2 μm filtered before lyophilization to provide the target phenol modified polyacrylamide linear polymer. Phenol loading of the linear polymer was determined by integration of phenol peaks δ = 7.1 - 7.5 ppm compared to alkyl polymer backbone at δ = 1.1 - 2.5.

D. Gelation of decorated cells

BAM-HRP decorated Jurkats cells (prepared as described above) were resuspended in a range of solutions with from 0.05% to 0.5% of the phenol-modified degradable polyacrylamide linear polymer and FITC albumin. A 1 mM solution of the HRP co-substrate $H_2O_2$ was added with stirring at 1500 rpm for 10 min at 37°C to initiate the HRP-catalyzed crosslinking of phenol-modified linear polymer to form a hydrogel coating around the decorated cell. The resulting suspension of hydrogel-coated cells was washed with PBS and evaluation via Flow sorting for the presence of fluorescence ($\lambda_{ex}$ = 494 nm/λem = 520 nm) using un-decorated Jurkats cells as control. Further, fluorescence ($\lambda_{ex}$ = 494 nm/λem = 520 nm) imaging of the hydrogel-coated cells generated at various concentrations of linear polymer was

performed using microscopy (Nikon Eclipse Ti).

Results

Analysis of fluorescence microscopy images verified formation of a hydrogel coating around the BAM-HRP decorated Jurkats cells in the presence of the solutions ranging from 0.05% to 0.5% of the phenol-modified degradable polyacrylamide linear polymer. An increase in fluorescence with increasing polymer concentration indicated the formation of a thicker hydrogel coating around the Jurkats cells at the higher 0.5% concentration of the phenol-modified degradable polyacrylamide linear polymer.

**Example 2:** Enzyme-mediated preparation of hydrogel-coated cells

This example illustrates the preparation of hydrogel-coated cells using cells decorated with HRP through a cleavable linker.

Materials and Methods

A. Preparation of cleavable BAM-S-S-HRP

Horseradish peroxidase (HRP) (Sigma-Aldrich, St. Louis, USA) is modified with cystamine dihydrochloride as shown in the top reaction scheme of **FIG. 1** to provide an HRP with a disulfide-linked free amine group (HRP-S-S-$NH_2$). The BAM linker, SUNBRIGHT® OE-080CS (NOF EUROPE GmbH; Frankfurt, Germany), which has a terminal N-hydroxysuccinimide moiety, then is reacted with the HRP-S-S-$NH_2$ as shown by the bottom reaction scheme of FIG. 1. The HRP-S-S-$NH_2$ to BAM linker coupling reaction is carried out described in Example 1 for the HRP to BAM linker coupling reaction. A solution of HRP-S-S-$NH_2$ in PBS, pH 7.4 (3 μL, 75 μM) is added to a solution of the SUNBRIGHT® OE-080CS BAM linker in PBS (1000 μL, 2.27 μM). The resulting mixture is shaken for 30 min at room temp and then purified with a 20 kD molecular weight cut-off (MWCO) Amicon Ultra centrifugal filters (EMD Millipore, Billerica, MA, USA), and concentrated to 0.1 mL in PBS by the same filters.

B. Decoration of fixed Jurkats cells with BAM-S-S-HRP

An aliquot of 1 million fixed Jurkats cells (prepared as described in Example 1) are centrifuged at 450 rpm for 5 min at 4C. Supernatant is removed and the cells are incubated with 100 μL of BAM-S-S-HRP solution (1μM in PBS) for 10 min. The solution volume was brought up to 2 mL with 0.04% BSA in PBS. The solution was spun at 450 rpm for 5 min, supernatant was removed. This BSA wash was repeated 3 times.

C. Gelation of BAM-S-S-HRP decorated cells

Resuspend the cells decorated with the cleavable HRP-S-S-BAM moiety in PBS along with a 2.5% solution of the degradable phenol-modified polyacrylamide linear polymer solution prepared as described in Example 1, and also including up to 2.5% of aqueous detergent, such as F-108.

A second aqueous "HRP release" mixture is prepared containing 2.5% degradable linear polymer solution and up to 2.5% aqueous detergent (e.g., F-108) along with 20 - 200 mM DTT. This solution is used to release the HRP from the decorated cells by DTT cleaving the disulfide bonds.

A range of alternative release agents may be used if the BAM-HRP cleavable linker moiety used is desired to be orthogonal to the degradable linkages that may be used in the phenol-modified linear polymer backbone forming the hydrogel. The aqueous solution optionally may include magnetic nano particles in a conc. of 0.05-1 % w/w.

The solution containing the suspended BAM-S-S-HRP decorated cells is mixed using an microfluidic device with an equal volume of the DTT containing HRP release solution during the generation of a fluoro-surfactant stabilized droplets. The droplets are generated to range in size from about 30 μm to about 150 μm. During the generation of the droplet, the presence of the DTT gradually initiates release the HRP from cells allowing some of the enzyme to diffuse throughout the full volume of the droplet.

After droplet generation, the emulsion pack is transferred to a strip tube where 50-200 μL of partitioning oil enriched with the HRP co-substrate $H_2O_2$ is added before being gently agitated for 10 to 60 min to initiate hydrogel formation.

After gelation is complete, the emulsion is broken and the hydrogel-coated cells of 50 μm - 200 μm are washed 3x in PBS.

### Example 3: Antibody Discovery

This Example describes an exemplary antibody discovery workflow in accordance with some embodiments of the methods disclosed herein.

In this antibody discovery workflow, a target antigen, which is a SARS-2 trimeric spike protein, is conjugated with horseradish peroxidase (HRP) used as an exemplary crosslinked-catalyzing moiety. However, other enzymatic crosslinked-catalyzing moieties, such as transglutaminase; tyrosinase; and laccase, can also be used. In some experiments, the target antigen is coupled with either or both a reporter oligonucleotide and a fluorescent label. In some experiments, the antigen is biotinylated.

Samples containing B cells or antibody receptor fragments of interest are first stained with the SARS-2 trimeric spike antigen. In experiments, where the samples contain plasma cells, a capture reagent (anti-Fc anti-B2M F(ab')2) is used to lock antibody onto the surface of the cell.

Subsequently, all stained cells are encapsulated within individual partitions (*e.g.*, droplets), followed by induction of gelation (via membrane anchor moiety-based methods) only in droplets containing cells that have the antigen of interest bound (by, for example, adding a suitable co-substrate, *e.g.*, hydrogen peroxide ($H_2O_2$)).

In some experiments, a streptavidin-bound HRP is introduced into individual droplets.

After streptavidin-HRP introduction, co-substrate hydrogen peroxide is introduced to initiate gelation formation to produce hydrogel-coated cells containing antigen-specific B cells of interest.

Membrane anchor moiety-based methods can be used to prepare hydrogel-coated cells as described above are then used in a variety of downstream assays including In-droplet OE-PCR and cloning / linked cloning in droplets as well as bulk BCR sequencing.

Optionally, single-cell sequencing by cell bead / gel bead (CBGB) workflows such as Barcode-enabled antigen mapping (BEAM), with optional FACS enrichment after introduction of one or more antigen or secondary labeling reagent to enable fluorescent detection of antibody properties of interest are also performed.

While the foregoing disclosure of the present disclosure has been described in some detail by way of example and illustration for purposes of clarity and understanding, this disclosure including the examples, descriptions, and embodiments described herein are for illustrative purposes, are intended to be exemplary, and should not be construed as limiting the present disclosure. It will be clear to one skilled in the art that various modifications or changes to the examples, descriptions, and embodiments described herein can be made within the appended claims.

## Claims

**1.** A method of partitioning cells comprising:

(a) generating a partition containing: (i) a cell comprising a plurality of crosslink-catalyzing moieties attached to its membrane through a linker comprising a membrane anchor moiety; and (ii) a linear polymer comprising a crosslink-precursor moiety;
(b) contacting the partition with a crosslink-forming initiator; whereby a hydrogel-coating of the cell is formed; and
(c) cleaving the linker;

whereby the crosslink-catalyzing moieties are released resulting in an increased thickness of hydrogel-coating of the cell.

**2.** The method of claim 1, wherein the partition is a discrete droplet or a well.

**3.** The method of claim 1, wherein the membrane anchor moiety is selected from a Biocompatible Anchor for cell Membrane (BAM) moiety; an antibody; an antibody to a cell membrane or surface protein; a cholesterol-oligonucleotide moiety; a 3'-cholesterol-TEG moiety; a cholesterol-decorated polymer; and a target antigen.

**4.** The method of claim 1, wherein the membrane anchor moiety is an antibody to a cell surface protein; optionally, wherein the cell surface protein is a cluster of differentiation ("CD") protein.

**5.** The method of claim 1, wherein the linker comprises a cleavable moiety selected from a disulfide spacer moiety; a carbamate spacer moiety; a photocleavable spacer; and Uracil-DNA glycosylase-cleavable (UDG-cleavable) spacer.

**6.** The method of claim 1, wherein cleaving the linker comprises contacting the partition with a reagent selected from dithiothreitol (DTT) and diethylenetriamine (DETA).

7. The method of claim 1, wherein the crosslink-catalyzing moieties are an enzyme selected from peroxidase; transglutaminase; tyrosinase; and laccase; optionally, wherein the enzyme is horseradish peroxidase (HRP).

8. The method of claim 1, wherein the crosslink-catalyzing moiety is a non-enzymatic compound selected from hematin and umbelliferone.

9. The method of claim 1, wherein the crosslink-catalyzing moieties are HRP, the crosslink-precursor moieties are phenol, and the crosslink-forming initiator is a compound comprising a peroxide moiety; optionally, wherein the compound comprising a peroxide moiety is $H_2O_2$.

10. The method of claim 1, wherein the crosslink-forming initiator is selected from $H_2O_2$, and $O_2$.

11. The method of claim 1, wherein contacting the partition with a crosslink-forming initiator comprises micelle-mediated transport of the initiator into the partition.

12. The method of claim 1, wherein the linear polymer is selected from an olefin copolymer, a polyolefin, an acrylic, a polyacrylamide, a poly(oxazoline), a vinyl polymer, a polyester, a polycarbonate, a polyamide, a polyimide, a formaldehyde resin, a polyurethane, an ether polymer, a cellulosic, a thermoplastic elastomer, and a thermoplastic polyurethane.

13. The method of claim 1, wherein the linear polymer further comprises a modifiable side-chain.

14. The method of claim 13, wherein the modifiable side-chain comprises an amine moiety.

15. The method of claim 13 or 14, further comprising contacting under suitable reaction conditions the hydrogel-coated cell with a detectable label moiety comprising a group capable of forming a covalent linkage to the modifiable side-chain of the hydrogel.

16. The method of claims 13-15, further comprising contacting under suitable reaction conditions the hydrogel-coated cell with a surface of a solid substrate, wherein the surface comprises a group capable of forming a covalent linkage to the modifiable side-chain of the hydrogel under the reaction condition, whereby the hydrogel-coated cell is covalently attached to the solid substrate.

## Patentansprüche

1. Verfahren zum Partitionieren von Zellen, umfassend:

   (a) Erzeugen einer Partition, die enthält: (i) eine Zelle, die eine Vielzahl von Vernetzung katalysierender Einheiten umfasst, die durch einen Linker, der eine Membranankereinheit umfasst, an ihrer Membran befestigt sind; und (ii) ein lineares Polymer, das eine Vernetzungsvorläufereinheit umfasst;
   (b) Inkontaktbringen der Partition mit einem Vernetzung bildenden Initiator; wodurch eine Hydrogelbeschichtung der Zelle gebildet wird; und
   (c) Spalten des Linkers;

   wobei die Vernetzung katalysierenden Einheiten freigesetzt werden, was zu einer erhöhten Dicke der Hydrogelbeschichtung der Zelle führt.

2. Verfahren nach Anspruch 1, wobei die Partition ein einzelnes Tröpfchen oder ein Näpfchen ist.

3. Verfahren nach Anspruch 1, wobei die Membranankereinheit ausgewählt wird aus einer biokompatiblen Anker-für-Zellmembran(Biocompatible Anchor for cell Membrane, BAM)-Einheit; einem Antikörper; einem Antikörper gegen eine Zellmembran oder ein Oberflächenprotein; einer Cholesterin-Oligonukleotid-Einheit; einer 3'-Cholesterin-TEG-Einheit; einem mit Cholesterin dekorierten Polymer; und einem Zielantigen.

4. Verfahren nach Anspruch 1, wobei die Membranankereinheit ein Antikörper gegen ein Zelloberflächenprotein ist; wobei optional das Zelloberflächenprotein ein Differenzierungscluster("CD")-Protein ist.

5. Verfahren nach Anspruch 1, wobei der Linker eine spaltbare Einheit umfasst, die ausgewählt wird aus einer Disulfid-Spacer-Einheit; einer Carbamat-Spacer-Einheit; einem photospaltbaren Spacer; und einem durch Uracil-DNA-Glycosylase spaltbaren (UDG-spaltbaren) Spacer.

6. Verfahren nach Anspruch 1, wobei das Abspalten des Linkers das Inkontaktbringen der Partition mit einem Reagenz, ausgewählt aus Dithiothreitol (DTT) und Diethylentriamin (DETA), umfasst.

7. Verfahren nach Anspruch 1, wobei die Vernetzung katalysierenden Einheiten ein Enzym sind, das ausgewählt wird aus Peroxidase; Transglutaminase; Tyrosinase; und Laccase; wobei optional das Enzym Meerrettichperoxidase (HRP) ist.

8. Verfahren nach Anspruch 1, wobei die Vernetzung katalysierende Einheit eine nichtenzymatische Verbindung ist, die aus Hämatin und Umbelliferon ausgewählt wird.

9. Verfahren nach Anspruch 1, wobei die Vernetzung katalysierenden Einheiten HRP sind, die Vernetzungsvorläufer-Einheiten Phenol sind und der Vernetzung bildende Initiator eine Verbindung ist, die eine Peroxideinheit umfasst; wobei optional die Verbindung, die eine Peroxideinheit umfasst, $H_2O_2$ ist.

10. Verfahren nach Anspruch 1, wobei der Vernetzung bildende Initiator aus $H_2O_2$ und $O_2$ ausgewählt wird.

11. Verfahren nach Anspruch 1, wobei das Inkontaktbringen der Partition mit einem Vernetzung bildenden Initiator einen Mizellen-vermittelten Transport des Initiators in die Partition umfasst.

12. Verfahren nach Anspruch 1, wobei das lineare Polymer aus einem Olefincopolymer, einem Polyolefin, einem Acryl, einem Polyacrylamid, einem Poly(oxazolin), einem Vinylpolymer, einem Polyester, einem Polycarbonat, einem Polyamid, einem Polyimid, einem Formaldehydharz, einem Polyurethan, einem Etherpolymer, einem zellulosischen Stoff, einem thermoplastischen Elastomer und einem thermoplastischen Polyurethan ausgewählt wird.

13. Verfahren nach Anspruch 1, wobei das lineare Polymer ferner eine modifizierbare Seitenkette umfasst.

14. Verfahren nach Anspruch 13,
wobei die modifizierbare Seitenkette eine Amineinheit umfasst.

15. Verfahren nach Anspruch 13 oder 14,
ferner umfassend, unter geeigneten Reaktionsbedingungen, das Inkontaktbringen der mit Hydrogel beschichteten Zelle mit einer nachweisbaren Markierungseinheit, die eine Gruppe umfasst, die fähig ist, eine kovalente Bindung zu der modifizierbaren Seitenkette des Hydrogels auszubilden.

16. Verfahren nach den Ansprüchen 13 bis 15,
ferner umfassend das Inkontaktbringen der mit Hydrogel beschichteten Zelle mit einer Oberfläche eines festen Substrats unter geeigneten Reaktionsbedingungen, wobei die Oberfläche eine Gruppe umfasst, die fähig ist, unter der Reaktionsbedingung eine kovalente Bindung zu der modifizierbaren Seitenkette des Hydrogels auszubilden, wodurch die mit Hydrogel beschichtete Zelle kovalent an das feste Substrat gebunden wird.

**Revendications**

1. Procédé de compartimentation de cellules comprenant :

(a) la génération d'un compartiment contenant : (i) une cellule comprenant une pluralité de fractions de catalyse de réticulation fixées à sa membrane par l'intermédiaire d'un lieur comprenant une fraction d'ancrage membranaire ; et (ii) un polymère linéaire comprenant une fraction précurseur de réticulation ;
(b) la mise en contact du compartiment avec un initiateur de formation de réticulation ; dans lequel un enrobage hydrogel de la cellule est formé ; et
(c) le clivage du lieur ;

moyennant quoi les fractions de catalyse de réticulation sont libérées, ce qui a pour résultat une épaisseur accrue d'enrobage hydrogel de la cellule.

2. Procédé selon la revendication 1, dans lequel le compartiment est une gouttelette distincte ou un puits.

3. Procédé selon la revendication 1, dans lequel la fraction d'ancrage membranaire est choisie parmi une fraction d'ancrage biocompatible pour membrane cellulaire (BAM) ; un anticorps ; un anticorps dirigé contre une membrane cellulaire ou une protéine de surface ; une fraction cholestérol-oligonucléotide ; une fraction 3'-cholestérol-TEG ; un polymère décoré par cholestérol ; et un antigène cible.

4. Procédé selon la revendication 1, dans lequel la fraction d'ancrage membranaire est un anticorps dirigé contre une protéine de surface cellulaire ; éventuellement, dans lequel la protéine de surface cellulaire est une protéine de groupe de différenciation (« CD »).

5. Procédé selon la revendication 1, dans lequel le lieur comprend une fraction clivable choisie parmi une fraction espaceur disulfure ; une fraction espaceur carbamate ; un espaceur photoclivable ; et un espaceur clivable par uracile-ADN glycosylase (clivable par UDG).

6. Procédé selon la revendication 1, dans lequel le clivage du lieur comprend la mise en contact du compartiment avec un réactif choisi parmi le dithiothréitol (DTT) et la diéthylènetriamine (DETA).

7. Procédé selon la revendication 1, dans lequel les fractions de catalyse de réticulation sont une enzyme choisie parmi la peroxydase ; la transglutaminase ; la tyrosinase ; et la laccase ; éventuellement dans lequel l'enzyme est la peroxydase de raifort (HRP).

8. Procédé selon la revendication 1, dans lequel la fraction de catalyse de réticulation est un composé non enzymatique choisi parmi l'hématine et l'ombelliférone.

9. Procédé selon la revendication 1, dans lequel les fractions de catalyse de réticulation sont la HRP, les fractions précurseurs de réticulation sont le phénol, et l'initiateur de formation de réticulation est un composé comprenant une fraction peroxyde ; éventuellement, dans lequel le composé comprenant une fraction peroxyde est $H_2O_2$.

10. Procédé selon la revendication 1, dans lequel l'initiateur de formation de réticulation est choisi parmi $H_2O_2$, et $O_2$.

11. Procédé selon la revendication 1, dans lequel la mise en contact du compartiment avec un initiateur de formation de réticulation comprend le transport à médiation micellaire de l'initiateur dans le compartiment.

12. Procédé selon la revendication 1, dans lequel le polymère linéaire est choisi parmi un copolymère d'oléfine, une polyoléfine, un acrylique, un polyacrylamide, une poly(oxazoline), un polymère vinylique, un polyester, un polycarbonate, un polyamide, un polyimide, une résine de formaldéhyde, un polyuréthane, un polymère d'éther, une matière cellulosique, un élastomère thermoplastique, et un polyuréthane thermoplastique.

13. Procédé selon la revendication 1, dans lequel le polymère linéaire comprend en outre une chaîne latérale modifiable.

14. Procédé selon la revendication 13,
dans lequel la chaîne latérale modifiable comprend une fraction amine.

15. Procédé selon la revendication 13 ou 14,
comprenant en outre la mise en contact, dans des conditions de réaction appropriées, de la cellule enrobée d'hydrogel avec une fraction à marqueur détectable comprenant un groupe capable de former une liaison covalente avec la chaîne latérale modifiable de l'hydrogel.

16. Procédé selon les revendications 13 à 15,
comprenant en outre la mise en contact, dans des conditions de réaction appropriées, de la cellule enrobée d'hydrogel avec une surface d'un substrat solide, dans lequel la surface comprend un groupe capable de former une liaison covalente avec la chaîne latérale modifiable de l'hydrogel dans les conditions de réaction, moyennant quoi la cellule enrobée d'hydrogel est fixée de manière covalente au substrat solide.

*FIG. 1*

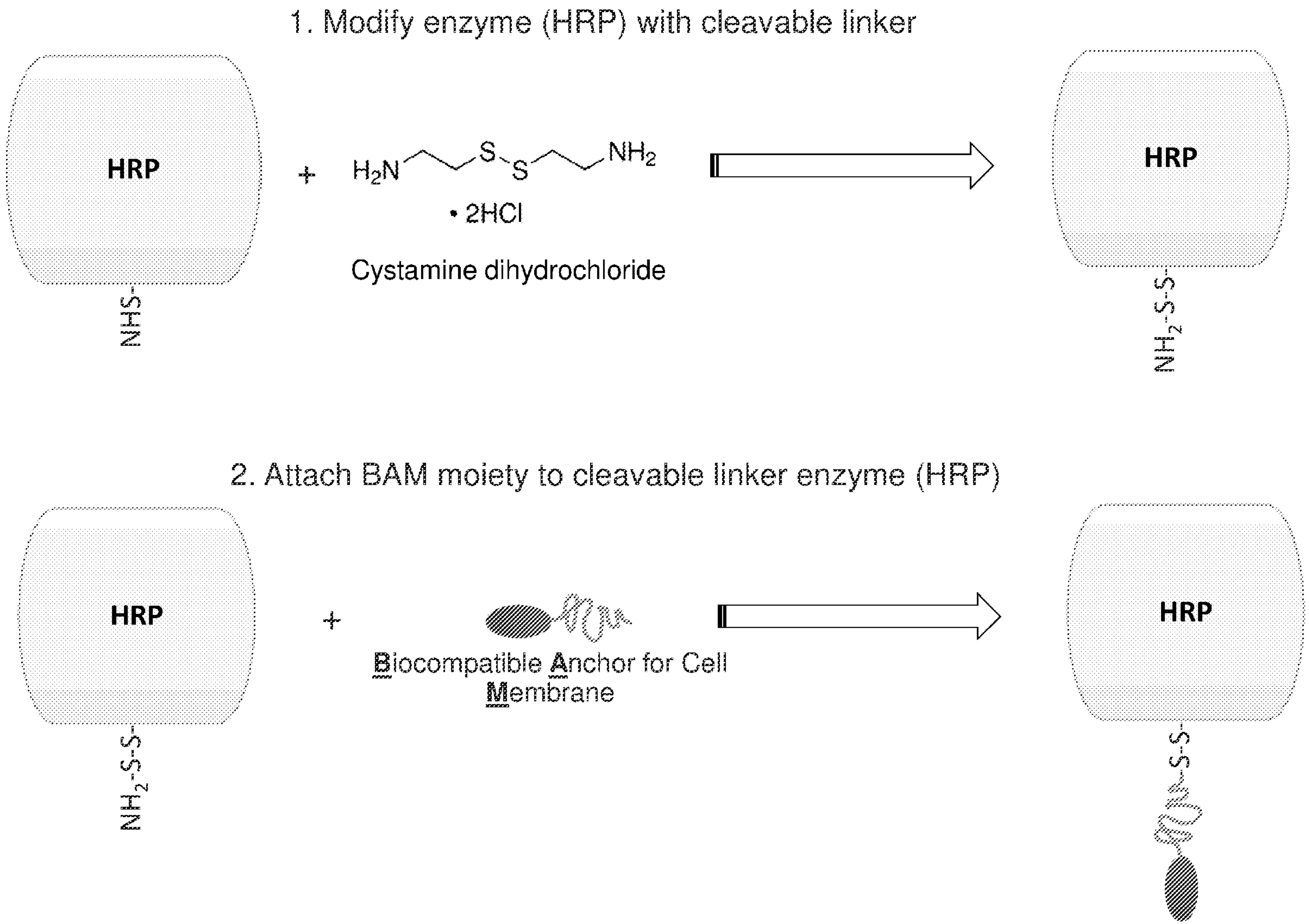
1. Modify enzyme (HRP) with cleavable linker
HRP
Cystamine dihydrochloride
HRP
2. Attach BAM moiety to cleavable linker enzyme (HRP)
HRP
Biocompatible Anchor for Cell Membrane
HRP

*FIG. 2*

3. Cell decoration

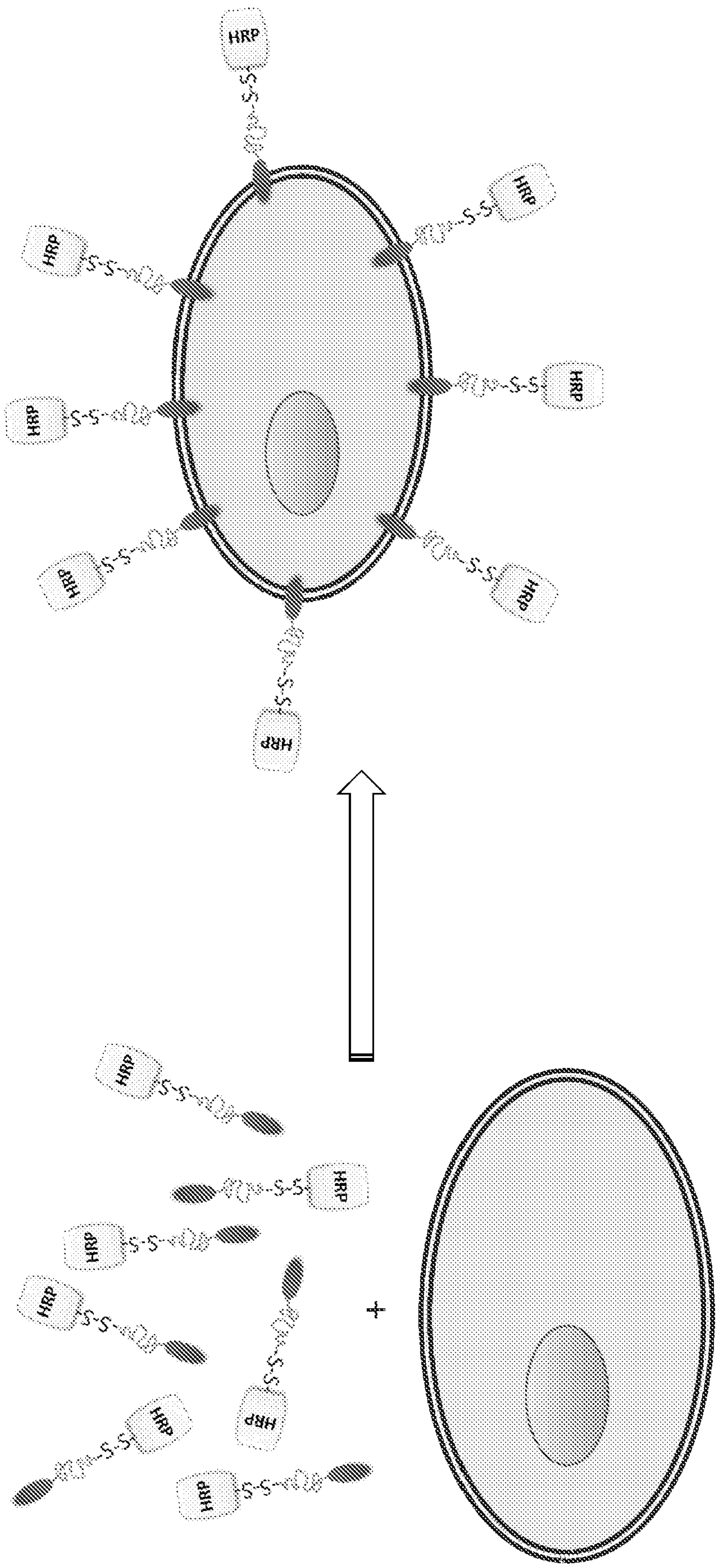

*FIG. 3*

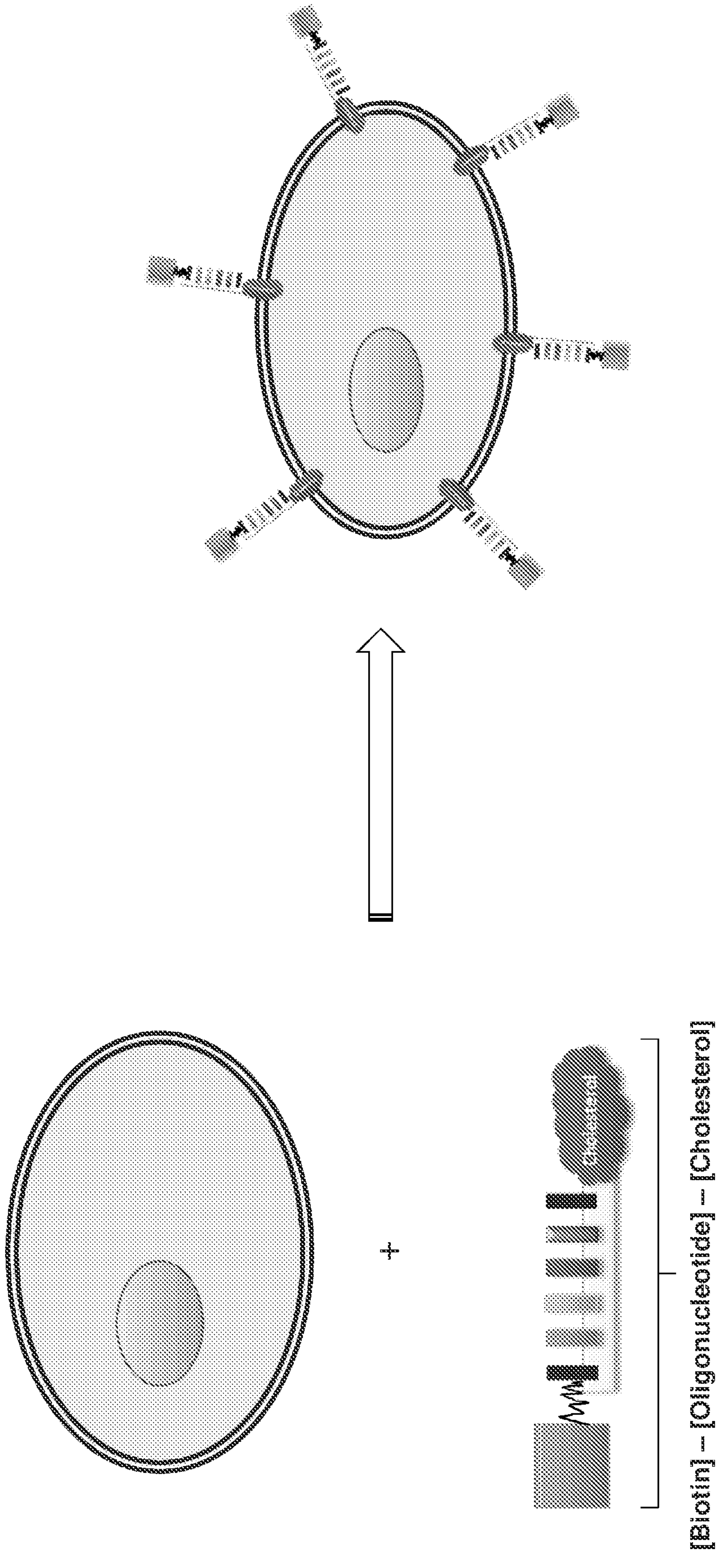

FIG. 4

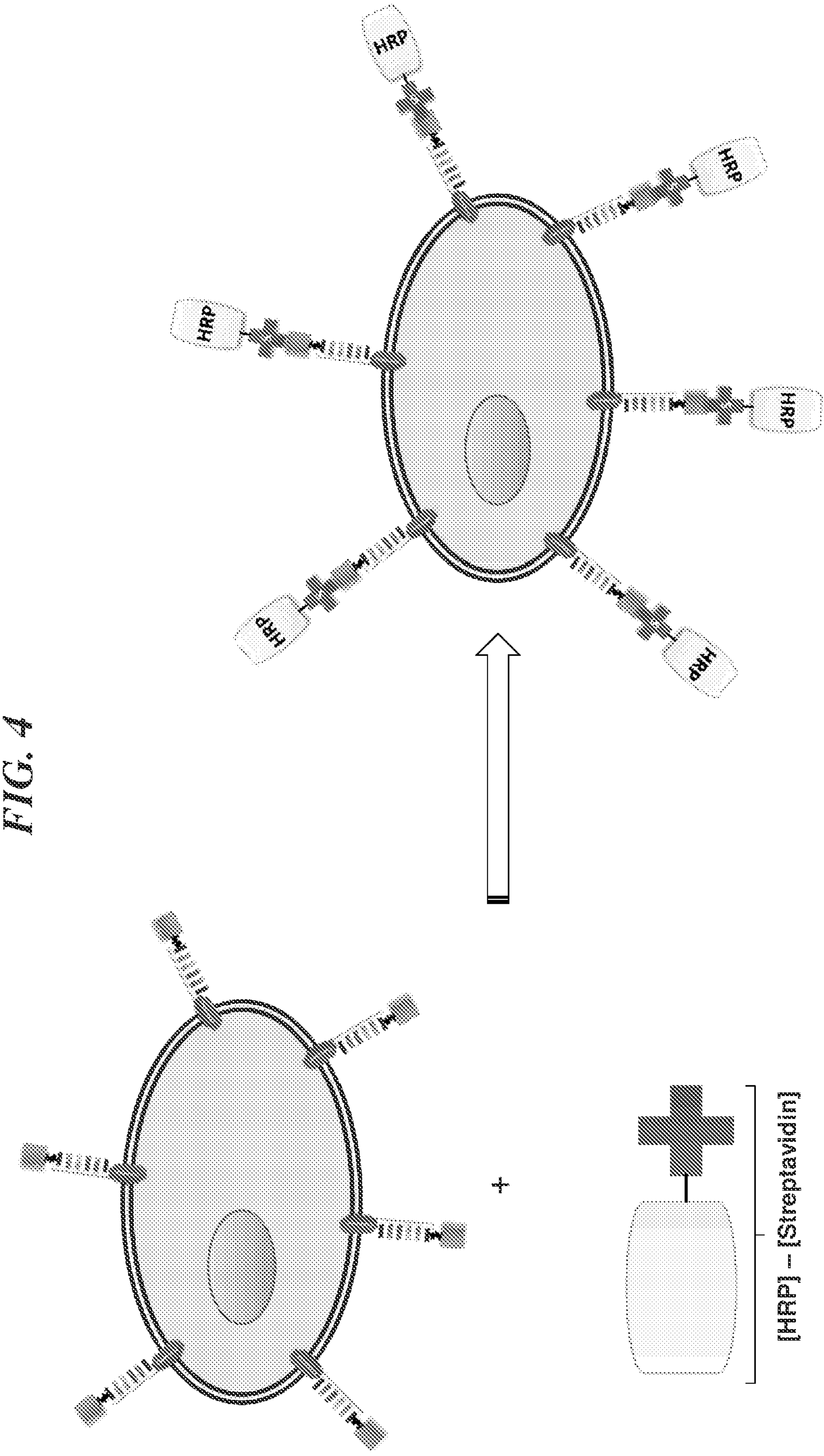

FIG. 5

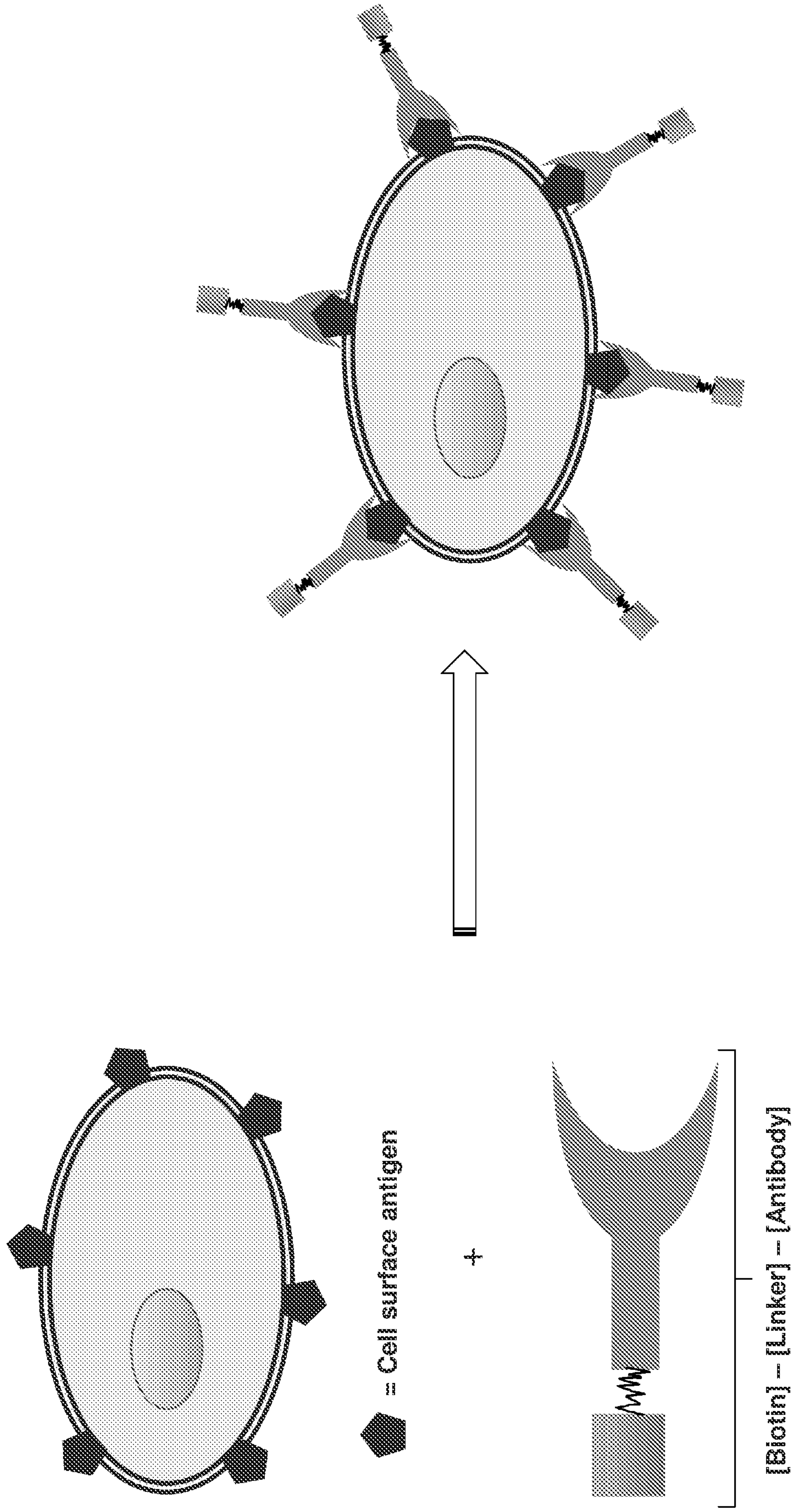

FIG. 6

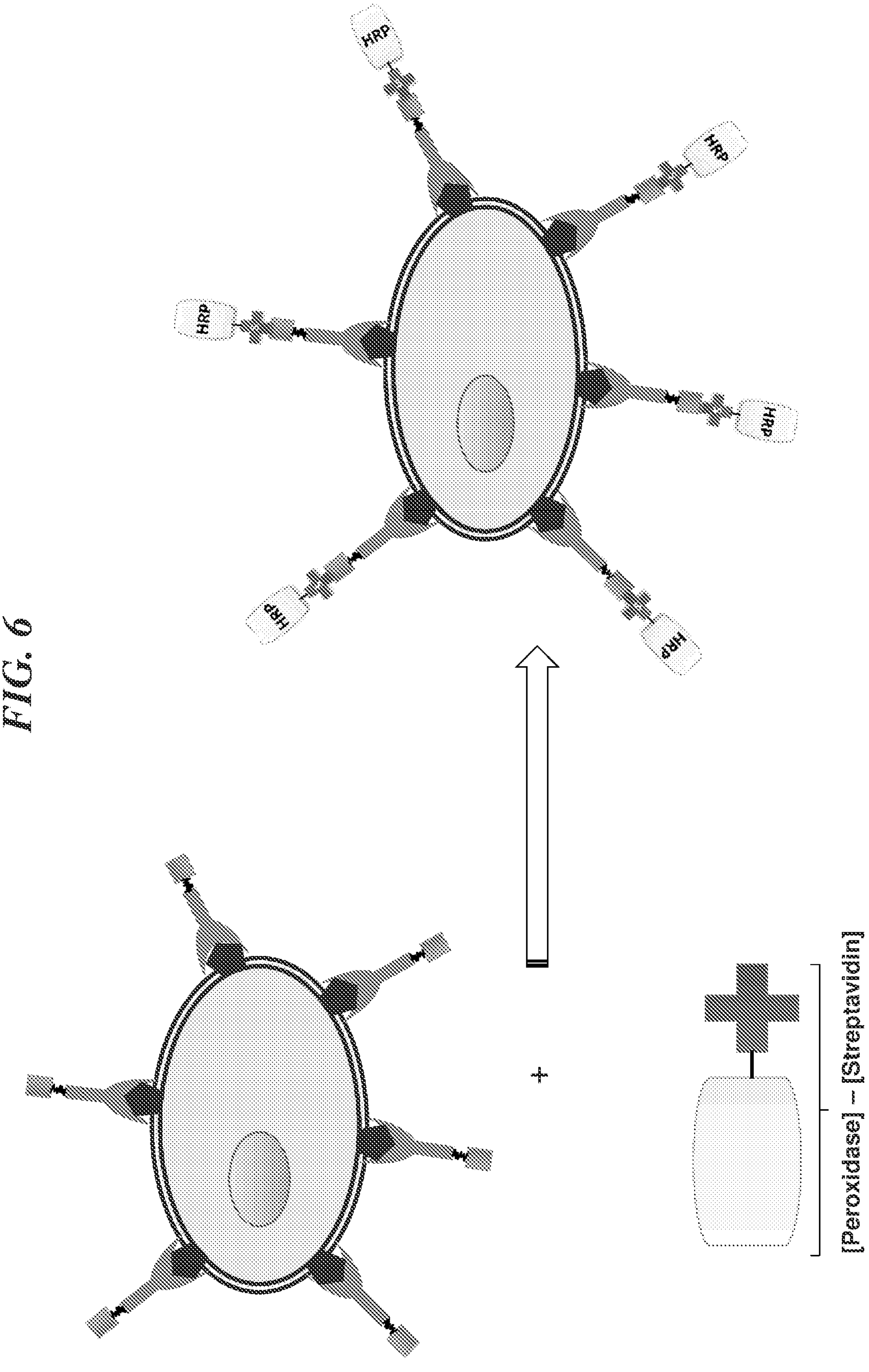

FIG. 7

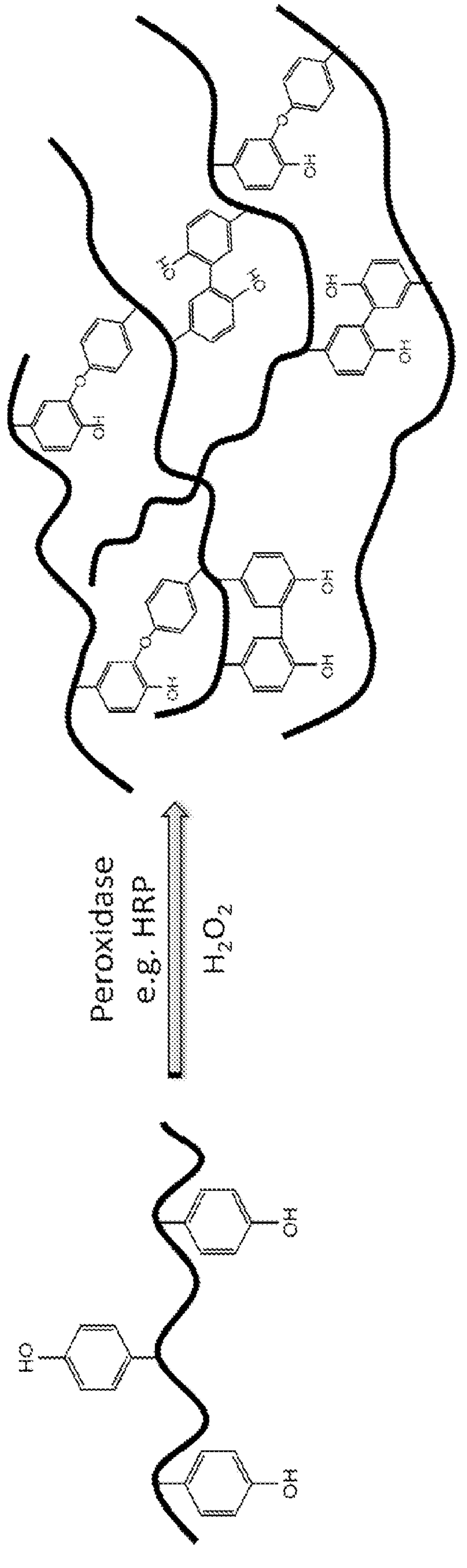

# *FIG. 8*

1. Prepare aqueous monomer solution

*acrylamide*

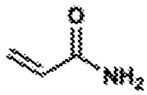

*3-aminopropyl methacrylamide*

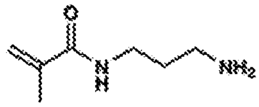

*sodium formate*

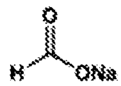

*5'-acrydite oligo*

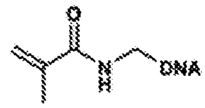

2. Generate linear polymers

VA-044 thermal initiated polymerization

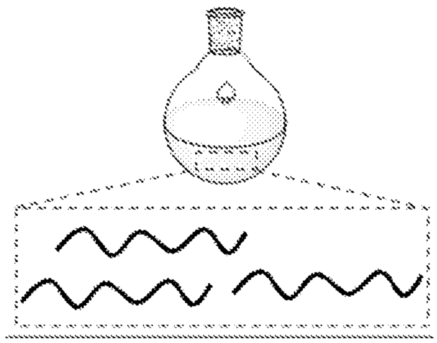

3. Phenol-functionalize linear polymers

Couple phenol to aminopropyl side chains

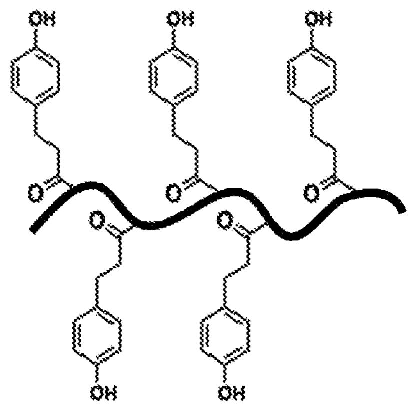

*FIG. 9*

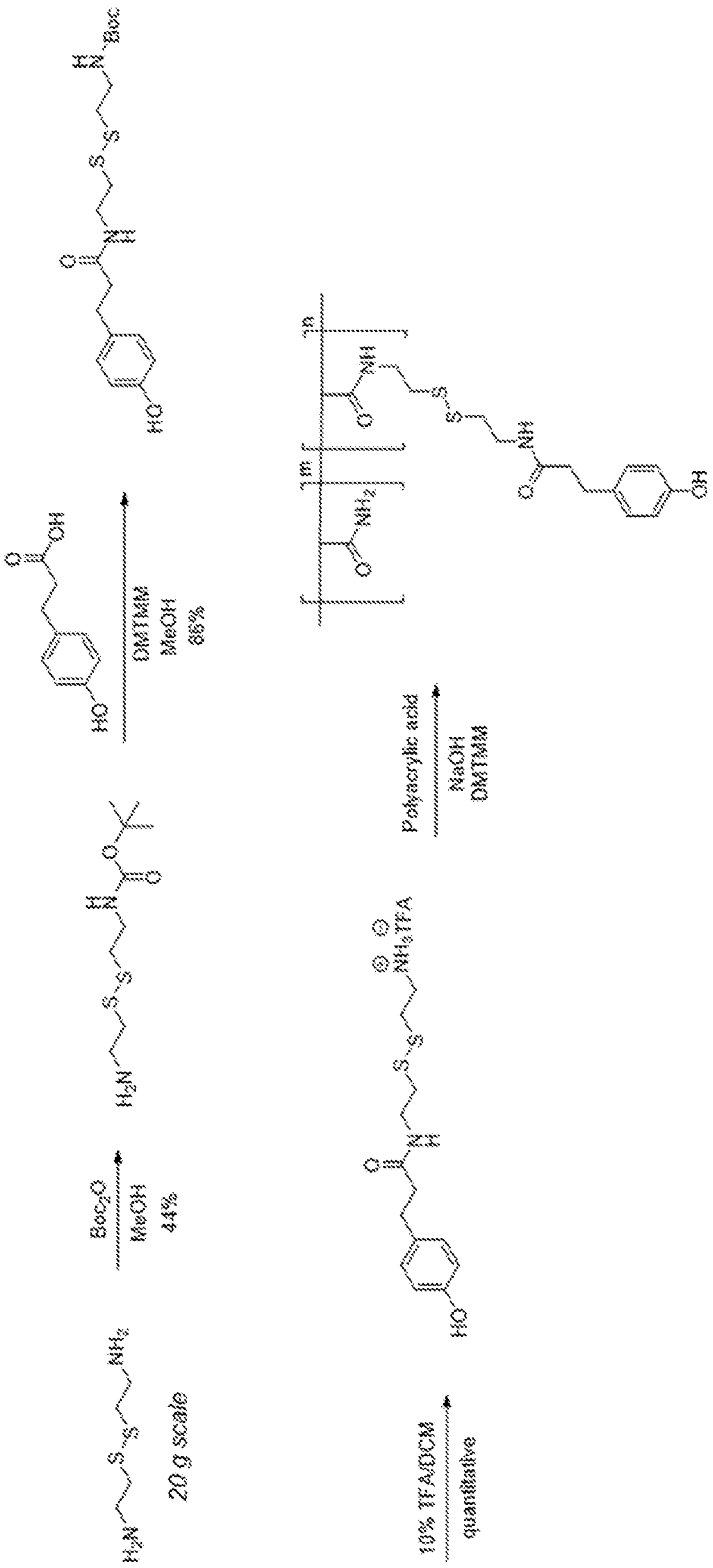
20 g scale
$Boc_2O$
MeOH
44%
DMTMM
MeOH
86%
10% TFA/DCM
quantitative
Polyacrylic acid
NaOH
DMTMM

FIG. 10

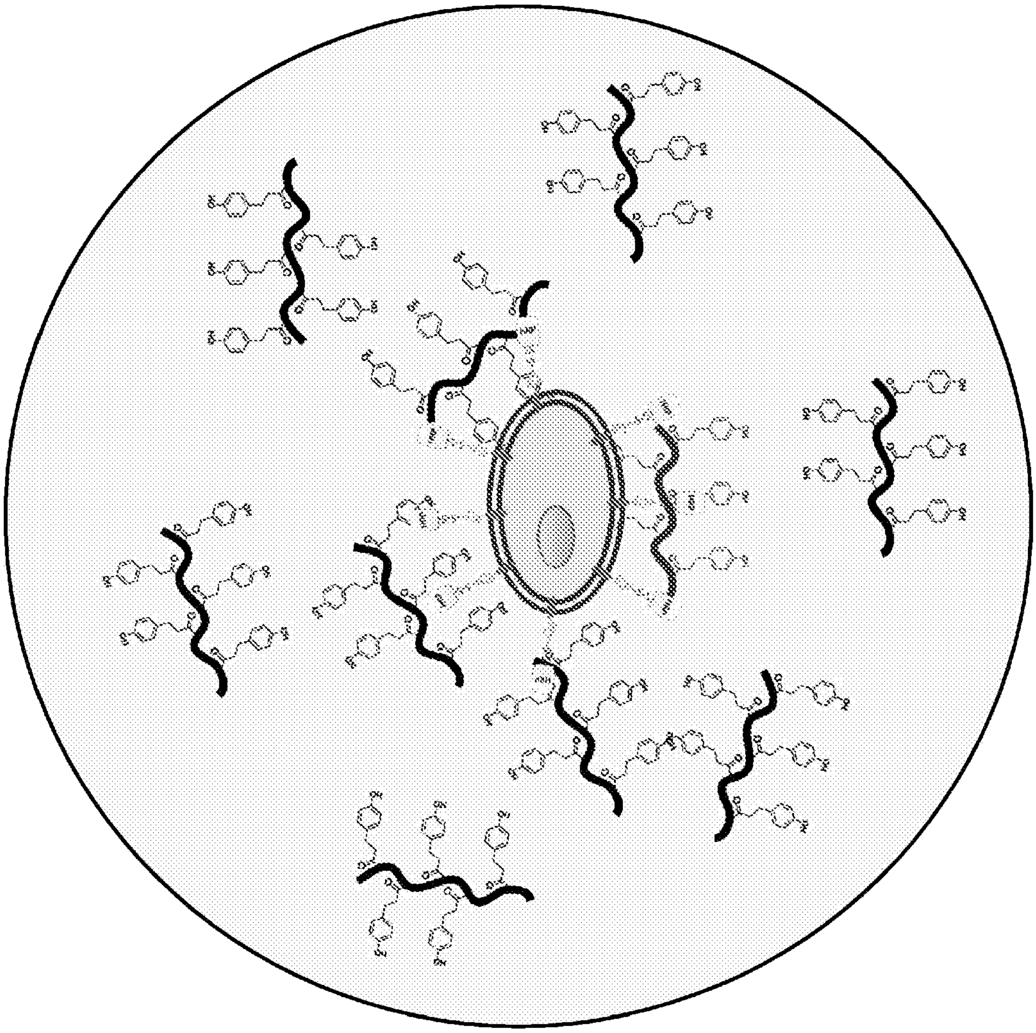

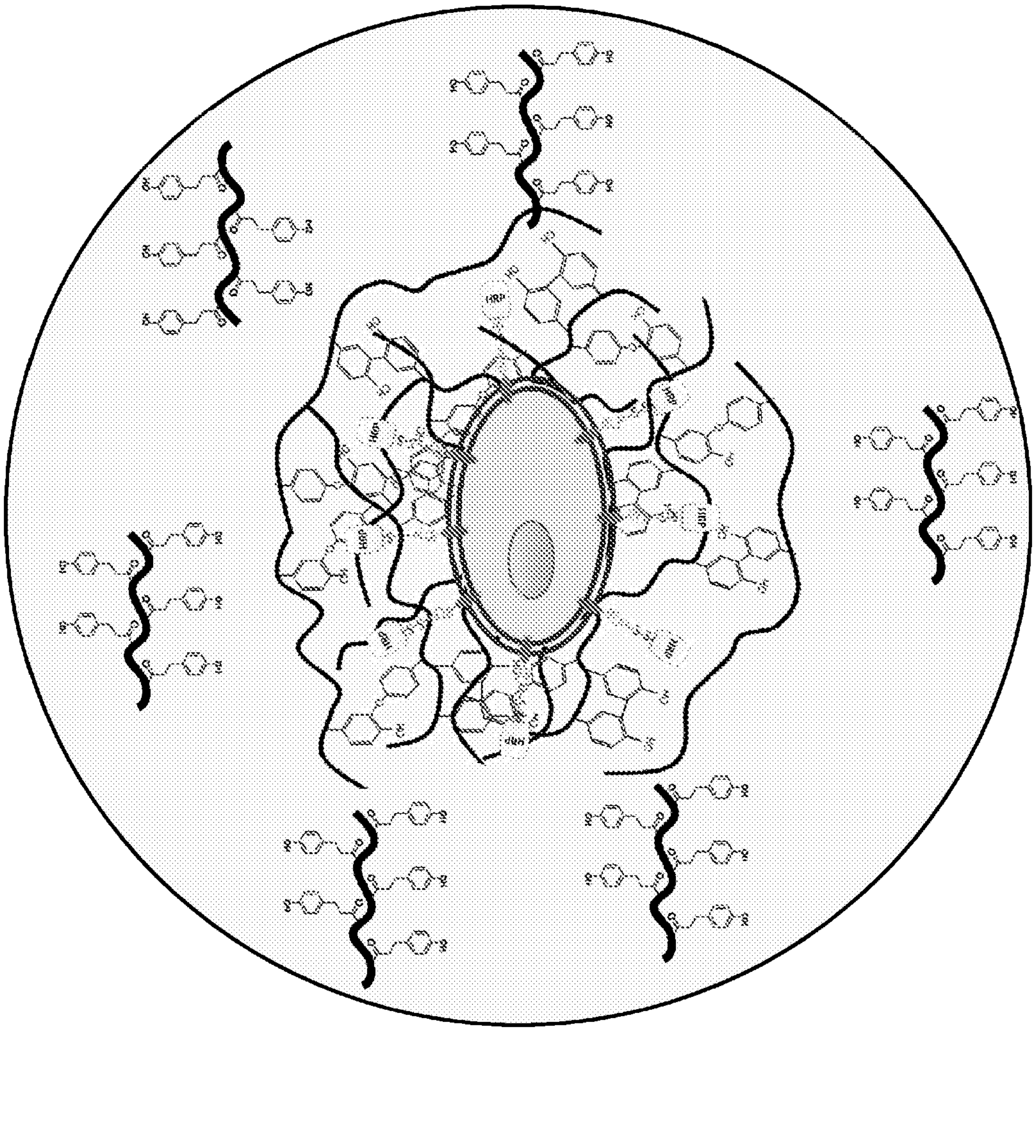

FIG. 11

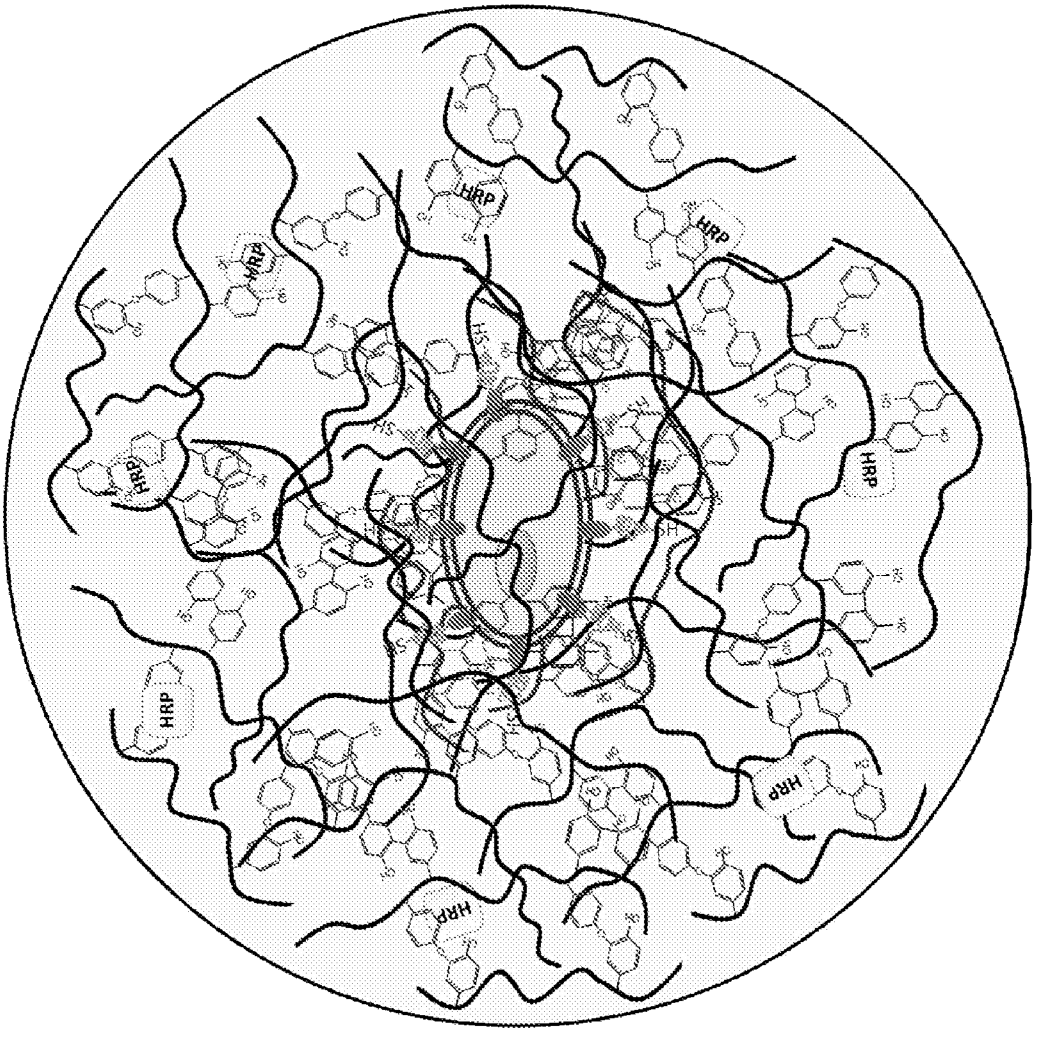


FIG. 12

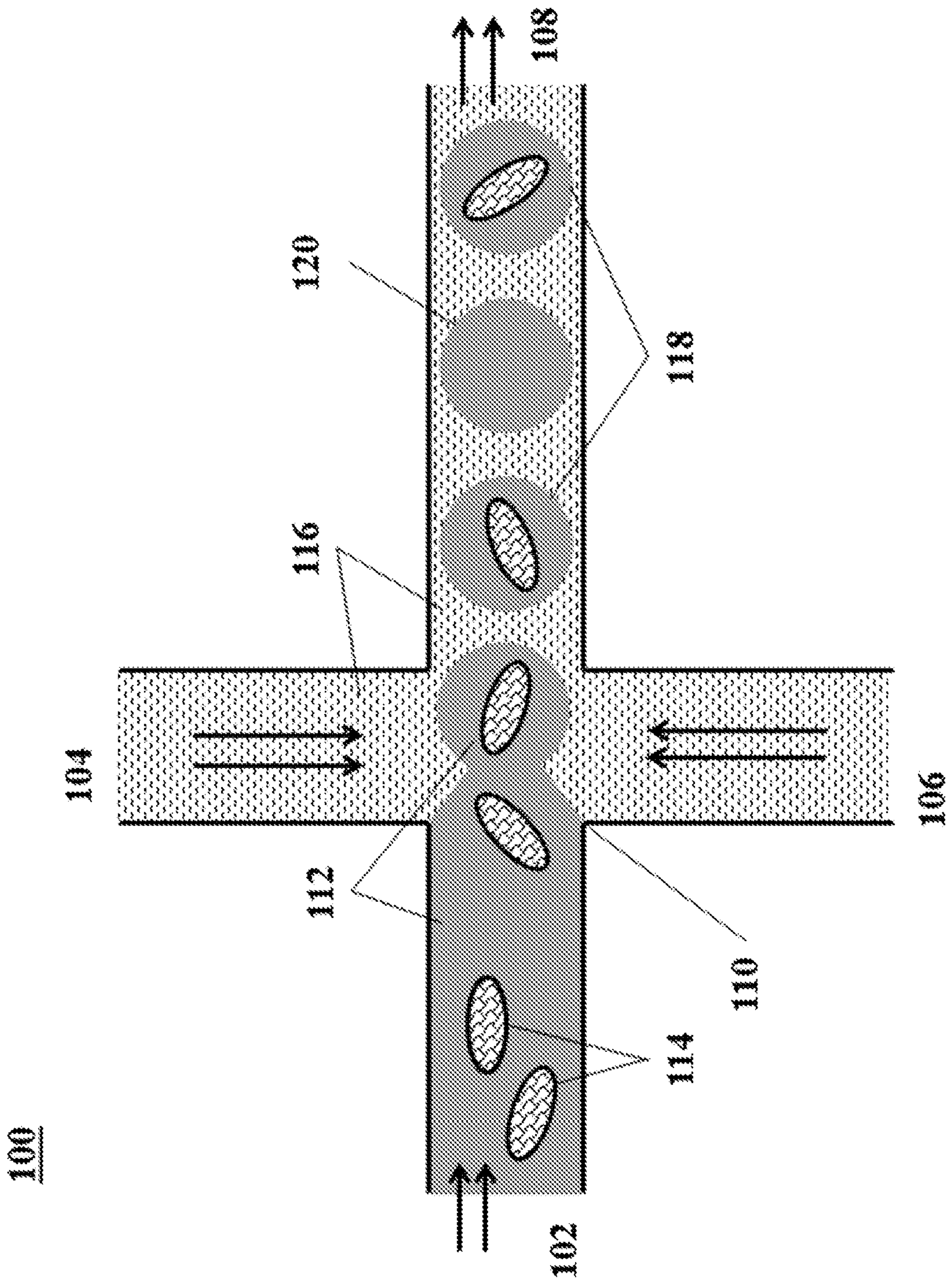


FIG. 13

FIG. 14

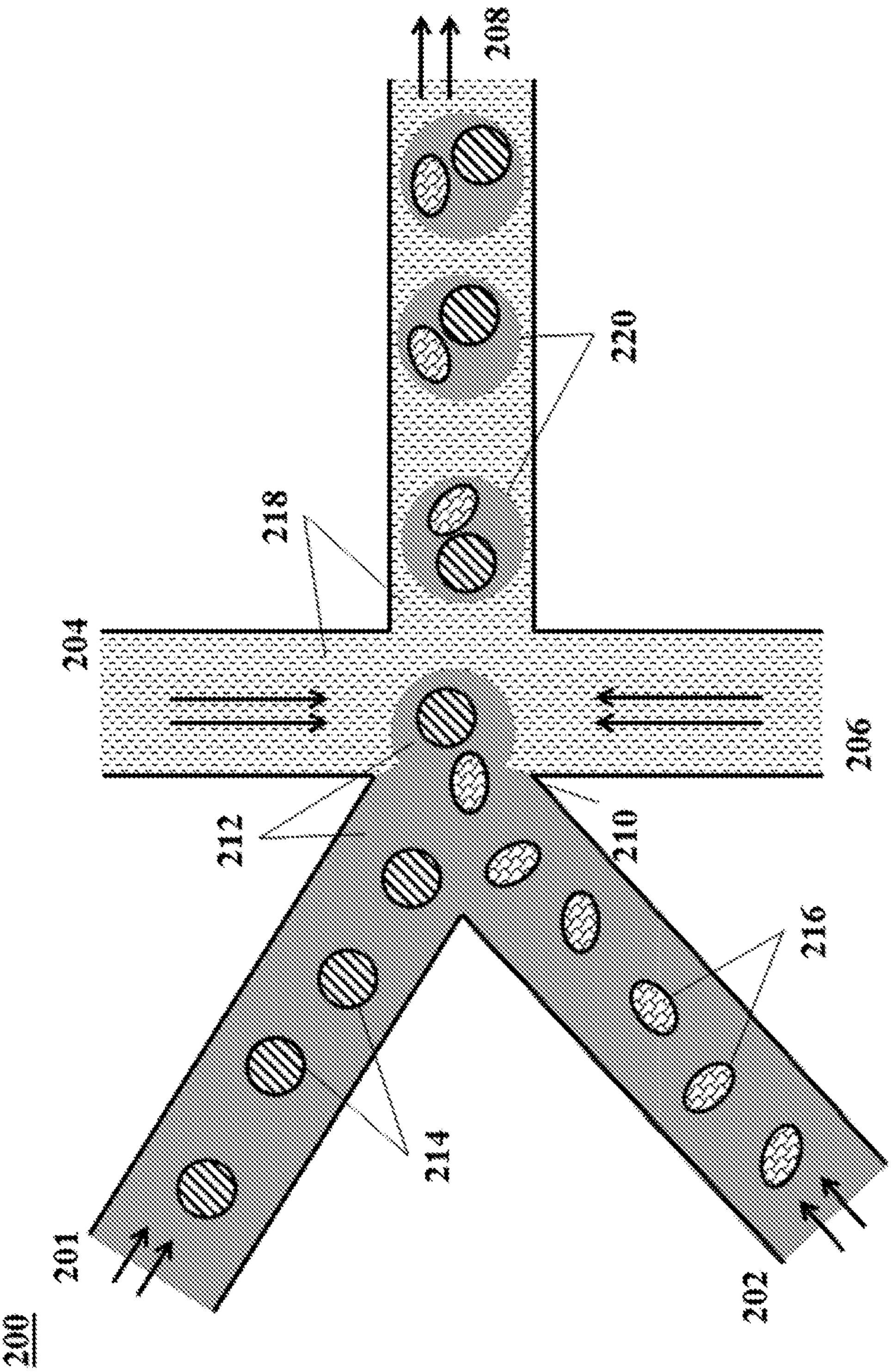

*FIG. 15*

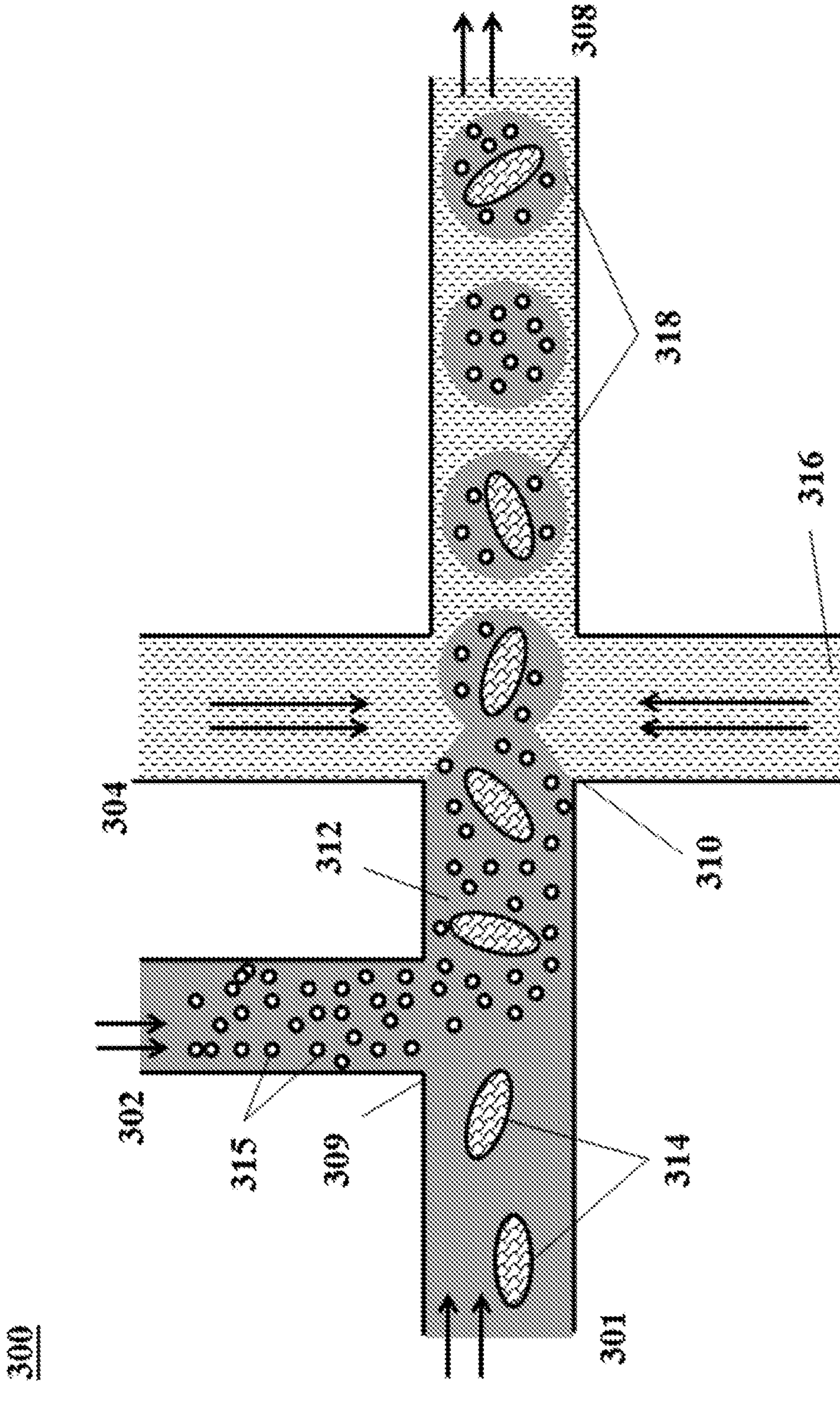

FIG. 16

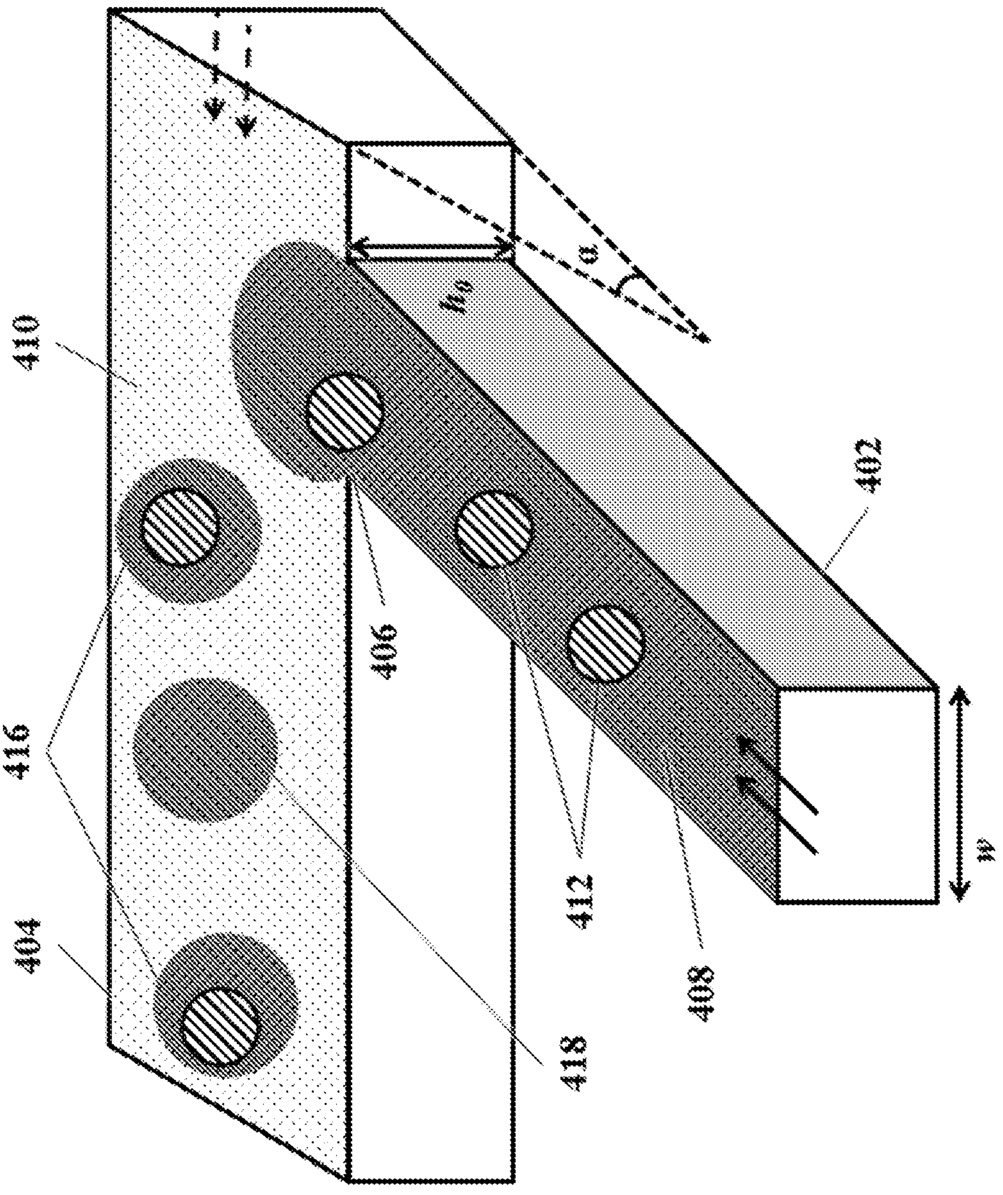


400

FIG. 17

500

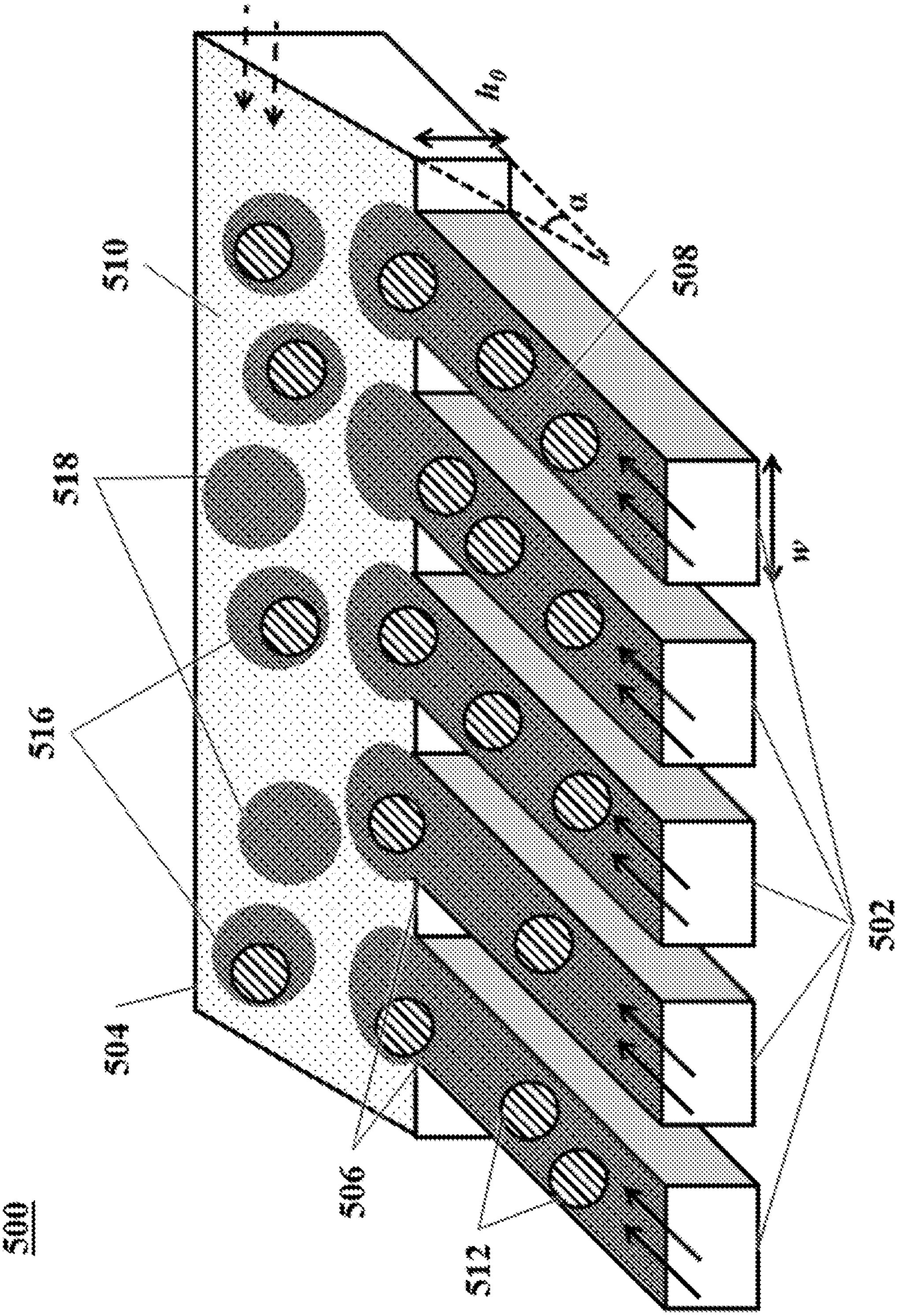

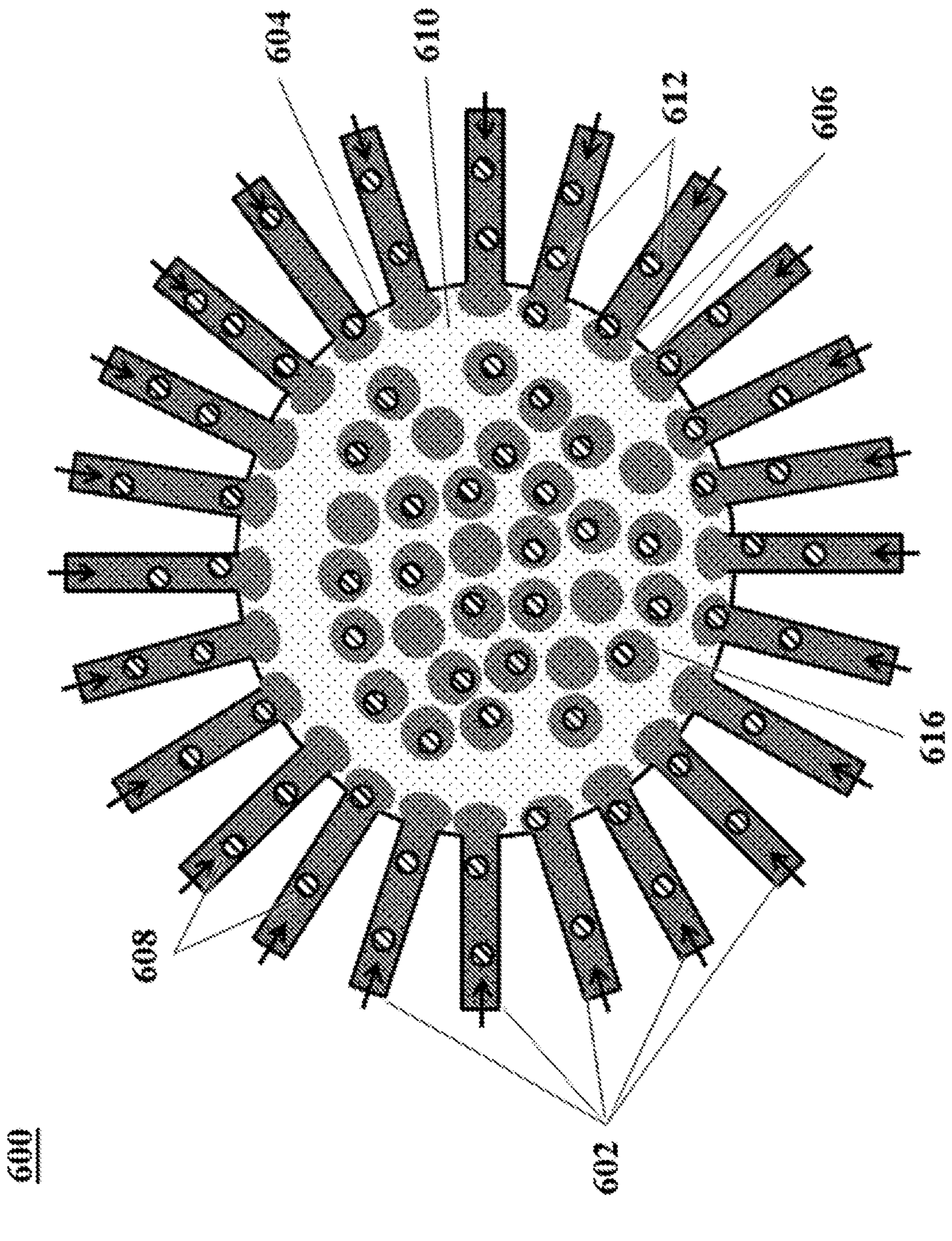


FIG. 18

*FIG. 19*

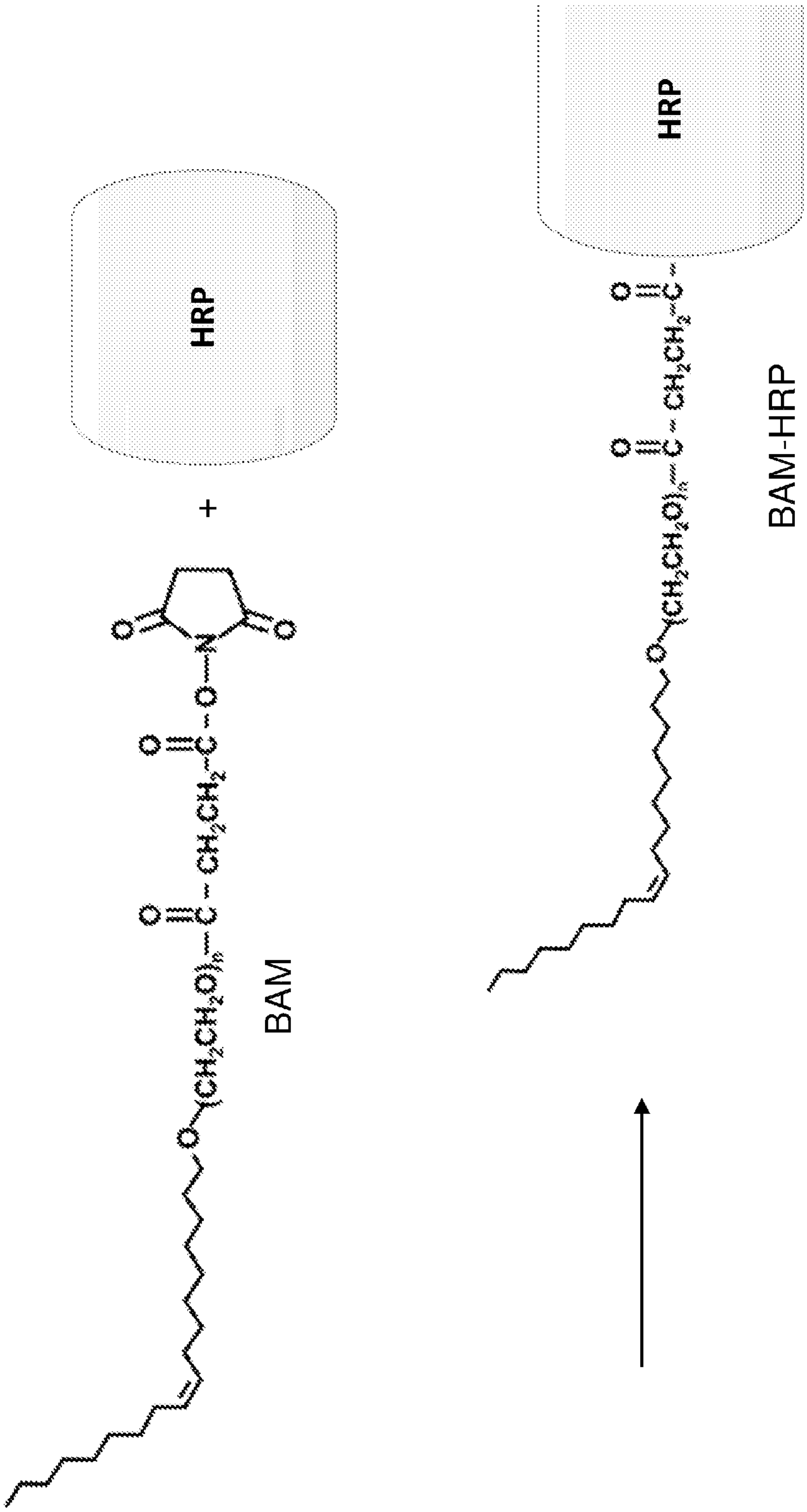

BAM

BAM-HRP

FIG. 20

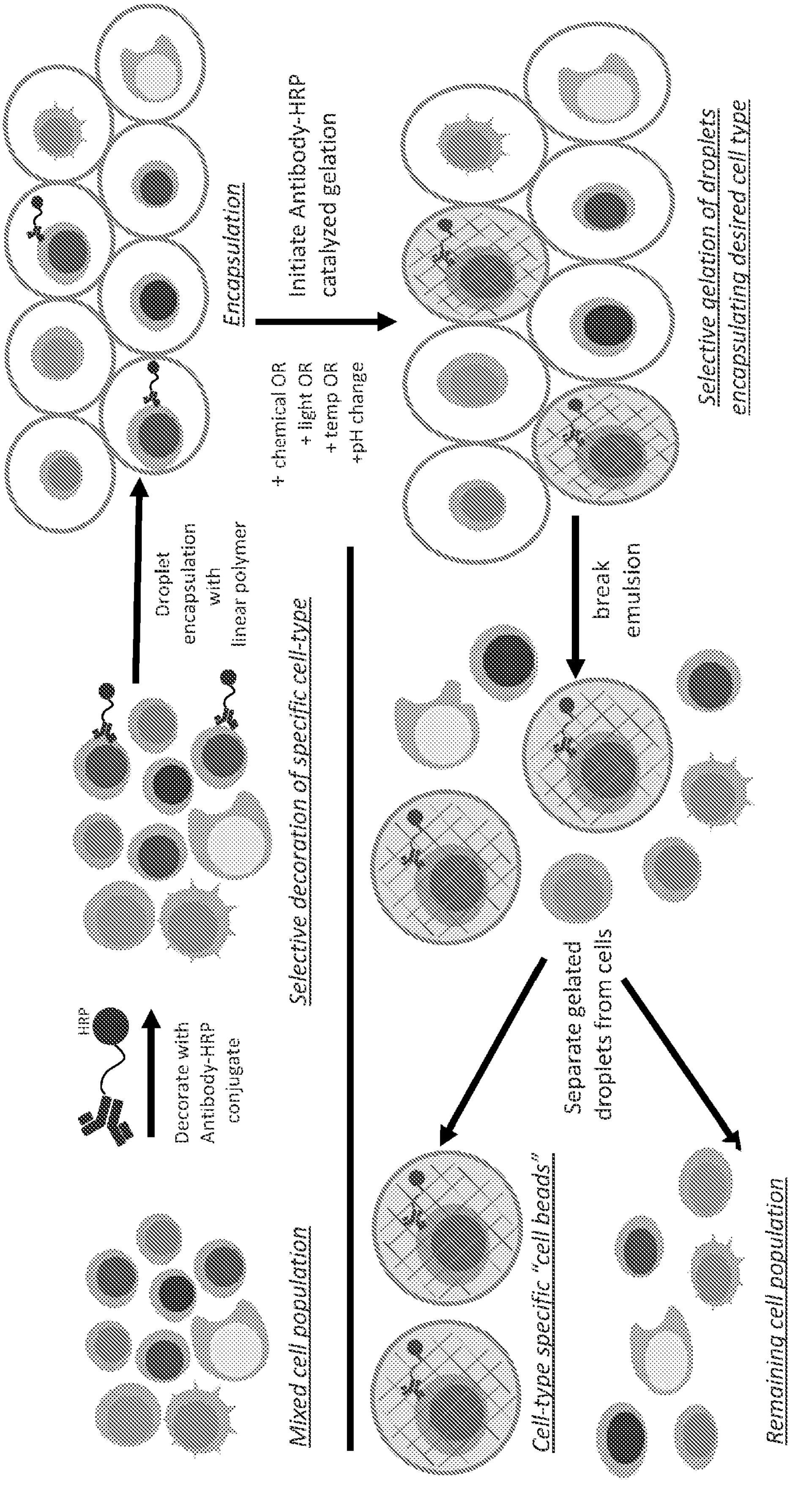
Mixed cell population
HRP
Decorate with Antibody-HRP conjugate
Selective decoration of specific cell-type
Droplet encapsulation with linear polymer
Encapsulation
Initiate Antibody-HRP catalyzed gelation
+ chemical OR
+ light OR
+ temp OR
+pH change
Selective gelation of droplets encapsulating desired cell type
break emulsion
Separate gelated droplets from cells
Cell-type specific "cell beads"
Remaining cell population

FIG. 21

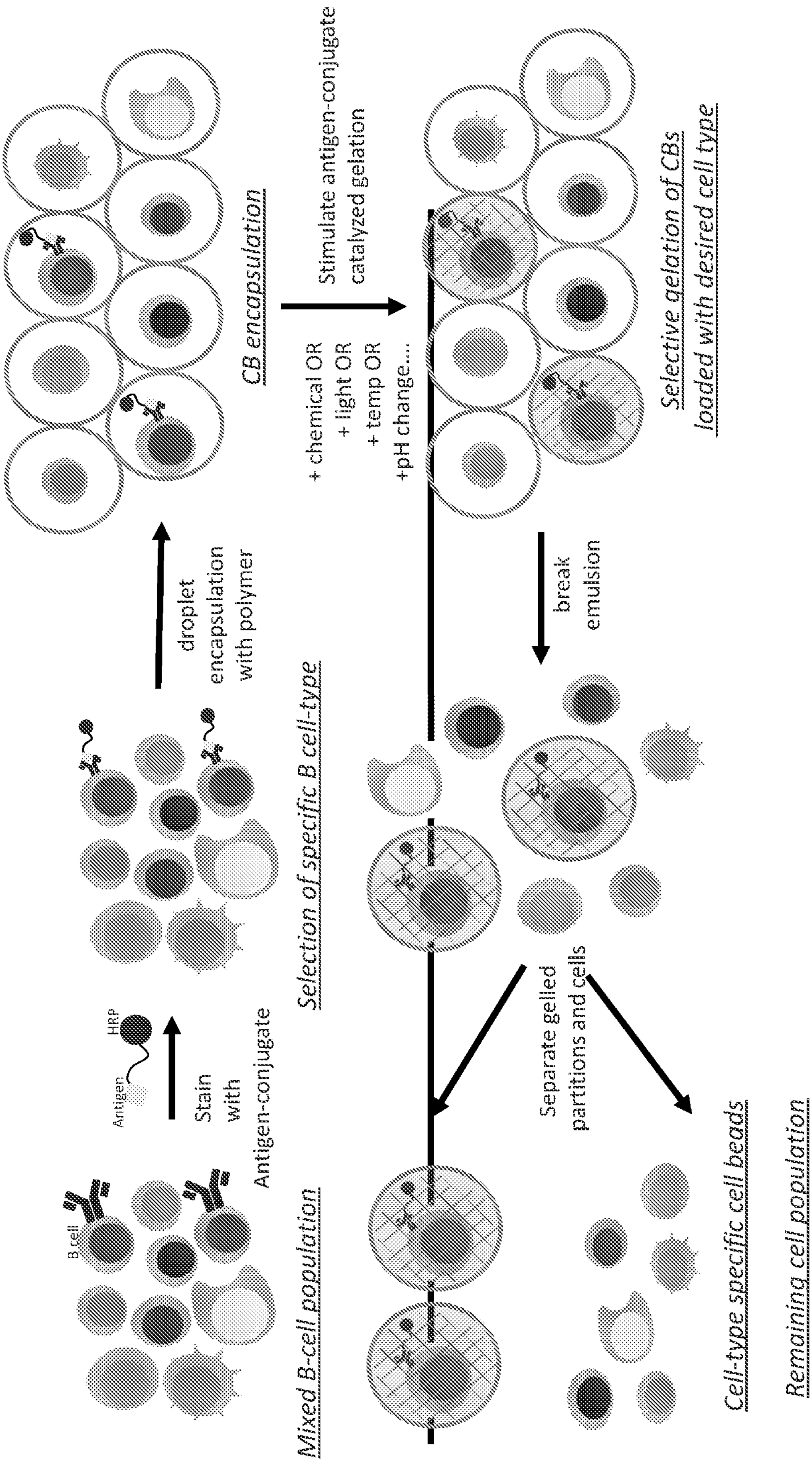
B cell
Mixed B-cell population
Antigen
HRP
Stain
with
Antigen-conjugate
Selection of specific B cell-type
droplet
encapsulation
with polymer
CB encapsulation
Stimulate antigen-conjugate
catalyzed gelation
+ chemical OR
+ light OR
+ temp OR
+pH change....
Selective gelation of CBs
loaded with desired cell type
break
emulsion
Separate gelled
partitions and cells
Cell-type specific cell beads
Remaining cell population

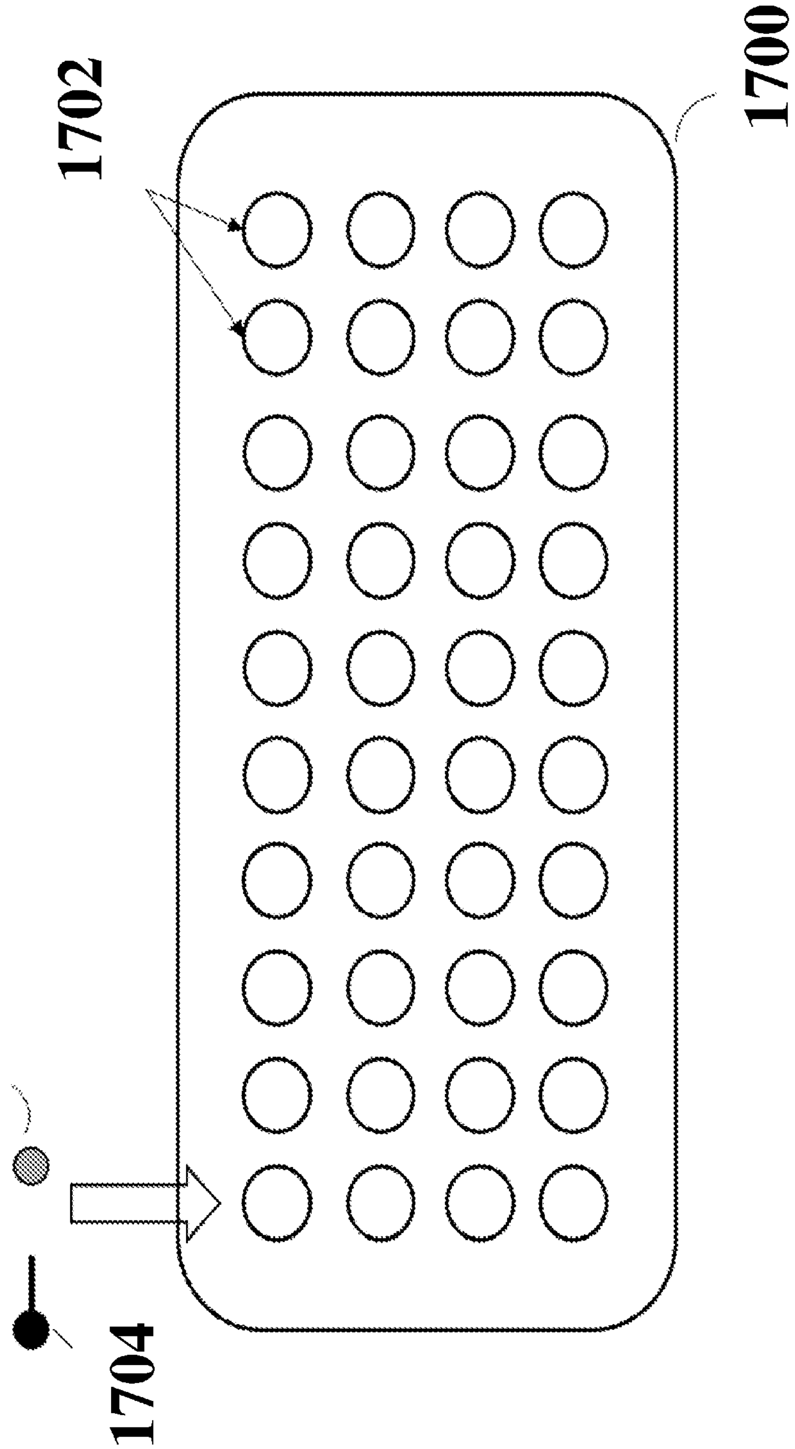


FIG. 22

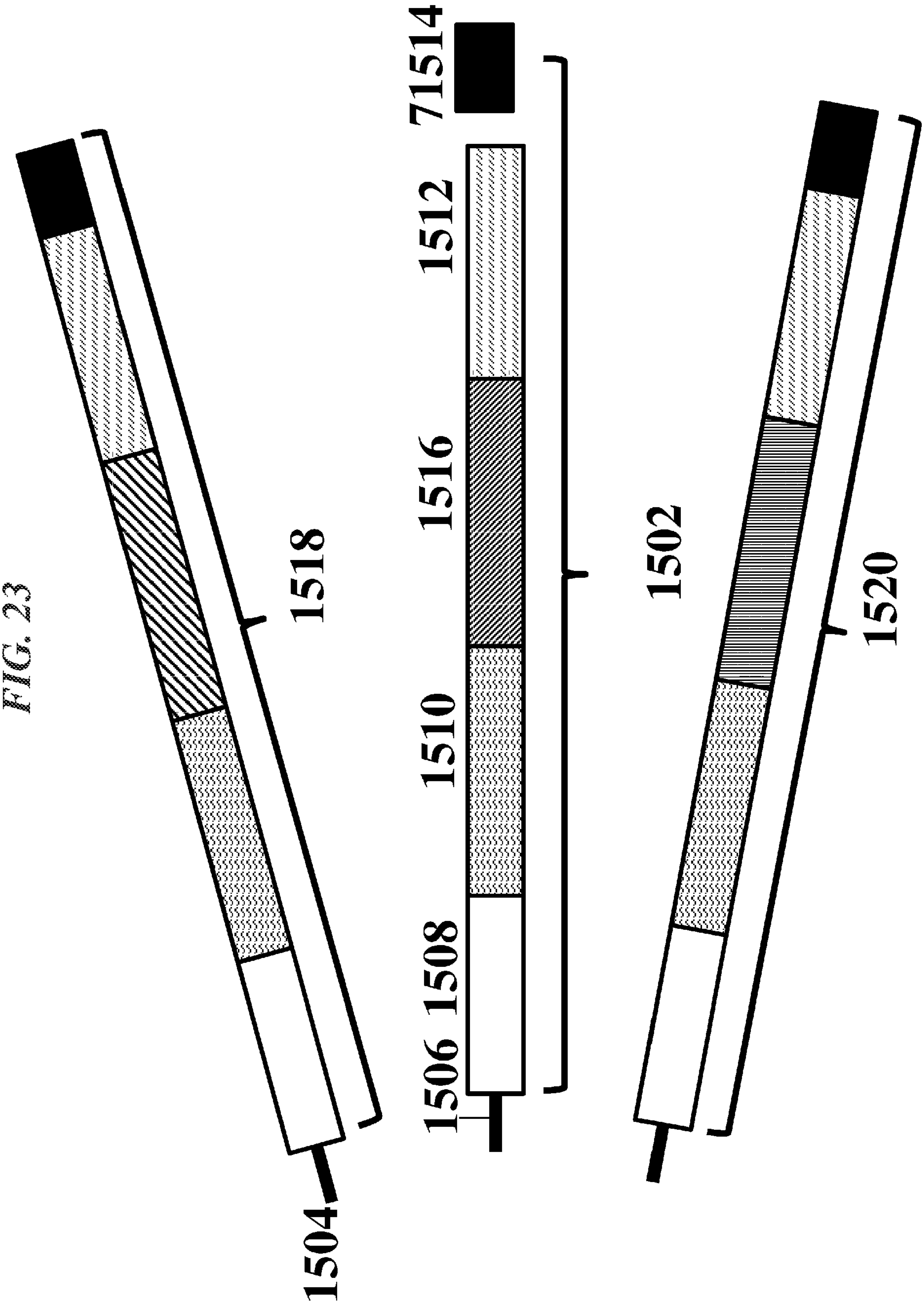


*FIG. 23*

*FIG. 24*

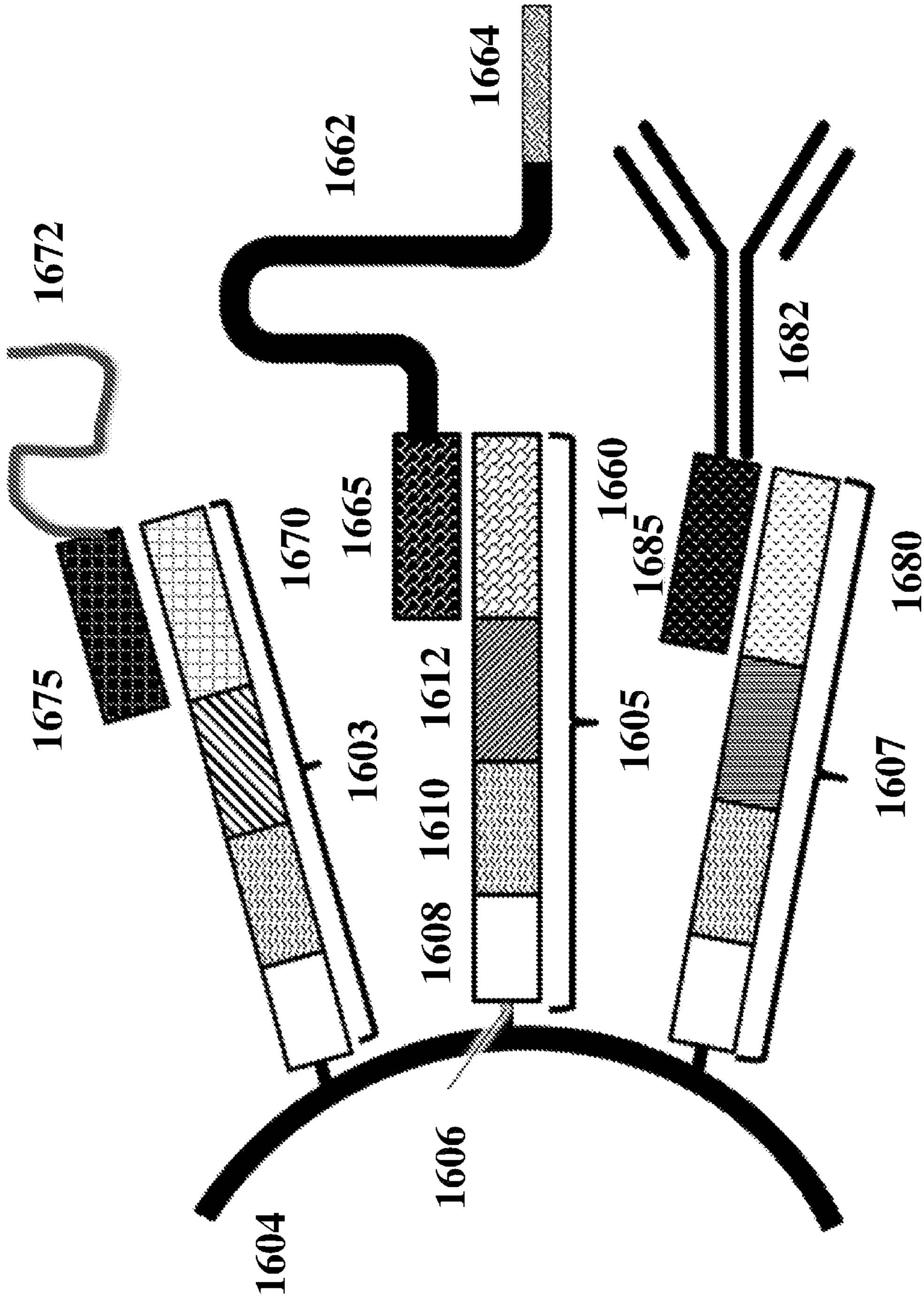
1672
1662
1664
1675
1670
1665
1660
1685
1680
1603
1612
1605
1610
1608
1607
1682
1606
1604

FIG. 25

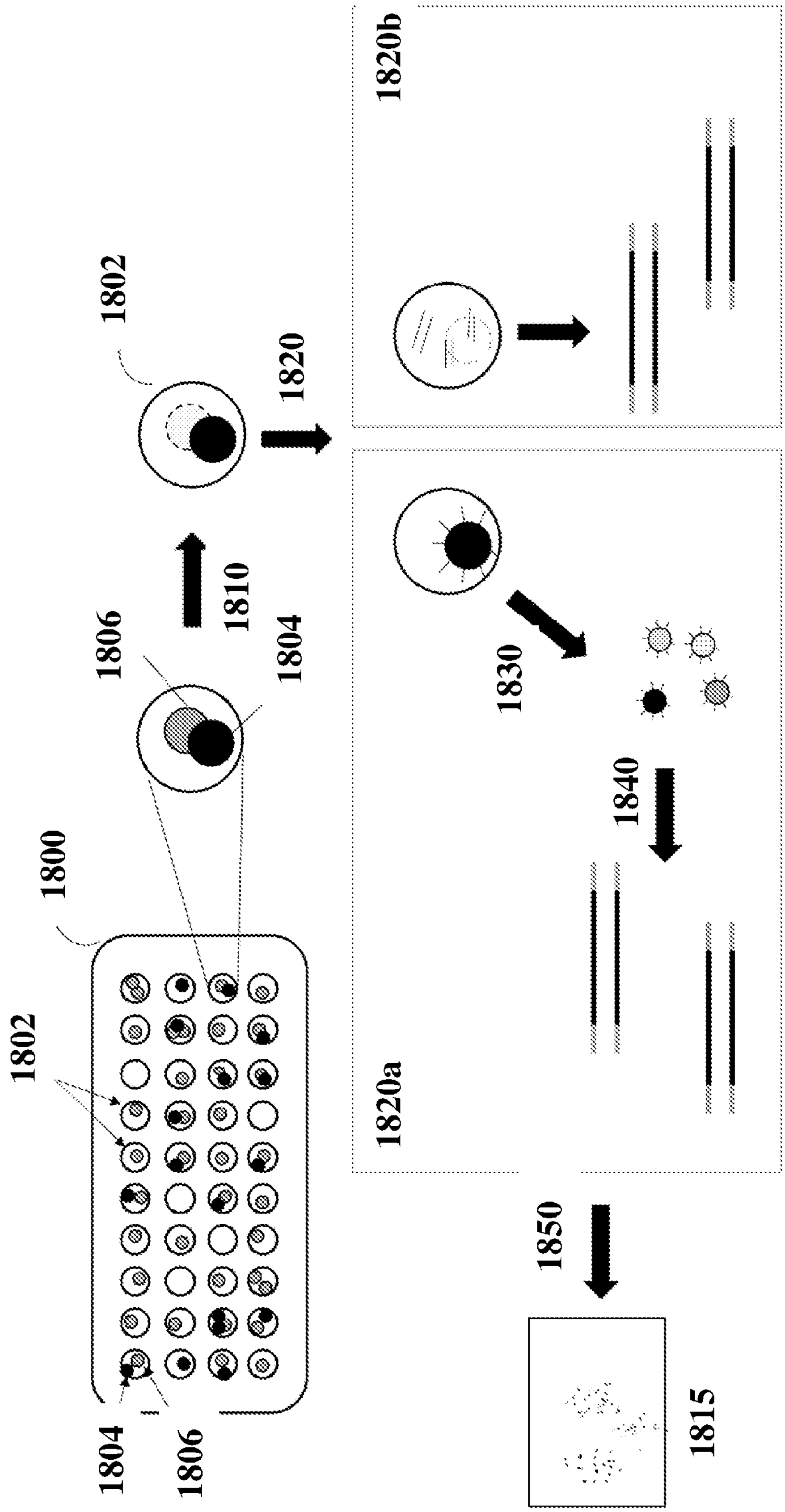
1800
1802
1804
1806
1810
1820
1820a
1820b
1830
1840
1850
1815

*FIG. 26*

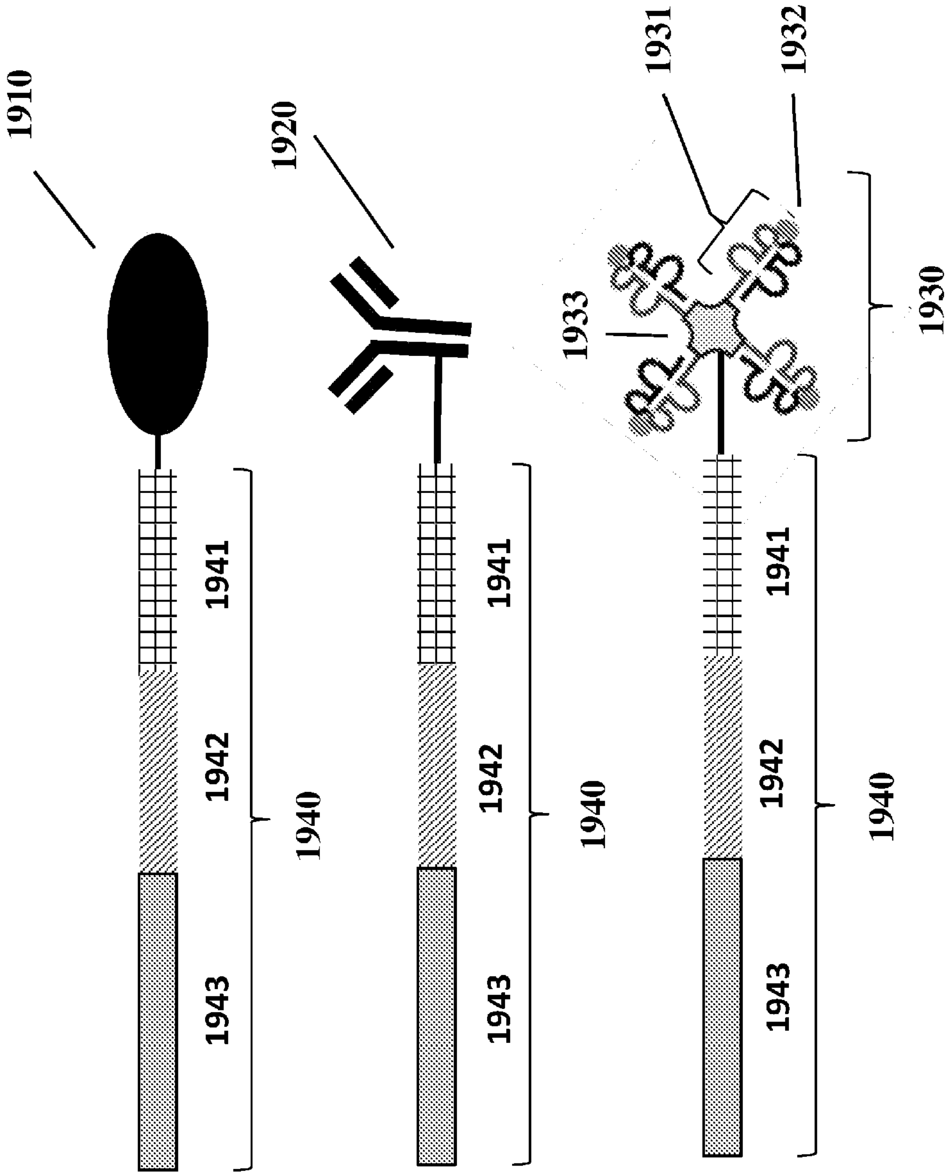
1910
1920
1931
1932
1933
1930
1941
1942
1943
1940
1941
1942
1943
1940
1941
1942
1943
1940

FIG. 27

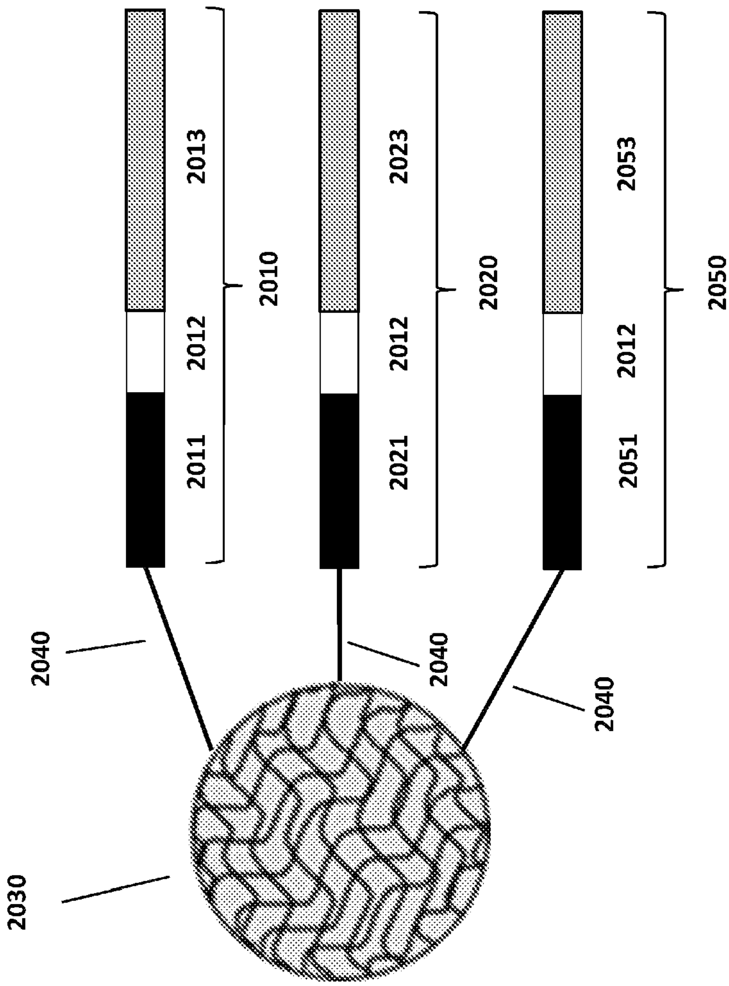
2013
2012
2011
2010
2023
2012
2021
2020
2053
2012
2051
2050
2040
2040
2040
2030

FIG. 28A

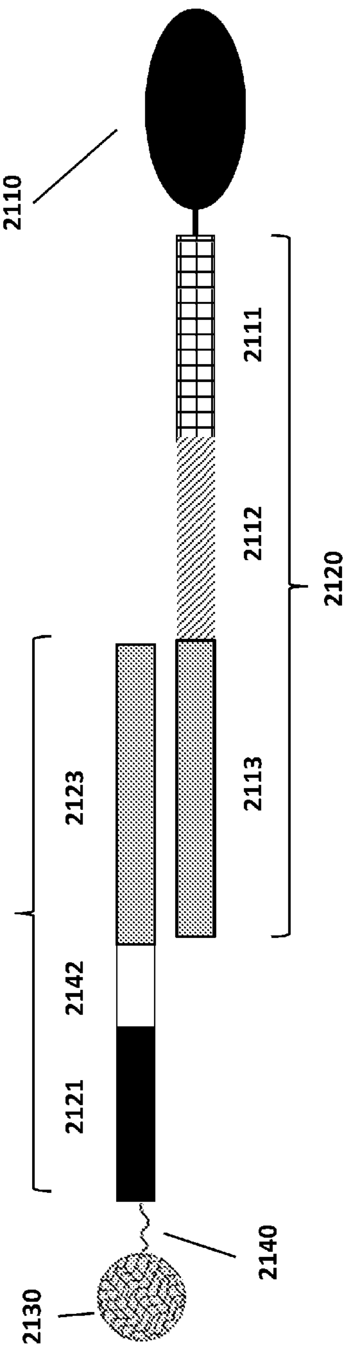


FIG. 28B

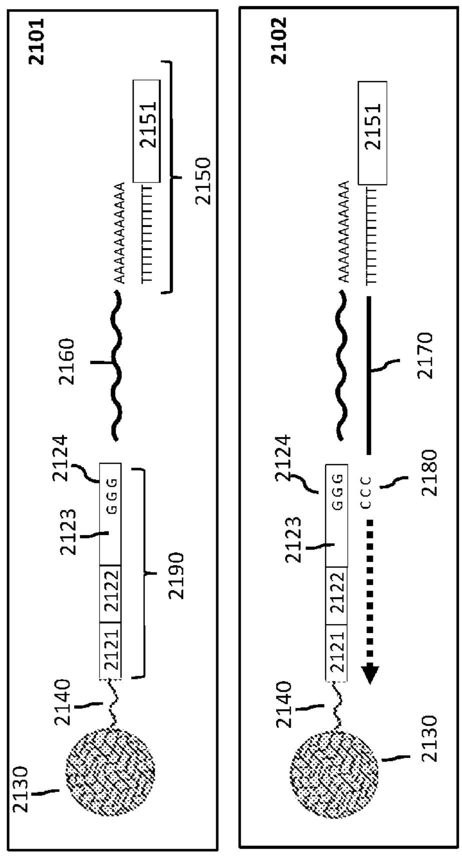

*FIG. 28C*

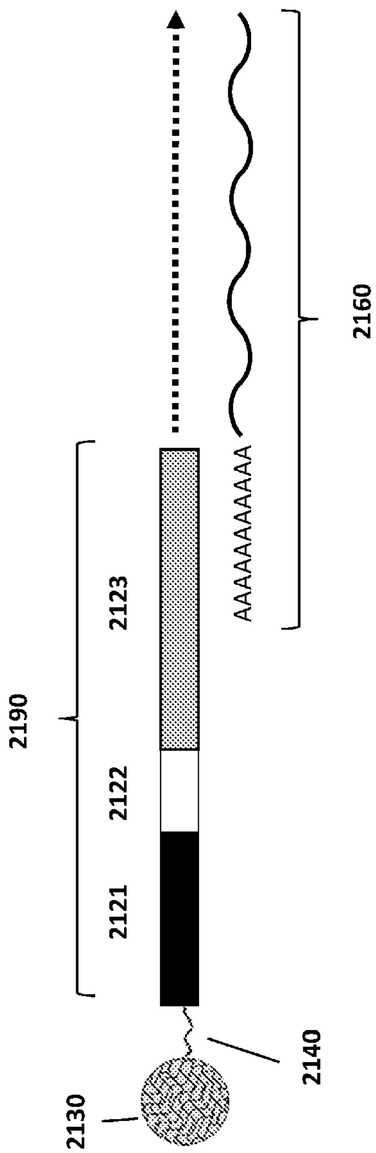

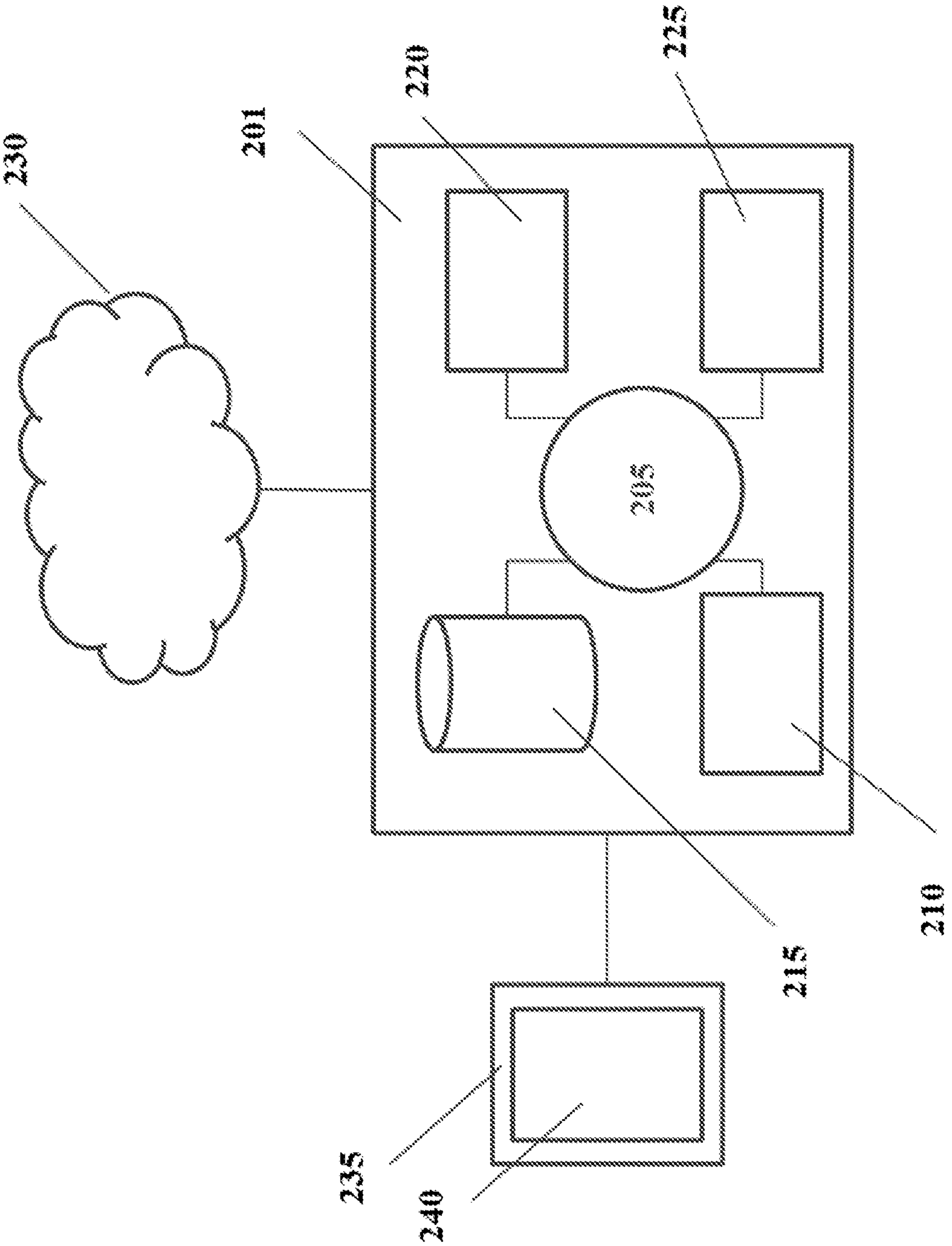


FIG. 29

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019071039 A
- WO 2020176449 A
- US 10550429 B
- US 20190177800 A
- US 20190367969 A
- US 2018064600 W
- US 20190323088 A
- US 6265552 B
- US 10428326 B
- US 20190100632 A
- US 20190100632 A1
- US 20190233878 A1
- WO 20167862 A
- US 20140155295
- US 20100105112 A1
- US 20100105112
- US 20150376609
- US 2018016019 W
- US 20140378345
- US 20190367997 A
- US 20190064173 A
- US 2017785 W
- US 20020486 W
- US 20150292988 A
- US 20140378345 A
- US 20150376609 A
- US 20180216162 A
- US 10590244 B
- US 20190233878 A
- US 20150292988 A1
- US 2020062195 W
- US 20190323088 A1
- US 20180105808 A
- WO 2018075693 A
- US 9694361 B
- US 10357771 B
- US 10273541 B
- US 10011872 B
- US 20180105808 A1
- US 20190367982 A1
- US 20190338353 A1
- US 20180216162 A1


### Non-patent literature cited in the description


- **S. SAKAI ; Y. LIU ; M. SENGOKU ; M. TAYA.** Cell-selective encapsulation in hydrogel sheaths via biospecific identification and biochemical cross-linking. *Biomaterials,* 2015, vol. 53, 494-501
- **S.V. GOHIL ; S.B. BRITTAIN ; H. KAN ; H. DRISSI ; D.W. ROWE ; L.S. NAIR.** Evaluation of enzymatically crosslinked injectable glycol chitosan hydrogel. *J. Mater. Chem. B,* 2015, vol. 3, 5511-5522
- **HUGHES L D et al.** *PLoS One.,* 04 February 2014, vol. 9 (2), e87649
- **FANG et al.** Fluoride-Cleavable Biotinylation Phosphoramidite for 5'-end-Labelling and Affinity Purification of Synthetic Oligonucleotides. *Nucleic Acids Res.,* 15 January 2003, vol. 31 (2), 708-715
- **LLG, PATRICK.** *Soft Matter,* 09 April 2013, (13), 3439-3682
- **JUS et al.** Enzymatic cross-linking of gelatin with laccase and tyrosinase. *Biocatalysis and Biotransformation,* 2012, vol. 30, 86-95
- **KIM et al.** Tissue adhesive, rapid forming, and sprayable ECM hydrogel via recombinant tyrosinase crosslinking. *Biomaterials,* 02 May 2018, vol. 178, 401-412
- **SAKAI et al.** Hematin is an Alternative Catalyst to Horseradish Peroxidase for In Situ Hydrogelation of Polymers with Phenolic Hydroxyl Groups In Vivo. *Biomacromolecules,* August 2010, vol. 11 (8), 2179-83
- **HICKEY et al.** Cross-Coupling of Amide and Amide Derivatives to Umbelliferone Nonaflates: Synthesis of Coumarin Derivatives and Fluorescent Materials. *J. Org. Chem.,* 2020, vol. 85 (12), 7986-7999